(19) Europäisches Patentamt / European Patent Office / Office européen des brevets

(11) **EP 2 684 561 B1**

(12) **EUROPEAN PATENT SPECIFICATION**

(45) Date of publication and mention
of the grant of the patent:
**14.09.2022 Bulletin 2022/37**

(51) International Patent Classification (IPC):
*A61K 33/44* (2006.01)    *A61K 9/20* (2006.01)
*A61P 1/16* (2006.01)    *A61P 13/12* (2006.01)
*A61P 25/18* (2006.01)    *A61P 39/00* (2006.01)

(21) Application number: **12754386.6**

(22) Date of filing: **05.03.2012**

(52) Cooperative Patent Classification (CPC):
**A61K 9/205; A61K 9/2095; A61K 33/44;
A61P 1/16; A61P 13/12; A61P 25/18; A61P 39/00**

(86) International application number:
**PCT/JP2012/055538**

(87) International publication number:
**WO 2012/121202 (13.09.2012 Gazette 2012/37)**

(54) **TABLET-TYPE COMPOSITION FOR ORAL ADMINISTRATION AND METHOD FOR PRODUCING SAME**

TABLETTENFÖRMIGE ZUSAMMENSETZUNG ZUR ORALEN VERABREICHUNG UND VERFAHREN ZU IHRER HERSTELLUNG

COMPOSITION DE TYPE COMPRIMÉ POUR ADMINISTRATION ORALE ET PROCÉDÉ POUR PRODUIRE CELLE-CI

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.03.2011 JP 2011047251**

(43) Date of publication of application:
**15.01.2014 Bulletin 2014/03**

(73) Proprietor: **Kureha Corporation**
**Chuo-ku**
**Tokyo 103-8552 (JP)**

(72) Inventors:
• **KAINOSE, Ayumi**
**Tokyo 103-8552 (JP)**
• **KOYA, Yohei**
**Tokyo 103-8552 (JP)**
• **MACHI, Yoshiki**
**Tokyo 103-8552 (JP)**
• **ONO, Saichi**
**Tokyo 103-8552 (JP)**
• **CHIBA, Tadahiko**
**Tokyo 103-8552 (JP)**

(74) Representative: **Novagraaf Group**
**Chemin de l'Echo 3**
**1213 Onex/ Geneva (CH)**

(56) References cited:
EP-A1- 0 688 568      EP-A1- 1 745 793
WO-A1-2009/049239     JP-A- 55 095 611
JP-A- 2002 308 786    JP-A- 2005 187 405
JP-A- 2005 524 604    US-A- 2 787 579
US-A- 4 761 284       US-B1- 6 294 189

• ABSTRACTS OF 124TH ANNUAL MEETING OF PHARMACEUTICAL SOCIETY OF JAPAN 05 March 2004, page 121, XP008170400

**Description**

TECHNICAL FIELD

The present invention relates to a tablet-formed pharmaceutical composition for oral administration, comprising a spherical activated carbon as an active ingredient for adsorbing toxic substances (hereinafter, sometimes referred to as tablet-type adsorbent composition for oral administration).

BACKGROUND ART

An adsorbent for oral administration can be taken orally, and liver or kidney disorder can be treated by adsorbing toxic substances in a gastrointestinal tract (Patent literature 1). In order to exhibit pharmacological effects of adsorbing the toxic substances, it is important that a spherical form of the spherical activated carbon is maintained and further pore structures thereof are maintained. For example, the adsorbent for oral administration is commercially available as a trade name "Kremezin (registered trademark) capsule 200 mg" and a trade name "Kremezin (registered trademark) fine granule package 2 g" (hereinafter referred to as a "Kremezin").

A daily dosage of Kremezin to a kidney disease patient is 6 grams, and the daily dosage of Kremezin is administered to the patient in three parts. Thus, a single dosage amount is 2 grams. A volume of 2 grams of the Kremezin fine granule is approximately 4 cm$^3$, and the volume thereof is never small. Therefore, when taking 4 cm$^3$ of fine granules, some patients dislike the feeling of any residue in the oral cavity due to an insolubility of the spherical activated carbon to water.

In the case of the Kremezin capsule, any residue in the oral cavity may not be felt. However, the capsule has a dead volume other than the volume of the spherical activated carbon. Thus, a volume of the capsule is increased by half (approximately 6 cm$^3$) in comparison with a volume of fine granules. Specifically, it is necessary to take ten capsules with a volume of 0.63 cm$^3$ per single dosage, and therefore, some patients dislike such a large volume thereof.

Further, many patients cannot take granules without taking in a large quantity of water, in order to avoid any residue in the oral cavity of the fine granules, and many patients cannot take capsules without taking in a large quantity of water, due to the large capsule volume. Further, for patients suffering from a kidney disease or renal failure, the amount of water to take is limited, and such patients are required to take minimal water when ingesting granules or capsules. Therefore, patients who essentially need a large quantity of water when taking granules or capsules feel intense pain when doing so.

CITATION LIST

PATENT LITERATURE

[Patent literature 1] Japanese Examined Patent Publication (Kokoku) No. 62-11611
[Patent literature 2] Japanese Unexamined Patent Publication (Kokai) No. 2006-8602
[Patent literature 3] Japanese Unexamined Patent Publication (Kokai) No. 2006-36734
[Patent literature 4] US 2,787,579 A

SUMMARY OF INVENTION

TECHNICAL PROBLEM

Accordingly, the object of the present invention is to provide an adsorbent for oral administration which can avoid any residue of fine granules in the oral cavity, reduce the administered volume thereof compared to capsules, and can be easily taken.

SOLUTION TO PROBLEM

The present inventors have conducted intensive studies for the formulation of the adsorbent for oral administration which can avoid any residue of fine granules in the oral cavity, reduce the volume thereof to be administered compared to capsules, and can be easy to take. As a result, the present inventors found that the above problems can be solved by the tablet-type adsorbent composition for oral administration using an excipient for tablet formulation with thin film-forming ability.

The term "excipient for tablet formulation" as used herein means one which can form a thin film when 0.5 mL of a water solution or water dispersions of the excipient for tablet formulation of 1 % by weight is put on a flat surface made of fluorine resin and heat-dried.

The spherical activated carbon has specific properties i.e. non-solubility to a solvent such as water, non-swelling property, hardness, and non-compaction property, unlike in the case of a general compound which is an active ingredient of medicine. Therefore, the spherical activated carbon is not applicable to a tablet compression method such as a wet granule compression method, dry granule compression method, or direct powder compression method, which is a general method for preparing a tablet. Patent literature 2 discloses that the spherical form of the activated carbon is destroyed by a tablet formation by compression, because the Kremezin has a spherical form. Therefore, the tablet formation of the spherical activated carbon is impossible. Thus, the adsorbent for oral administration is provided as the fine granules which are the spherical activated carbon per se or the capsules wherein the spherical activated carbons are packed. That is to say, tablet-type formulation, wherein fillers, lubricants, disintegrating agents, and binders are added, has not been commercially available.

In the general tablets, it is necessary to add sufficient amounts of excipients such as fillers and binders, other than active ingredients, in order to maintain a tablet form, and obtain a practical hardness and friability thereof. The fillers or binders exhibit properties as a powder which can form a tablet. In other words, the powder used as the fillers or binders combines a property of an elastic body such as a gum, and a property of a viscous fluid, and thus the powder can be formed by strong compression over a short period of time. Further, the compound, per se, which is the active ingredient, is provided as powders or granules, and therefore, has properties suitable for tablet formation. Generally, a mechanical characteristic of the solid is examined by applying an external force. That is, the rerationship between the force and the response. The relationship means a ratio (De) of a solid-specific response time to a force-applying time to the solid. A substance having a De extremely greater than 1 is solid such as a glass or metal which has a low viscoelasticity. On the other hand, a substance having a De close to zero exhibits a liquid property. The usual powder has properties between solids and liquids. Thus, if an active ingredient has properties of the usual powder (unless the De thereof is extremely high or is close to zero), the active ingredient can be formed into an appropriate tablet by a combination of the active ingredient with properties of powder and excipients, using a compression method.

However, the spherical activated carbon is solidly sintered, and thus a De of the spherical activated carbon is infinitely high as well as glass. The usual powder changes shape by straining according to an applied stress. Then, if the stress is removed, the usual powder is restored to the original state as time advances. On the other hand, the spherical activated carbon changes shape by applied stress. However, if a certain level or more of stress is applied to the spherical activated carbon, it is smashed to pieces. That is to say, the De of the spherical activated carbon is infinitely high as well as glass. If the usual powder with a certain level of deformability (i.e. fillers, binders and the like), and the particles without deformability (i.e. spherical activated carbons, etc.) are mixed and formed by compression, the particles without deformability prevent the compression formation. Thus, the compression formation of the mixture of the usual powder and the particles is difficult.

Accordingly, as described in Patent literature 2, the tablet formation of the spherical activated carbons by compression is impossible.

The present inventors have conducted intensive studies for a tablet having excellent hardness and friability, wherein shapes of the spherical activated carbons therein are maintained. However, as long as the spherical activated carbon is formed by compression using the conventional tableting machine, it is impossible to prepare the tablet containing a sufficient amount of spherical activated carbon, even if the composition of what kind of fillers, binders, and the like is used. In particular, a tablet containing a small amount of fillers, binders, and the like, could not be formed by compression. Alternatively, the spherical activated carbons in the tablet were destroyed. For example, Example 4 in Patent literature 3 discloses a tablet, which is formed by compression using an oil hydraulic press machine, containing 44.4 % by weight of spherical activated carbons. However, as shown in Comparative examples 16 to 18, the compressed tablets containing 20 % by weight, 15 % by weight, or 50.5 % by weight of the spherical activated carbons respectively, cannot be formed, and further, the spherical activated carbons are destroyed. Therefore, it is presumed that shapes of the spherical activated carbons contained in the tablet disclosed in Patent literature 3 are also destroyed.

Patent literature 4 discloses a medicinal preparation comprising a shaped product consisting essentially of activated carbon, which shaped product is coated with a thin hydrophilic film. The film consists of water-soluble carboxyalkyl ether of cellulose and is of a thickness of less than 0.01 mm.

The tablet-formed pharmaceutical composition for oral administration of the present invention contains a sufficient amount of spherical activated carbon, and the spherical activated carbon is not destroyed. Further, it is surprising that the tablet-formed pharmaceutical composition for oral administration of the present invention has a sufficient hardness and friability, from the above technical information of those skilled in the art.

The present inventors found that the tablet-formed pharmaceutical composition for oral administration wherein the amount of one or more excipients for tablet formulation totals 1 % or more by weight with respect to the tablet-formed pharmaceutical composition for oral administration, contains a sufficient amount of spherical activated carbon, and further has excellent hardness and friability.

The present invention is based on the above findings.

Subject matter of the present invention is a tablet-formed pharmaceutical composition for oral administration as defined in claim 1, and a method for preparing a tablet-formed pharmaceutical composition as defined in claim 9. The dependent claims relate to particular embodiments thereof.

Namely, the present invention relates to:

[1] a tablet-formed pharmaceutical composition for oral administration consisting of 65 % or more by weight of a spherical activated carbon as an active ingredient, and one or more excipients; wherein

(a) the spherical activated carbon does not exhibit a water solubility and a swelling characteristic; a distortion rate thereof is 2 % or less when 2 MPa of pressure is applied; and a fracture strength thereof is 5 MPa or more;
(b) the tablet-formed pharmaceutical composition for oral administration comprises one or more excipients for tablet formulation selected from the group consisting of pullulan, polyvinyl alcohol, gelatin, povidone, pregelatinized starch,

oxidized starch, hypromellose, hydroxypropyl starch, dextrin, polyvinyl alcohol-acrylic acid-methyl methacrylate copolymer, Kanbaiko, propylene glycol alginate, agar, glucomannan, carmellose sodium, hydroxyethylcellulose, hydroxypropylcellulose, tara gum, tamarind gum, carrageenan, methylcellulose, sodium hyaluronate, sodium alginate, carboxyvinyl polymer, partly pregelatinized starch, tragacanth, guar gum, xanthan gum, quince seed gum, locust bean gum, distarch phosphate, ghatti gum, and gellan gum, wherein the amount of the excipients for tablet formulation totals 1 % or more by weight (preferably 1.5 % or more by weight, more preferably 2 % or more by weight, further preferably 3 % or more by weight, further preferably 4 % or more by weight, further preferably 5 % or more by weight) with respect to the tablet-formed pharmaceutical composition for oral administration, provided that when the excipients for tablet formulation include pullulan, the amount of pullulan is 1 % or more by weight with respect to the tablet-formed pharmaceutical composition for oral administration, and the excipient for tablet formulation forms a thin film when 0.5 mL of a water solution or a water dispersion of the excipient for tablet formulation of 1 % by weight is heat-dried after being placed on a flat surface made of fluorine resin,

[2] the tablet-formed pharmaceutical composition for oral administration of the item [1], wherein the composition satisfies the following equation(1) :

$$V_1/V_2 \leq 1.53 \quad (1)$$

wherein $V_1$ is a volume of the tablet-formed pharmaceutical composition for oral administration, and V2 is a bulk volume of the spherical activated carbons contained in the tablet-formed pharmaceutical composition for oral administration in the case that the spherical activated carbons are closely packed (preferably 1.53 or less, more preferably 1.4 or less, further preferably 1.3 or less, further preferably 1.2 or less, most preferably 1.1 or less),

[3] the tablet-formed pharmaceutical composition for oral administration of the item [1] or [2], wherein an average particle diameter of the spherical activated carbon is 0.02 to 1 mm, and a specific surface thereof is 500 $m^2$/g or more,

[4] the tablet-formed pharmaceutical composition for oral administration of any one of the items [1] to [3], wherein a volume of the tablet-formed pharmaceutical composition for oral administration is 3.06 $cm^3$ or less with respect to 1 g of the spherical activated carbon contained therein as an active ingredient,

[5] the tablet-formed pharmaceutical composition for oral administration of any one of the items [1] to [4], wherein a spherical form of 80 % or more of the spherical activated carbon contained therein is maintained,

[6] the tablet-formed pharmaceutical composition for oral administration of any one of the items [1] to [5], wherein a hardness of the tablet-formed pharmaceutical composition for oral administration is 20N or more (preferably 30 N or more, more preferably 50 N or more),

[7] the tablet-formed pharmaceutical composition for oral administration of any one of the items [1] to [6], wherein a friability of the tablet-formed pharmaceutical composition for oral administration is 7 wt% or less (preferably 5 wt% or less, more preferably 3 wt% or less, further preferably 2 wt% or less, further preferably 1 wt% or less),

[8] the tablet-formed pharmaceutical composition for oral administration of any one of the items [1] to [7], wherein a weight-average molecular weight of the excipient for tablet formulation is 10,000 or more,

[9] a method for preparing a tablet-formed pharmaceutical composition for oral administration comprising the steps of:

(1) mixing a total of 100 parts by weight of spherical activated carbon and one or more excipients selected from the group consisting of pullulan, polyvinyl alcohol, gelatin, povidone, pregelatinized starch, oxidized starch, hypromellose, hydroxypropyl starch, dextrin, polyvinyl alcohol-acrylic acid-methyl methacrylate copolymer, Kanbaiko, propylene glycol alginate, agar, glucomannan, carmellose sodium, hydroxyethylcellulose, hydroxypropylcellulose, tara gum, tamarind gum, carrageenan, methylcellulose, sodium hyaluronate, sodium alginate, carboxyvinyl polymer, partly pregelatinized starch, tragacanth, guar gum, xanthan gum, quince seed gum, locust bean gum, distarch phosphate, ghatti gum, and gellan gum, and 10 parts or more by weight of solvent, wherein the spherical activated carbon is 65 to 99 parts by weight and the excipient is 1 to 35 parts by weight, and (a) the spherical activated carbon does not exhibit a water solubility and a swelling characteristic; a distortion rate thereof is 2 % or less when 2 MPa of pressure is applied; and a fracture strength thereof is 5 MPa or more, (b) one or more excipients for tablet formulation as the excipient totals 1 part or more by weight, provided that when the excipients for tablet formulation include pullulan, the amount of pullulan is 1 % or more by weight with respect to the tablet-formed pharmaceutical composition for oral administration, and the excipient for tablet formulation forms a thin film when 0.5 mL of a water solution or a water dispersion of the excipient for tablet formulation of 1% by weight is heat-dried after being placed on a flat surface made of fluorine resin, (2) forming the mixture into a tablet form product, and (3) drying the tablet form product,

[10] the method for preparing a tablet-formed pharmaceutical composition for oral administration of item [9], the drying process in the drying step is freeze drying, drying under reduced pressure, draught drying, or heat drying.

## ADVANTAGEOUS EFFECTS OF INVENTION

According to the tablet-formed pharmaceutical composition for oral administration of the present invention, the volume of the composition can be reduced compared to a capsule volume which contains the same amount of spherical activated carbon, and thus a composition for oral administration with an improved feel of ingestion can be provided. Further, according to the tablet-type adsorbent composition for oral administration of the present invention, a composition for oral administration, wherein disadvantages of the feel of ingestion such as any residue in the oral cavity are improved, can be provided, compared to the fine granules in which the spherical activated carbons per se as an active ingredient are taken.

Even further, the tablet-formed pharmaceutical composition for oral administration of the present invention has a large amount of spherical activated carbons, but has excellent hardness, friability, and stability, as a tablet.

In particular, according to the tablet-type adsorbent composition for oral administration of the present invention, an adsorbent composition for oral administration wherein a function of the spherical activated carbon is sufficiently maintained and the feel of ingestion is improved, can be provided. That is, it is possible to provide the adsorbent composition for oral administration capable of maintaining the spherical form of the spherical activated carbon, preventing destruction of pore structures, and exhibiting the function of the adsorbent for oral administration.

## BRIEF DESCRIPTION OF THE DRAWINGS

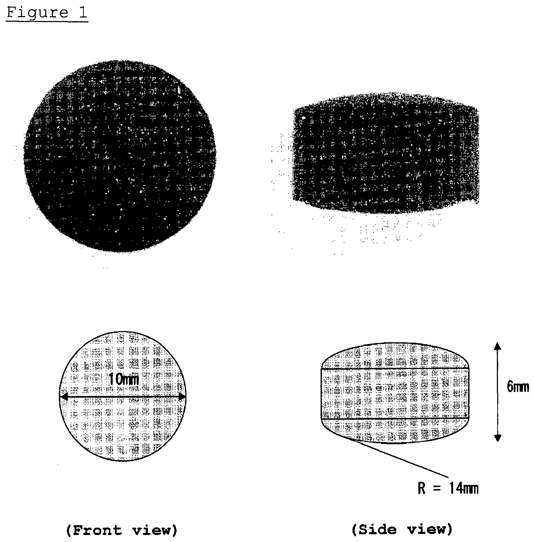

FIG. 1 is a photograph showing an embodiment of the tablet-type adsorbent composition for oral administration of the present invention.

FIG. 2 is a photograph showing the tablet-type adsorbent composition for oral administration in Comparative Examples 1 to 6, which are prepared by using a maltitol as the excipient.

FIG. 3 is a photograph of polyvinyl alcohol which can form a thin film (A) and a photograph of carmellose which cannot form a thin film (B).

FIG. 4 is a scanning electron micrograph of the surface of a tablet prepared by using an excipient for tablet formulation "pullulan" which exhibits thin film-forming ability (A) and a scanning electron micrograph of the surface of a tablet prepared by using an excipient "copovidone" which does not exhibit thin film-forming ability (B).

FIG. 5 is an electron micrograph of the inside of a tablet prepared by using an excipient for tablet formulation "pullulan" which exhibits thin film-forming ability (A) and an electron micrograph of the inside of a tablet prepared by using an excipient "copovidone" which does not exhibit thin film-forming ability (B).

FIG. 6 is a graph showing a relationship between load and displacement against the spherical activated carbon. From these results, a fracture strength and a distortion rate can be obtained in the destruction of a particle. Fig. 6(A) is a graph of the spherical activated carbon prepared in Manufacturing Examples 1, and Fig. 6(B) is a graph of the spherical activated carbon prepared in Manufacturing Examples 2.

## DESCRIPTION OF EMBODIMENTS

[1] Tablet-formed pharmaceutical composition for oral administration

The tablet-formed pharmaceutical composition for oral administration of the present invention consists of 65 % by weight of a spherical activated carbon as an active ingredient, and one or more excipients selected from the group consisting of pullulan, polyvinyl alcohol, gelatin, povidone, pregelatinized starch, oxidized starch, hypromellose, hydroxypropyl starch, dextrin, polyvinyl alcohol-acrylic acid-methyl methacrylate copolymer, Kanbaiko, propylene glycol alginate, agar, glucomannan, carmellose sodium, hydroxyethylcellulose, hydroxypropylcellulose, tara gum, tamarind gum, carrageenan, methylcellulose, sodium hyaluronate, sodium alginate, carboxyvinyl polymer, partly pregelatinized starch, tragacanth, guar gum, xanthan gum, quince seed gum, locust bean gum, distarch phosphate, ghatti gum, and gellan gum. The spherical activated carbon does not exhibit water solubility and a swelling characteristic. A distortion rate thereof is 2 % or less when 2 MPa of pressure is applied. A fracture strength thereof is 5 MPa or more.

(Spherical activated carbon)

The spherical activated carbon does not exhibit water solubility. The wording "a substance does not exhibit water solubility" as used herein means that 10000 mL or more of water is needed to dissolve 1 g of the substance. Specifically, the substance to be tested is milled into powder, and then the substance is added to water. The resulting mixture is strongly agitated for 30 seconds every five minutes, at 20 °C plus or minus 5 °C. Then, an amount dissolved in water within 30 minutes is examined. When 10000 mL or more of water is needed to dissolve 1 g of the substance, it is decided that the substance does not exhibit water solubility.

Further, the spherical activated carbon does not exhibit a swelling characteristic in response to water. The wording "a substance does not exhibit a swelling characteristic in response to water" as used herein means that a rate of volume increase is 5 % or less, when water is added to substances to be tested. In particular, a volume of the substances of 1 g is measured and 10 mL of water are added thereto. The mixture is agitated and allowed to stand for 1 hour. The substances are filtered and dried by a blower at 40 °C or less for 30 minutes. Then, all substances are collected and a volume thereof is measured. When the rate of volume increase is 5 % or less, it is decided that the substance does not exhibit a swelling characteristic in response to water. Alternatively, substances to be tested are filtered and a picture of them is taken under an appropriate background. When an increase rate of the average diameter is 2 % or less compared to an average diameter of substances before the addition of water and agitation, it is decided that the substance does not exhibit a swelling characteristic in response to water.

A distortion rate of the spherical activated carbon used in the present invention is 2 % or less, when 2 MPa of pressure is applied thereto. The term "pressure" as used herein means a value which is obtained by dividing a load applied to the spherical activated carbon by a cross-sectioned area of the spherical activated carbon. That is, when the pressure is applied, the spherical activated carbon does not change shape substantially. The distortion rate of the spherical activated carbon is examined using Better Hardness Tester (BHT-500, SEISHIN ENTERPRISE CO.,LTD.). A load and displacement at fracturing of the spherical activated carbon are plotted on a graph, and the distortion rate is calculated from the slope of the plotted points.

The spherical activated carbon used in the present invention has a fracture strength of 5 MPa or more. When the spherical activated carbon is fractured, an apparent stress (load) is reduced to zero. That is to say, when a certain level or more of stress is applied to the spherical activated carbon, the spherical activated carbon is destroyed without deformation. The fracture strength of the spherical activated carbon is examined using Better Hardness Tester (BHT-500, SEISHIN ENTERPRISE CO.,LTD.), and then it can be calculated by the following equation.

$$\text{Fracture strength (MPa)} = \text{Load at fracturing (N)}/\text{Cross-section area of loading plane (mm}^2)$$

A tablet-formed pharmaceutical composition for oral administration containing the spherical activated carbon for adsorbing harmful substances is a tablet-type adsorbent composition for oral administration. The spherical activated carbon does not exhibit water solubility and a swelling characteristic, as mentioned above. Further, a distortion rate thereof is 2 % or less when 2 MPa of pressure is applied, and a fracture strength thereof is 5 MPa or more, as shown in the Examples. That is, the spherical activated carbon is not deformable when stress is applied. When a certain level or more of stress is applied to the spherical activated carbon, it is smashed to pieces. Therefore, the tablet formation of the spherical activated carbon with the above physical properties is impossible, according to conventional methods.

However, the tablet-formed pharmaceutical composition for oral administration in the present invention comprises the excipient for tablet formulation which has thin film-forming ability. Therefore, the tablet-formed pharmaceutical composition for oral administration containing the spherical activated carbon can be formed. Further, it has practical hardness and friability as a tablet.

(Excipient)

The tablet-formed pharmaceutical composition for oral administration of the present invention consists of a spherical activated carbon and one or more excipients.

The term "excipient" used herein means all substances which can be contained in a tablet other than an active ingredient.

The tablet-formed pharmaceutical composition for oral administration of the present invention is characterized by comprising the excipient for tablet formulation as the excipient. However, the tablet-formed pharmaceutical composition for oral administration may comprise one or more excipients other than the excipient for tablet formulation (hereinafter sometimes referred to as "the other excipient"). That is, the tablet-formed pharmaceutical composition for oral administration of the present invention may contain the excipient for tablet formulation and the excipient other than the excipient for tablet formulation (the other excipient). Alternatively, it may contain the excipient for tablet formulation only. In other words, the excipient used in the present invention may consist of the excipient for tablet formulation and the excipient other than the excipient for tablet formulation (the other excipient), or may consist of the excipient for tablet formulation only.

(Excipient for tablet formulation)

The tablet-formed pharmaceutical composition for oral administration of the present invention comprises totally 1 % or more by weight of one or more excipients for tablet formulation. It comprises preferably 1.5 % or more by weight, more preferably 2 % or more by weight, further preferably 3 % or more by weight, further preferably 4 % or more by weight, further preferably 5 % or more by weight of one or more excipients for tablet formulation. When the excipients for tablet formulation are less than 1 % by weight, practical hardness and/or friability as a tablet may not be obtained. An amount of excipients for tablet formulation contained in the tablet-formed pharmaceutical composition for oral administration is 1 % or more by weight in total. In other words, an amount of one excipient for tablet formulation contained therein may be 1 % or more by weight. An amount of two or more excipients for tablet formulation contained therein may be 1 % or more by weight in total.

The excipient for tablet formulation contained in the tablet-formed pharmaceutical composition for oral administration of the present invention can form thin film when 0.5 mL of a water solution or a water dispersion of the excipient for tablet formulation of 1 % by weight is put on a flat surface made of fluorine resin and heat-dried at 125 °C. That is, when the water solution or water dispersions of the excipient for tablet formulation of 1 % by weight is thinly applied to a solution-non-permeable surface and heat-dried, the excipient for tablet formulation exhibits a thin film-forming ability.

As mentioned above, the excipient for tablet formulation can form a thin film, when the water solution or water dispersion of the excipient for tablet formulation of 1 % by weight is thinly applied to a solution-non-permeable surface and heat-dried. A test method for thin film-forming ability is as follows.

[Test method]

(1) An adhesive tape made of fluorine resin (NITOFLON Tape; NITTO DENKO Corp.) is put on a heat plate surface of stirrer with heater (12 cm × 12 cm).
(2) A water solution or a water dispersion of the excipient for tablet formulation of 1 % by weight is prepared. The purified water can be heated for dissolution or dispersion.
(3) 0.5 mL of the resulting water solution or water dispersion is put on the adhesive tape made of fluorine resin. The water solution or water dispersion forms a circular shape with a diameter of about 1 to 5 cm.
(4) The temperature of the heater is set at about 125 °C, and the water solution or water dispersion is heat-dried for 10 minutes.

In connection to this, the atmospheric conditions of the test are as follows.

Atmosphere: in the presence of air
Humidity: 20 to 80 % RH
Air current: general laboratory conditions
Pressure: atmospheric pressure

[Judgment]

(5) Tweezers are inserted between the adhesive tape made of fluorine resin and a thin film, and the thin film is gently peeled away.

(6) When a sheet of thin film can be peeled away from the adhesive tape made of fluorine resin, it is determined that a thin film is formed. In many excipients which cannot form the thin film, the thin film cannot be peeled away from the adhesive tape made of fluorine resin due to cracks in the thin film. The wording "a sheet of thin film can be peeled away" as used herein means that the thin film is not separated into two or more pieces when the thin film is peeled away gently.

If a judgment cannot be carried out within 10 minutes, the judgment is carried out after heat drying for 20 minutes. That is, if the thin film cannot be formed within 10 minutes or the thin film cannot be peeled away within 10 minutes, the judgment is carried out after heat drying for 20 minutes. However, if a formation of thin film and a separation of thin film cannot be observed by heat drying for 30 minutes, it is determined that an excipient does not have a thin film-forming ability.

As an excipient used in medicine, 479 items of pharmaceutical excipients are described in the "Japanese Pharmaceutical Excipients 2003"

(Yakuji Nippo Limited, 2003). Further, 1228 items of pharmaceutical excipients are described in the "Japanese Pharmaceutical Excipients Directory 2007" (Edited by International Pharmaceutical Excipients Council Japan, Yakuji Nippo Limited, 2007). Further, as food additives, 418 items (as of 2007, 3rd, Aug.) are described in the "List of Existing Food Additives". However, many types of compounds are sometimes described as one item such as "higher fatty acid", and therefore practically 418 or more of additives are used as food additives. Among these food additives, the compounds used as thickener (polysaccharide thickener), viscous agent, stabilizer, binder, or starch adhesive, can be used in the tablet-formed pharmaceutical composition for oral administration of the present invention.

The compound of the above pharmaceutical excipient or the above food additive can be used without limitation, so long as the compound has the thin film-forming ability. For example, fillers, lubricants, disintegrating agents, binders, or a combination thereof in the above pharmaceutical excipients can be used for tablet formulation. However, an excipient having at least a function as a binder is preferable, in order to obtain practical hardness and friability, and not to increase the volume of the tablet-type adsorbent composition for oral administration as far as possible. Further, an excipient having a binding property and a disintegrating property simultaneously is more preferable.

As an excipient for tablet formulation, in particular, there may be mentioned polysaccharide (such as starch, alginic acid and derivatives thereof, natural gum, simple polysaccharide, complex polysaccharide, and mucopolysaccharide), proteins, semisynthetic polymer, and synthetic polymer. More specifically, there may be mentioned pullulan, polyvinyl alcohol, gelatin, povidone, pregelatinized starch, oxidized starch, hypromellose, hydroxypropyl starch, dextrin, polyvinyl alcohol-acrylic acid-methyl methacrylate copolymer, Kanbaiko, propylene glycol alginate, agar, glucomannan, carmellose sodium, hydroxyethylcellulose, hydroxypropylcellulose, tara gum, tamarind gum, carrageenan, methylcellulose, sodium hyaluronate, sodium alginate, carboxyvinyl polymer, partly pregelatinized starch, tragacanth, guar gum, xanthan gum, pectin, hyaluronic acid, modified starch, acetylated starch, starch acetate, soluble starch, amylose, amylopectin, quince seed gum, linseed gum, cassia gum, locust bean gum, sodium caseinate, collagen, soybean peptide, distarch phosphate, ghatti gum, and gellan gum, and combinations of two or more thereof.

In connection with this, as the partly pregelatinized starch, a starch wherein normal starch is pregelatinized via heating may be used. In particular, a starch wherein a corn starch is dissolved into hot water to thereby pregelatinize may be used. Further, the carmellose sodium can be prepared by dissolving carmellose with 0.1 mol/L of aqueous sodium hydroxide, and then the resulting carmellose sodium may be used.

Further, as a preferable excipient for tablet formulation in the light of friability and disintegrating property, pullulan, polyvinyl alcohol, gelatin, povidone, pregelatinized starch, oxidized starch, hypromellose, hydroxypropyl starch, dextrin, polyvinyl alcohol-acrylic acid-methyl methacrylate copolymer, Kanbaiko, propylene glycol alginate, agar, glucomannan, carmellose sodium, hydroxyethylcellulose, hydroxypropylcellulose, tara gum, tamarind gum, carrageenan, methylcellulose, sodium hyaluronate, sodium alginate, carboxyvinyl polymer, partly pregelatinized starch, tragacanth, guar gum, and xanthan gum are preferable. Further, the more preferable excipients for tablet formulation are pullulan, polyvinyl alcohol, gelatin, povidone, pregelatinized starch, oxidized starch, hypromellose, hydroxypropyl starch, dextrin, polyvinyl alcohol-acrylic acid-methyl methacrylate copolymer, Kanbaiko, propylene glycol alginate, agar, glucomannan, carmellose sodium, hydroxyethylcellulose, hydroxypropylcellulose, tara gum, and tamarind gum.

Furthermore, the even more preferable excipients for tablet formulation are pullulan, polyvinyl alcohol, gelatin, povidone, pregelatinized starch, oxidized starch, and hypromellose. Even further, most preferable excipients for tablet formulation are pullulan, polyvinyl alcohol, gelatin, and povidone.

According to the present invention, the tablet-formed pharmaceutical composition for oral administration comprises one or more excipients for tablet formulation selected from the group consisting of pullulan, polyvinyl alcohol, gelatin, povidone, pregelatinized starch, oxidized starch, hypromellose, hydroxypropyl starch, dextrin, polyvinyl alcohol-acrylic acid-methyl methacrylate copolymer, Kanbaiko, propylene glycol alginate, agar, glucomannan, carmellose sodium, hydroxyethylcellulose, hydroxypropylcellulose, tara gum, tamarind gum, carrageenan, methylcellulose, sodium hyaluronate, sodium alginate, carboxyvinyl polymer, partly pregelatinized starch, tragacanth, guar gum, xanthan gum, quince seed gum, locust bean gum, distarch phosphate, ghatti gum, and gellan gum.

In addition, the amount of the excipient for tablet formulation in the tablet-formed pharmaceutical composition for oral administration is 1 % or more by weight, preferably 1.5 % or more by weight, more preferably 2 % or more by weight, further preferably 3 % or more by weight, further preferably 4 % or more by weight, further preferably 5 % or more by weight. Specifically, the amount of pullulan, gelatin, povidone, pregelatinized starch, hypromellose, propylene glycol alginate, agar, glucomannan, carmellose sodium, hydroxyethylcellulose, hydroxypropylcellulose, tara gum, sodium hyaluronate, sodium alginate, carboxy vinyl polymer, tragacanth, guar gum, and xanthan gum in the tablet-formed pharmaceutical composition for oral administration is 1 % or more by weight, preferably 1.5 % or more by weight, further preferably 2 % or more by weight, further preferably 3 % or more by weight, further preferably 4 % or more by weight, further preferably 5 % or more by weight. Further, the amount of polyvinyl alcohol, hydroxypropyl starch, polyvinyl alcohol-acrylic acid-methyl methacrylate copolymer, carrageenan, methylcellulose, partly pregelatinized starch, dextrin and quince seed gum in the tablet-formed pharmaceutical composition for oral administration is 1 % or more by weight, preferably 2.0 % or more by weight, further preferably 3.5 % or more by weight, further preferably 4 % or more by weight, further preferably 5 % or more by weight. Furthermore, the amount of locust bean gum, and Kanbaiko in the tablet-formed pharmaceutical composition for oral administration is 1 % by weight, preferably 3.0 % or more by weight, further preferably 4.5 % or more by weight, further preferably 5 % or more by weight. Even further, the amount of oxidized starch, phosphoric acid-cross-linked starch, ghatti gum, and gellan gum in the tablet-formed pharmaceutical composition for oral administration is 1 % or more by weight, preferably 5.0 % or more by weight, further preferably 7.0 % or more by weight. The amount of tamarind gum in the tablet-formed pharmaceutical composition for oral administration is 1 % or more by weight, preferably 10 % or more by weight, further preferably 13 % or more by weight. The amount of pectin, hyaluronic acid, modified starch, acetylated starch, starch acetate, soluble starch, amylose, amylopectin, linseed gum, cassia gum, sodium caseinate, collagen, and soybean peptide may be 1 % or more by weight, preferably 1.5 % or more by weight, more preferably 2 % or more by weight, more preferably 3.0 % or more by weight, more preferably 3.5 % or more by weight, further preferably 4.5 % or more by weight, further preferably 5.0 % or more by weight, further preferably 7.0 % or more by weight, further preferably 10 % or more by weight, further preferably 13 % or more by weight.

The upper limit of the amount of the excipient for tablet formulation in the tablet-type adsorbent composition for oral administration is not particularly limited, so long as it is 35 % by weight or less, but preferably 30 % by weight or less, more preferably 25 % by weight or less, most preferably 20 % by weight or less.

A weight-average molecular weight of the excipient for tablet formulation is not particularly limited, but is preferably 10,000 or more. If the weight-average molecular weight is 10,000 or more, the excipient for tablet formulation tends to have an excellent thin film-forming ability. The weight-average molecular weight of the excipient for tablet formulation can be measured by the following method.

10 mg of an excipient for tablet formulation is dissolved with 1 mL of distilled water, and the mixture is applied to a high performance size exclusion chromatography under the following analysis condition. A weight-average molecular weight is determined by applying the obtained measurement value to a standard curve prepared using pullulan with a known weight-average molecular weight.

Table 1

| Analysis conditions | |
| --- | --- |
| Column | TSKgel G2000SWXL 7.8 mmI.D.x300 mm(TOSOH Corp.) |
| | TSKgel G3000SWXL 7.8 mmI.D.×300 mm(TOSOH Corp.) or TSKgel G4000SWXL 7.8 mmI.D.×300 mm(TOSOH Corp.) |
| Mobile phase | Water |
| Temperature of column | 40°C |
| Flow rate | 1.0 mL/min |
| Detection | Refractive index detector |
| Volume of applied sample | 10 $\mu$L |

When the excipient for tablet formulation is mixed with water in the concentration of 1 % or more by weight, the water solution wherein the excipient is solved, the water dispersions wherein the excipient is dispersed, or the water mixture wherein a part of the excipient is solved and a part of the excipient is dispersed, is obtained. As a method for mixing, the following method shown as a general rule of Japanese Pharmacopoeia may be used. In particular, the excipient for tablet formulation is milled into powder, and then the substance is added to a solvent. The resulting mixture is strongly agitated for 30 seconds every five minutes at 20 °C plus or minus 5 °C. Alternatively, the method for mixing shown by a supplier of the excipient for tablet formulation may be used. A temperature of water to which the excipient for tablet formulation is mixed may be 1 °C to 99 °C. The excipient for tablet formulation may be solved or dispersed by swelling at a concentration of 1 % or more by weight, and the resulting water solution or water dispersions are clear or slightly cloudy.

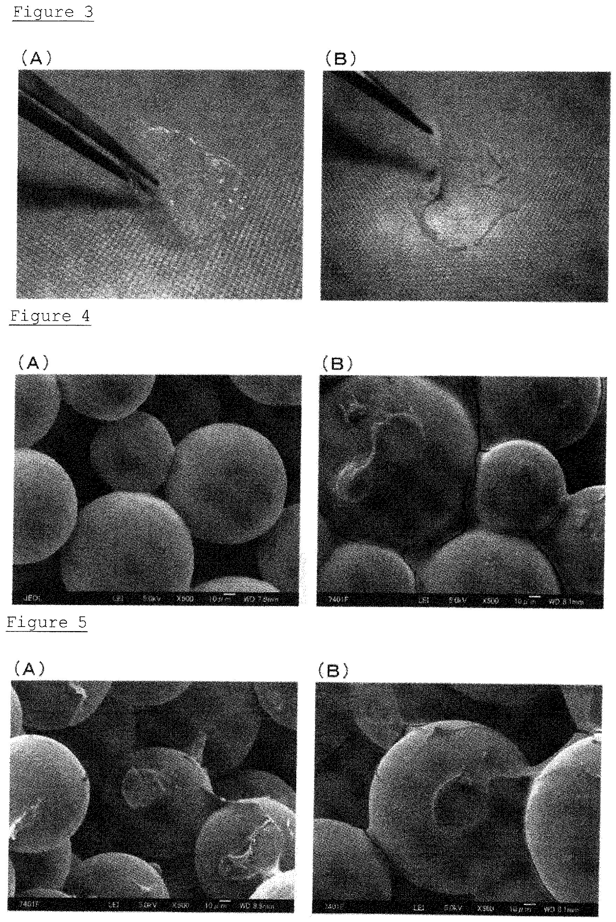

The excipients for tablet formulation used in the tablet-formed pharmaceutical composition for oral administration of the present invention have a thin film-forming ability. As shown in the Examples, the spherical activated carbon contained in the tablet-formed pharmaceutical composition for oral administration as an active ingredient does not deform in response to stress. However, when a certain level or more of stress is applied thereto, it is destroyed. That is, the spherical activated carbon has a property wherein De is infinitely high. In the tablet-formed pharmaceutical composition for oral administration of the present invention, such undeformable spherical activated carbons are coated with a thin film of the excipient for tablet formulation, and whereby a tablet with excellent hardness and friability can be formed. That is, the excipients for tablet formulation used in the present invention have a thin film-forming ability. As shown in Figure 4A, in the tablet-formed pharmaceutical composition for oral administration of the present invention, the thin film is formed on the surface of a spherical activated carbon such as a spherical activated carbon. The thin film has certain levels of tension strength and adherence properties. Therefore, it is estimated that the spherical activated carbons are bound over a large area via the thin film, and thus the binding state therebetween becomes stable. However, when an excipient not capable of forming thin film is used, the thin film covered on the spherical activated carbon is brittle and thus cracks after drying. Therefore, the tablet-type composition cannot be formed.

The thin film on the surface of the spherical activated carbon in the tablet-formed pharmaceutical composition for oral administration is formed by drying the excipient for tablet formulation dissolved or dispersed in a solvent. The tablet-formed pharmaceutical composition for oral administration of the present invention may be prepared according to the method of tablet-formed pharmaceutical composition for oral administration wherein the excipient for tablet formulation solved or dispersed in a solvent is dried and thin film is formed on the spherical activated carbon. For example, the tablet-formed pharmaceutical composition for oral administration may be prepared according to, but is by no means limited to, the method for preparing the tablet-formed pharmaceutical composition for oral administration of the present invention.

(Excipients other than excipient for tablet formulation)

"Excipients" used in the present invention can be divided into the excipient for tablet formulation and the excipient other than that for tablet formulation (the other excipient). The excipient other than the excipient for tablet formulation includes fillers, lubricants, disintegrating agents, binders, and other substances which can be used for preparing tablets. However, compounds which may be used as excipients for tablet formulation may be contained in such fillers, lubricants, disintegrating agents, binders, and the like.

The tablet-formed pharmaceutical composition for oral administration of the present invention can comprise the excipient other than the excipient for tablet formulation, so long as it has practical hardness, friability, and disintegrating and dispersing properties for a tablet. The amount of the excipient other than the excipient for tablet formulation is 34 % by weight or less, more preferably 20 % by weight or less, further preferably 10 % by weight or less, further preferably 5 % by weight or less, most preferably 2 % by weight or less.

However, the excipient comprised in the tablet-formed pharmaceutical composition for oral administration of the present invention is substantially the excipient for tablet formulation, preferably. Therefore, most preferably, the tablet-formed pharmaceutical composition for oral administration of the present invention consists of the spherical activated carbon and the excipient for tablet formulation. That is to say, the tablet-formed pharmaceutical composition for oral administration consisting of 65 % to 99 % by weight of the spherical activated carbon as an effective component, and 1 % to 35 % by weight of the excipient for tablet formulation is most preferable.

However, even if a sufficient hardness and friability of the tablet-formed pharmaceutical composition for oral administration are obtained by adding the excipient for tablet formulation, disintegrating and dispersing properties thereof may be insufficient. In this case, a good balance between the binding property and the disintegrating property can be obtained by adding an appropriate disintegrating agent as the excipient other than the excipient for tablet formulation. Examples of the disintegrating agents which can be used in the tablet-formed pharmaceutical composition for oral administration of the present invention are a low substituted hydroxypropylcellulose, croscarmellose sodium, crospovidone, carmellose calcium, and the like, but it is not limited thereto.

(The other excipients)

The excipients which can be used as the excipients other than the excipients for tablet formulation (the other excipients) will be illustrated as follows. Among these excipients, an excipient with thin film-forming ability can be used as the excipient for tablet formulation.

Excipients used in general medicine are described in the "Japanese standards of medical package inserts 2003" and the "Japanese Pharmaceutical Excipients Directory 2007", and for example, there may be mentioned therein fillers, lubricants, disintegrating agents, binders, and the like. Each function of fillers, lubricants, disintegrating agents, and binders is not necessarily single. For example, microcrystalline cellulose classified into fillers also has a function of disintegrating agents in many cases, and also has a function of binders for increasing formability in a direct compression method. Thus, the respective functions of fillers, lubricants, disintegrating agents, and binders may be often overlapped.

The fillers are mainly used for volume and weight increase (bulking fillers). In particular, the fillers include starch, calcium hydrogen phosphate, synthetic aluminum silicate, magnesium trisilicate, or the like.

Further, binders are used to form tablets by applying adhesive power to active ingredients and bulking fillers. It is used for maintaining the shape of a formulation, preventing breaks in a packaging process or transportation, and increasing mechanical strength. In particular, binders include microcrystalline cellulose, low substituted hydroxypropylcellulose, carmellose sodium, powdered cellulose, hypromellose, methylcellulose, hydroxypropylcellulose, starch, pregelatinized starch, partly pregelatinized starch, dextrin, acacia, sodium alginate, tragacanth, purified gelatin, polyvinyl alcohol, povidone or the like.

Further, disintegrating agents are used for disintegrating and dispersing. That is, the tablet is disintegrated into fine powder and dispersed by the disintegrating agent that is wetted in a gastrointestinal tract. In particular, disintegrating agents include carmellose, carmellose calcium, low substituted hydroxypropylcellulose, hypromellose, powder cellulose, starch, sodium carboxymethyl starch, hydroxypropyl starch or the like.

Lubricants can improve properties of powder in tablet making, i.e. fluidity, filling property, adherability, and formability of powder. Therefore, lubricants may be used in improving quality and productivity of a tablet. In particular, lubricants include sucrose fatty acid ester, talc, magnesium stearate, stearic acid, or the like.

(The amount of spherical activated carbon and excipient for tablet formulation)

The amount of the spherical activated carbon contained in the tablet-formed pharmaceutical composition for oral administration of the present invention is 65% or more by weight, preferably 70 % or more by weight, more preferably 75 % or more by weight, further preferably 80 % or more by weight, most preferably 85 % or more by weight. For example, when the amount of the spherical activated carbon is less than 65 % by weight, the amount of the excipient with respect to the amount of the spherical activated carbon is increased. Thus, the volume of a tablet to be taken per one dose is increased, and thus, a volume of water for administration is also increased. This is not preferable. Further, an upper limit of the amount of the spherical activated carbon is not particularly limited, but preferably 99 % by weight or less, preferably 98.5 % by weight or less, more preferably 98 % by weight or less, further preferably 97 % by weight or less, further preferably 96 % by weight or less, further preferably 95 % by weight or less.

The tablet-formed pharmaceutical composition for oral administration of the present invention consists of 65 % or more by weight of the spherical activated carbon and one or more excipients, wherein (a) the amount of one or more excipients for tablet formulation contained therein as an excipient is 1 % or more by weight in total. Then, the tablet-formed pharmaceutical composition for oral administration of the present invention may comprise (b) the other excipients. That is to say, the tablet-formed pharmaceutical composition for oral administration of the present invention consists of 65 % to 99 % by weight of the spherical activated carbon and one or more excipients, wherein (a) the amount of one or more excipients for tablet formulation contained therein is 1 % to 35 % by weight in total, and (b) the amount of the other excipients is 0 % to 34 % by weight.

More preferably, the tablet-formed pharmaceutical composition for oral administration of the present invention consists of 65 % or more by weight of the spherical activated carbon and one or more excipients, wherein (a) the amount of one or more excipients for tablet formulation contained therein as excipients is 1.5 % or more by weight in total. Then, the tablet-formed pharmaceutical composition for oral administration of the present invention may comprise (b) the other excipients. That is to say, the tablet-formed pharmaceutical composition for oral administration of the present invention consists of 65 % to 98.5 % by weight of the spherical activated carbon and one or more excipient, wherein (a) the amount of one or more excipients for tablet formulation contained therein is 1.5 % to 35 % by weight in total, and (b) the amount of the other excipients is 0 % to 33.5 % by weight.

Further preferably, the tablet-formed pharmaceutical composition for oral administration of the present invention consists of 65 % or more by weight of the spherical activated carbon and one or more excipients, wherein (a) the amount of one or more excipients for tablet formulation contained therein as excipients is 2 % or more by weight in total. Then, the tablet-formed pharmaceutical composition for oral administration of the present invention may comprise (b) the other excipients. That is to say, the tablet-formed pharmaceutical composition for oral administration of the present invention consists of 65 % to 98 % by weight of the spherical activated carbon and one or more excipients, wherein (a) the amount of one or more excipients for tablet formulation contained therein is 2 % to 35 % by weight in total, and (b) the amount of the other excipients is 0 % to 33 % by weight.

Further preferably, the tablet-formed pharmaceutical composition for oral administration of the present invention consists of 65 % or more by weight of the spherical activated carbon and one or more excipients, wherein (a) the amount of one or more excipients for tablet formulation contained therein as excipients is 3 % or more by weight in total. Then, the tablet-formed pharmaceutical composition for oral administration of the present invention may comprise (b) the other excipients. That is to say, the tablet-formed pharmaceutical composition for oral administration of the present invention consists of 65 % to 97 % by weight of the spherical activated carbon and one or more excipients, wherein (a) the amount of one or more excipients for tablet formulation contained therein is 3 % to 35 % by weight in total, and (b) the amount of other excipients is 0 % to 32 % by weight.

Further preferably, the tablet-formed pharmaceutical composition for oral administration of the present invention consists of 65 % or more by weight of the spherical activated carbon and one or more excipients, wherein (a) the amount of one or more excipients for tablet formulation contained therein as excipients is 4 % or more by weight in total. Then, the tablet-formed pharmaceutical composition for oral administration of the present invention may comprise (b) the other excipients. That is to say, the tablet-formed pharmaceutical composition for oral administration of the present invention consists of 65 % to 96 % by weight of the spherical activated carbon and one or more excipients, wherein (a) the amount of one or more excipients for tablet formulation contained therein is 4 % to 35 % by weight in total, and (b) the amount of the other excipients is 0 % to 31 % by weight.

Further preferably, the tablet-formed pharmaceutical composition for oral administration of the present invention consists of 65 % or more by weight of the spherical activated carbon and one or more excipients, wherein (a) the amount of one or more excipients for tablet formulation contained therein as excipients is 5 % or more by weight in total. Then, the tablet-formed pharmaceutical composition for oral administration of the present invention may comprise (b) the other excipients. That is to say, the tablet-formed pharmaceutical composition for oral administration of the present invention consists of 65 % to 95 % by weight of the spherical activated carbon and one or more excipients, wherein (a) the amount of one or more excipients for tablet formulation contained therein is 5 % to 35 % by weight in total, and (b) the amount of the other excipients is 0 % to 30 % by weight.

The amounts of the spherical activated carbon, the excipients for tablet formulation, and the other excipients contained in the tablet-formed pharmaceutical composition for oral administration, are not limited, so long as they are included in the above ranges.

(Spherical activated carbon)

The spherical activated carbon included in the tablet-formed pharmaceutical composition is not particularly limited, so long as it can be used for medical purposes, but preferably a spherical activated carbon for oral administration. That is, a spherical activated carbon which can be internally administered is preferable.

An average particle diameter of the spherical activated carbon contained in the adsorbent composition for oral administration of the present invention is not particularly limited, but preferably 0.02 to 1 mm, more preferably 0.03 to 0.90 mm, further preferably 0.05 to 0.80 mm. In addition, a range of particle sizes (diameter) of the spherical activated carbon is preferably 0.01 to 2 mm, more preferably 0.02 to 1.5 mm, further preferably 0.03 to 1 mm.

The term "spherical activated carbon" as used herein means one with a specific surface area of 100 $m^2/g$ or more. However, the specific surface area of the spherical activated carbon used in the present invention is preferably 500 $m^2/g$ or more, more preferably 700 $m^2/g$ or more, further preferably 1300 $m^2/g$ or more, most preferably 1650 $m^2/g$ or more.

Preferably, the tablet-formed pharmaceutical composition for oral administration (hereinafter, sometimes referred to as "tablet-type composition") of the present invention meets the following equation (1):

$$V_1/V_2 \leq 1.53 \quad (1)$$

wherein $V_1$ is the volume of the tablet-type composition, and V2 is a bulk volume of the spherical activated carbons in the case that the spherical activated carbons contained in the tablet-type composition are closely packed. That is to say, $V_1/V_2$ is preferably 1.53 or less, more preferably 1.4 or less, further preferably 1.3 or less, further preferably 1.2 or less, most preferably 1.1 or less. When $V_1/V_2$ is 1.53 or less, a volume of the tablet is reduced compared to that of a capsule which has the same volume of the spherical activated carbons. Thus, the ingestion feeling is improved. In other words, when the adsorbent for oral administration is taken, a volume of water for administration can be reduced.

For example, a capsule volume of commercially available "Kremezin capsule 200 mg" is 0.613 $cm^3$, and thus a volume of ten capsules per one dose is 6.13 $cm^3$. In this case, a total weight of the spherical activated carbons is 2 g. When 2 g of a spherical activated carbons with bulk density of 0.50 $g/cm^3$ is used in the tablet-type adsorbent composition for oral administration of the present invention, a bulk volume of the spherical activated carbon, in the case that the spherical activated carbons are closely packed, is 4 $cm^3$. Therefore, when $V_1/V_2$ is 1.53, $V_1$ is 6.12 $cm^3$. The

total volume of tablets is lower than the total volume of 10 capsules. Accordingly, the volume of the tablet-type adsorbent composition for oral administration of the present invention is smaller than one capsule. Therefore, the ingestion feeling is improved, and a volume of water for administration can be reduced.

The spherical activated carbon used in the present invention does not exhibit a water solubility and a swelling characteristic. Further, it is hard and does not have compaction property. Hitherto, there is no tablet wherein the spherical form of the spherical activated carbon with the above properties is maintained and a volume of tablet is 1.53 times or less with respect to the bulk volume of the spherical activated carbon. Further, it is surprising that a value of $V_1/V_2$ can be reduced to preferably 1.4 or less, more preferably 1.3 or less, further preferably 1.2 or less, or most preferably 1.1 or less.

The "volume of tablet-type composition ($V_1$)" in the equation (1) can be obtained by measuring an outer size of the tablet and calculating. The tablet includes a round tablet, ellipsoidal tablet, non-fully round tablet, non-fully ellipsoidal tablet, cylindrical tablet, disc-shaped tablet, lens-shaped tablet, rod-shaped tablet, rectangular tablet, polygonal tablet (such as triangle, quadrangle, pentagon, hexagon or the like). The volumes of most of these tablets can be calculated by the outer size such as diameter, thickness and the like. Further, the tablet-type composition is covered with a heat-shrinkable plastic film, and the heat-shrinkable plastic film is contracted while heating. Hereby, the water absorption of the resulting tablet-type composition is prevented. Thus, the resulting tablet-type composition is dipped in water, and an increased volume, that is, the tablet-type composition, can be measured.

The "a bulk volume of the spherical activated carbons in the case that the spherical activated carbons contained in the tablet-type composition are closely packed ($V_2$)" in the equation (1) can be measured as follows. For example, the bulk volume in close packing of the spherical activated carbons actually contained in the tablet-type composition may be measured. Further, the bulk volume in close packing of a spherical activated carbon used as a source corresponding to the spherical activated carbon actually contained in the tablet-type composition may be measured. Furthermore, the bulk volume in close packing of a spherical activated carbon having the same quality as the spherical activated carbon actually contained in the tablet-type composition (for example, spherical activated carbon contained in fine granules, or capsules) may be measured.

Specifically, a bulk volume of the spherical activated carbons in the case that the spherical activated carbons contained in the tablet-type composition are closely packed ($V_2$)" can be measured as follows.

Spherical activated carbons wherein a weight thereof is known are filled to a graduated cylinder, and the graduated cylinder is tapped fifty times. Then, a volume of the spherical activated carbons is measured; a tap density is calculated by dividing the weight by the volume. The "bulk volume of the spherical activated carbons in the case that the spherical activated carbons contained in the tablet-type composition are closely packed ($V_2$)" is obtained by dividing the weight of spherical activated carbons in a tablet by the tap density of the spherical activated carbons. For example, 20 g of spherical activated carbons are filled to 50 mL of graduated cylinder, and the graduated cylinder is tapped fifty times. Subsequently, a volume of the spherical activated carbons is measured, and the resulting volume is divided by the weight thereof (20 g) so as to obtain a tap density. Then, the "bulk volume of the spherical activated carbons in the case that the spherical activated carbons contained in the tablet-type composition are closely packed ($V_2$)" can be obtained by dividing the weight of the spherical activated carbons in a tablet by the tap density.

A rate (weight or bulk volume) of the spherical activated carbon contained in the tablet-formed pharmaceutical composition for oral administration of the present invention can be measured by a washing method. The washing method can be carried out as follows. An appropriate volume of water is added to a tablet-type composition, and then the tablet-type composition is disintegrated and dispersed by a treatment such as a sonication. If excipients in the tablet type composition are soluble in water, the excipients other than the spherical activated carbons are soluble in water. Thus, the spherical activated carbons can be separated by an appropriate method such as filtration or centrifugation. If necessary, the spherical activated carbons are further washed by water, and then a sample may be obtained by drying at 105 °C for 4 hours. A weight or a bulk density of the resulting sample is divided by a weight or a volume of the original tablet-type composition. Whereby, a rate (weight or bulk volume) of the spherical activated carbons contained in the tablet-formed pharmaceutical composition for oral administration of the present invention can be obtained directly.

Further, a rate of the spherical activated carbon contained in the tablet-type adsorbent composition for oral administration of the present invention can be measured by an elemental analysis method. The elemental analysis method can be carried out as follows. A tablet-type composition is put in a mortar, and it is grounded in the mortar. Alternatively, the tablet-type composition is broken up into smaller pieces using an appropriate grinder such as a vibratory ball mill. The milled products are homogeneously mixed, and a part thereof is analyzed by an elemental analyzer, to thereby measure the amount of carbon. When the amount of carbon is about 79 % or more by weight, 65 % or more by weight of the spherical activated carbon are contained therein.

A volume of the tablet-formed pharmaceutical composition for oral administration of the present invention is not particularly limited, so long as a tablet with the volume can be orally administered. The volume of tablet-type composition with respect to 1 g of the spherical activated carbons contained therein is 3.06 cm$^3$ or less, preferably 2.80 cm$^3$ or less, more preferably 2.60 cm$^3$ or less, further preferably 2.40 cm$^3$ or less, most preferably 2.20 cm$^3$ or less. The smaller volume of one tablet, the better the feel of ingestion.

As to a form of the spherical activated carbon contained in the adsorbent composition for oral administration, it is preferable to maintain a spherical form, in order to prevent side effects such as constipation. Further, as described below, adsorbability of harmful substances, for example selective adsorbability, is influenced by a diameter, an average particle diameter, a specific surface area, a pore volume within a scope of specific pore diameters, and the like. Therefore, it is preferable that the spherical activated carbons are not broken, the spherical form which has an effect on diameter or average particle diameter, is maintained, and a pore structure which has an effect on specific surface area or pore volume is maintained.

Therefore, a maintenance ratio of the spherical form in the spherical activated carbons contained in the adsorbent composition for oral administration is preferably 80 % or more, more preferably 85 % or more, most preferably 90 % or more. If the spherical form of 80 % or more of the spherical activated carbons is maintained, a function of the adsorbent for oral administration can be achieved.

A spherical particle rate of the spherical activated carbon contained in the tablet-type composition can be measured by particle shape image analyzer (PITA-2, SEISHIN ENTERPRISE CO.,LTD.). The measurement method is as follows.

In order to separate spherical activated carbons including destroyed particles from the tablet-type composition, the following pretreatment is carried out. The 1.5 g to 2.5 g of the tablets is put in 50 mL of plastic centrifuging tube, and further 50 mL of purified water are added thereto. The tablets are disintegrated and dispersed by a sonication for 10 minutes. Then, the spherical activated carbons are collected by filtering through membrane filter. The collected spherical activated carbons are dried at 105 °C, for 4 hours, to obtain a sample to be tested. In order to prevent a clogging of a device by an aggregate, if necessary, the aggregates are removed by an appropriate sieve.

Conditions of measurement and analysis using particle shape image analyzer (PITA-2, SEISHIN ENTERPRISE CO.,LTD.) are follows.

Table 2

| | Measurement conditions |
|---|---|
| Dispersion medium | Isopropanol |
| Object lens | $\times 2$ or $\times 4$ |
| Measurement range | $8 \sim 1500$ $\mu$m |
| Number of measured particles | 500 or more (0.1 g) |

Analysis conditions

- The particles having a degree of circularity of less than 0.6, and the particles having a maximum length of D90 (accumulating 90 % particle-sizes) or more, and a degree of circularity of less than 0.925, are considered as an aggregate. Such particles is removed from objects to be analyzed.

- The particles having a degree of circularity of 0.925 or more is considered as particles maintaining spherical form, and the particles having a degree of circularity of less than 0.925 are considered as particles not maintaining spherical form.

- Volume of each particle is calculated by the following equation.

$$\text{Volume} = 4/3 \times \pi \times (1/2 \times \text{equivalent circle diameter})^3$$

- Spherical particle rate (measured value) (%) is calculated by the following equation.

$$\text{Spherical particle rate (measured value) (\%)} = (\text{Total volume of particles maintaining spherical form / Total volume of all particles}) \times 100$$

- Additionally, spherical particle rate (measured value) (%) of a standard preparation, wherein spherical activated carbons and crushed products of spherical activated carbons are mixed at a constant rate, is measured, and thereby primary regression formula ($y = ax + b$) of true value X and measured value y is calculated. The spherical particle rate (true value) (%) of spherical activated carbons contained in the tablet-type composition is calculated according to the primary regression formula.

In order not to be broken in transport or packaging, it is preferable that the tablet-formed pharmaceutical composition for oral administration has a practical hardness. The hardness of the tablet-type composition is not particularly limited, but preferably 20 N or more, more preferably 30 N or more, further preferably 50 N or more. If the hardness is less than 20 N, the tablet-type composition may be broken in transport, or in unpackaging. Further, an upper limit of hardness of the tablet-type composition is not particularly limited. However, it is preferable that the disintegrating property of the tablet-type composition is not influenced by a value of hardness.

A hardness of the tablet-type composition is measured using tablet hardness tester (TBH320, ERWEKA). A thickness of the tablet to be tested is measured. The obtained thickness is put into the hardness tester, and then the hardness is measured at room temperature. Measurement conditions are as follows.

Table 3

| Measurement conditions | |
| --- | --- |
| Measurement mode | Constant Speed |
| Compaction rate | 2.3 mm / s |

A friability of the composition for oral administration of the present invention is not particularly limited, but preferably 7 wt% or less, more preferably 5 wt% or less, further preferably 3 wt% or less, further preferably 2 wt% or less, most preferably 1 wt% or less. If the friability becomes higher, the tablet composition is worn out in the package by an oscillatory load in transport thereof. That is to say, fine powders are developed and the tablet-type composition is broken.

The friability can be measured based on the tablet friability test of general information of "The Japanese Pharmacopoeia Fifteenth Edition", as follows.

1 to 6.5 g of tablet-type compositions is prepared. Powders attached to surfaces of the tablet-type compositions are removed before measurement, and then a weight of the tablet-type compositions is precisely measured. The tablet-type compositions are put into drum. The drum is rotated a hundred times, and the tablet-type compositions are taken out therefrom. In the same manner as before measurement, powders attached to surfaces of the tablet-type compositions are removed, and then a weight of the tablet-type compositions is precisely measured. The equation of friability is as follows.

(Calculus equation)

$$\text{Friability } (\%) = (\text{Weight of tablet-type compositions before measurement (g)} - \text{Weight of tablet-type composition after measurement (g)}) / \text{Weight of tablet-type compositions before measurement (g)} \times 100$$

The tablet-type adsorbent composition for oral administration containing spherical activated carbon as an active ingredient, is disintegrated in a body (gastrointestinal tract), and then separated spherical activated carbons act as adsorbent. In other words, if the tablet is not disintegrated, or excipients are bound to the spherical activated carbons, it is difficult that harmful substances are adsorbed to the spherical activated carbons. Therefore, if a disintegration time of the tablet-type adsorbent composition for oral administration containing the spherical activated carbon is long, it is preferable that the disintegration time is controlled by adding an appropriate disintegrating agent. The disintegration time of the adsorbent composition for oral administration of the present invention meets requirements of disintegration test stated in the Japanese Pharmacopoeia. That is, the disintegration time is preferably 30 minutes or less, more preferably 20 minutes or less, most preferably 10 minutes or less.

As the spherical activated carbon used in the tablet-type adsorbent composition for oral administration of the present invention, the spherical activated carbon described in, for example, Japanese Unexamined Patent Publication (Kokai) No. 11-292770, Japanese Unexamined Patent Publication (Kokai) No. 2002-308785 (Patent No. 3522708), WO2004/39381, WO2004/39380, Japanese Unexamined Patent Publication (Kokai) No. 2005-314415, Japanese Unexamined Patent Publication (Kokai) No. 2005-314416, Japanese Unexamined Patent Publication (Kokai) No. 2004-244414, Japanese Unexamined Patent Publication (Kokai) No. 2007-197338, and Japanese Unexamined Patent Publication (Kokai) No. 2008-303193 can be used. The spherical activated carbon described in the above patent literatures will be illustrated in order, below.

The spherical activated carbon disclosed in JP11-292770 is a spherical activated carbon having a diameter of preferably 0.05 to 2 mm, more preferably 0.1 to 1 mm. A specific surface area thereof is preferably 500 to 2000 $m^2$/g, more preferably 700 to 1500 $m^2$/g. Further, a volume of pores having a pore radius of 100 to 75000 angstrom is preferably 0.01 to 1 mL/g, more preferably 0.05 to 0.8 mL/g. In this connection, the specific surface area is measured by a methanol adsorption method using an automatic adsorption measuring apparatus. The pore volume is measured by a mercury press-injection porosimeter. The spherical activated carbon mentioned above has advantages in that it can be used as a dose without scattering, and that constipation is not caused by a repetitive administration, in comparison with a powdery activated carbon.

The shape of the spherical activated carbon is an important factor, and a substantially spherical shape is most important.

In the manufacture of the spherical activated carbon disclosed in JP11-292770, any starting materials for an active carbon, such as sawdust, coal, coconut shell, petroleum or coal pitches, or organic synthetic polymers, may be used. The spherical activated carbon may be produced, for example, by carbonizing the starting material and activating the carbonized substance. The activation may be performed by, for example, a steam-activation method, a chemical activation method, an air-activation method, or a $CO_2$ activation method, so long as a medically acceptable purity is maintained.

As the spherical activated carbon disclosed in JP11-292770, there are a granulated active carbon obtained from carbonaceous powder, a spherical activated carbon prepared by burning an organic polymer, or a spherical activated carbon obtained from a petroleum hydrocarbon (a petroleum pitch) and the like.

As the spherical activated carbon which may be used as the active ingredient in the present invention, (1) a spherical activated carbon treated with ammonia, or (2) a spherical activated carbon treated with an oxidation treatment and/or a reduction treatment, may be used. The treatments can be applied to, for example, the spherical activated carbon obtained from a petroleum pitch, the granulated active carbon obtained from carbonaceous powder, or the spherical activated carbon prepared by burning an organic polymer, as described above.

The above-mentioned oxidation treatment means a heat treatment at a high temperature in an oxidative atmosphere containing oxygen. As an oxygen source, for example, pure oxygen, nitrogen oxide, or air can be used. The above-mentioned reduction treatment means a heat treatment at a high temperature in an inert atmosphere with respect to carbon. The inert atmosphere with respect to carbon can be formed by using nitrogen gas, argon gas, or helium gas, or a mixed gas thereof.

The spherical activated carbon disclosed in JP2002-308785 is a spherical activated carbon having a diameter of 0.01 to 1 mm, a specific surface area determined by a BET method of 700 $m^2$/g or more, a volume of pores having a pore diameter of 20 to 15000 nm ranges from not less than 0.04 mL/g to less than 0.10 mL/g, a total amount of acidic groups of 0.30 to 1.20 meq/g, and a total amount of basic groups of 0.20 to 0.70 meq/g. The spherical activated carbon disclosed in JP2002-308785 has a specific range of pore volume, namely, a volume of pores having a pore diameter of 20 to 15000 nm is from not less than 0.04 mL/g to less than 0.10 mL/g. Further, a spherical activated carbon having a total amount of basic groups of 0.20 to 1.00 meq/g (see Japanese Patent Application No. 2002-293906 or Japanese Patent Application No. 2002-293907) may be used in the present invention.

In the spherical activated carbon disclosed in JP11-292770, a volume of pores having a pore radius of 100 to 75000 angstrom, i.e., a volume of pores having a pore diameter of 20 to 15000 nm, is 0.01 to 1 mL/g. According to the disclosures in JP2002-308785, when the volume of pores having a pore diameter of 20 to 15000 nm is adjusted to a range of from not less than 0.04 mL/g to less than 0.10 mL/g, an adsorbability of $\alpha$-amylase that is a useful substance, is significantly lowered, while maintaining a high adsorbability of $\beta$-aminoisobutyric acid, that is a toxic substance. When the volume of pores of the spherical activated carbon having a pore diameter of 20 to 15000 nm is increased, the useful substances such as digestive enzymes are more easily adsorbed. Therefore, a smaller volume of pores having a pore diameter of 20 to 15000 nm is preferable from a viewpoint that an adsorption of useful substances is reduced. On the other hand, if the volume of pores having such a pore diameter becomes too small, the adsorption of harmful substances is lowered. Therefore, in the adsorbent for an oral administration, a ratio (T/U) of an adsorption amount (T) of toxic substances to an adsorption amount (U) of useful substances, that is, a selective adsorption rate is important. For example, the selective adsorption rate of the spherical activated carbon can be evaluated by the ratio (Tb/Ua) of an adsorption amount (Tb) of DL-$\beta$-aminoisobutyric acid (toxic substance) to an adsorption amount (Ua) of $\alpha$-amylase (useful substance). More particularly, the selective adsorption rate can be evaluated by, for example, an equation:

$$A = Tb/Ua$$

wherein A denotes a selective adsorption rate, Tb denotes an adsorption amount of DL-$\beta$-aminoisobutyric acid, and Ua denotes an adsorption amount of $\alpha$-amylase.

The spherical activated carbon disclosed in JP2002-308785 exhibits an excellent selective adsorption rate when the volume of pores having a pore diameter of 20 to 15000 nm ranges from not less than 0.04 mL/g to less than 0.10 mL/g, and a more excellent selective adsorption rate when the volume of pores having a pore diameter of 20 to 15000 nm ranges from not less than 0.05 mL/g to less than 0.10 mL/g.

The spherical activated carbon disclosed in JP2002-308785 has a diameter of 0.01 to 1 mm, preferably 0.02 to 0.8 mm. In this connection, the expression that "a diameter is Dl to Du" as used herein means that a screen passing percentage (%) in a range of a screen opening Dl to Du is 90% or more in a particle-sizes accumulating standard curve prepared in accordance with JIS K 1474 as mentioned below in relation to a method for determining an average particle diameter.

The spherical activated carbon disclosed in JP2002-308785 has a specific surface area (referred to as "SSA" hereinafter) determined by a BET method of 700 $m^2/g$ or more. When the spherical activated carbon has the SSA of less than 700 $m^2/g$, an adsorbability of toxic substances is lowered. The SSA is preferably 800 $m^2/g$ or more. The upper limit of the SSA is not particularly limited, but the SSA is preferably 2500 $m^2/g$ or less in view of a bulk density and strength.

The spherical activated carbon disclosed in JP2002-308785 has a special constitution of functional groups, that is, a total amount of acidic groups is 0.30 to 1.20 meq/g, and a total amount of basic groups is 0.20 to 0.70 meq/g. When the spherical activated carbon does not satisfy the functional-groups requirement, i.e., the total amount of acidic groups is 0.30 to 1.20 meq/g and the total amount of basic groups is 0.20 to 0.70 meq/g, the adsorbability of the harmful substances is lowered. In the functional-groups requirement, the total amount of acidic groups is preferably 0.30 to 1.00 meq/g and the total amount of basic groups is preferably 0.30 to 0.60 meq/g. A preferable functional-groups constitution is that the total amount of acidic groups is 0.30 to 1.20 meq/g, the total amount of basic groups is 0.20 to 0.70 meq/g, a phenolic hydroxyl group is 0.20 to 0.70 meq/g, and a carboxyl group is 0.15 meq/g or less, and a ratio (a/b) of the total amount of acidic groups (a) to the total amount of basic groups (b) is 0.40 to 2.5, and a relation [(b+c)-d] between the total amount of basic groups (b), the phenolic hydroxyl group (c), and the carboxyl group (d) is 0.60 or more.

Properties of the spherical activated carbon disclosed in JP2002-308785, namely, the average particle diameter, the specific surface area, the pore volume, the total amount of acidic groups, and the total amount of basic groups are measured by the following methods.

(1) Average particle diameter

A particle-sizes accumulating standard curve is prepared in accordance with JIS K 1474 for the spherical activated carbon. The average particle diameter is determined from a screen opening (mm) at an intersection point with a line that is horizontal to an abscissa axis and starts from an intersection point in the particle-sizes accumulating standard curve with a perpendicular line from a 50 % point of the abscissa axis.

(2) Specific surface area

An amount of gas adsorbed is measured by a specific surface area measuring apparatus (for example, Flow Sorb II 2300 manufactured by MICROMERITICS) in accordance with a gas adsorbing method of a continuous flow for the spherical activated carbon sample, and a specific surface area can be calculated by a BET equation. More particularly, the spherical activated carbon is charged as a sample in a sample tube. A helium gas stream containing 30 % by volume of nitrogen is passed through the sample tube, and an amount of nitrogen adsorbed to the spherical activated carbon sample is measured by the following procedures. Specifically, the sample tube is cooled to -196°C, whereby nitrogen is adsorbed to the spherical activated carbon sample, and then the temperature of the sample tube is raised to room temperature. During the raising of the temperature, nitrogen is emitted from the spherical activated carbon sample. The amount of nitrogen emitted is measured by a heat conductivity type detector as an amount (v) of gas adsorbed.

A value $v_m$ is calculated in accordance with a one-point method (relative pressure x = 0.3) by a nitrogen adsorption at a temperature of liquid nitrogen, using an approximate equation:

$$v_m \;=\; 1/(v \cdot (1-x))$$

derived from the BET equation. Then, a specific surface area of the sample is calculated by an equation:

$$\text{specific surface area} = 4.35 \times v_m (m^2/g).$$

In the above equations, $v_m$ is an adsorption amount ($cm^3/g$) necessary to form a monomolecular layer on a surface of the sample, v is an adsorption amount ($cm^3/g$) actually found, and x is a relative pressure.

(3) Pore volume by a mercury injection method

The pore volume can be measured by a mercury press-injection porosimeter (for example, AUTOPORE 9200 manufactured by MICROMERITICS). The spherical activated carbon is charged as a sample in a sample vessel, and degassed under a pressure of 2.67 Pa or less for 30 minutes. Then, mercury is introduced into the sample vessel, and a pressure applied is gradually increased (maximum pressure = 414 MPa) to force the mercury into the micropores in the spherical activated carbon sample. A pore volume distribution of the spherical activated carbon sample is measured from a relationship between the pressure and an amount of forced mercury by equations as given below. Specifically, a volume of mercury inserted into the spherical activated carbon sample while a pressure is applied is increased from a pressure (0.07 MPa) corresponding to a pore diameter of 15 μm to the maximum pressure (414 Mpa) corresponding to a pore diameter of 3 nm. A pore diameter can be calculated as follows. When mercury is forced into a cylindrical micropore having a diameter (D) by applying a pressure (P), a surface tension (y) of mercury is balanced with a pressure acting on a section of the micropore, and thus, the following equation is held:

$$-\pi D\gamma\cos\theta = \pi(D/2)^2 \cdot P$$

wherein θ is a contact angle of mercury and a wall of the micropore. Therefore, the following equation:

$$D = (-4\gamma\cos\theta)/P$$

is held.

In the present specification, the relationship between the pressure (P) and the pore diameter (D) is calculated by an equation:

$$D = 1.27/P$$

given that a surface tension of mercury is 484 dyne/cm, a contact angle of mercury and carbon is 130°, a unit of the pressure P is MPa, and a unit of the pore diameter D is μm. The volume of pores having a pore diameter of 20 to 15000 nm in the present invention corresponds to a volume of mercury inserted by applying a pressure increasing from 0.07 MPa to 63.5 MPa.

(4) Total amount of acidic groups

The total amount of acidic groups is an amount of NaOH consumed, which may be determined by adding 1 g of the spherical activated carbon sample, after being crushed to form particles having a size of less than 200 mesh size (opening: 75 μm), to 50 mL of a 0.05 mol/L NaOH solution; shaking the mixture for 48 hours; then filtering out the spherical activated carbon sample; and titrating until neutralization.

(5) Total amount of basic groups

The total amount of basic groups is an amount of HCl consumed, which may be determined by adding 1 g of the spherical activated carbon sample after being crushed to form particles having a less than 200 mesh size (opening: 75 μm), to 50 mL of a 0.05 mol/L HCl solution; shaking the mixture for 24 hours; then filtering out the spherical activated carbon sample; and titrating until neutralization.

Further, as the spherical activated carbon which is active ingredient of the tablet-type adsorbent composition of the present invention, the spherical activated carbon, or the surface-modified activated carbon disclosed in WO2004/39381, can be used. That is, an adsorbent for oral administration, comprising a spherical activated carbon prepared from a thermosetting resin as a carbon source, wherein a diameter is 0.01 to 1 mm, and a specific surface area determined by Langmuir's adsorption equation is 1000 m$^2$/g or more, or the surface-modified activated carbon thereof, can be used.

In the spherical activated carbon or the surface-modified spherical activated carbon thereof disclosed in WO2004/39381, a more excellent adsorbability can be obtained when the spherical activated carbon of the present invention has a total amount of basic groups of 0.40 meq/g or more. The total amount of basic groups is more preferably 0.6 meq/g or more, most preferably 0.7 meq/g or more.

The thermosetting resin used as a starting material may be, for example, a phenolic resin, such as a novolak phenolic resin, a resol phenolic resin, a novolak alkylphenolic resin, or a resol alkylphenolic resin, or a furan resin, a urea resin, a melamine resin, or an epoxy resin. A copolymer of divinylbenzene and styrene, acrylonitrile, acrylic acid, or methacrylic acid may be used as the thermosetting resin.

Further, an ion-exchange resin may be used as the thermosetting resin. Generally, an ion-exchange resin comprises a copolymer of divinylbenzene and styrene, acrylonitrile, acrylic acid, or methacrylic acid, that is, a thermosetting resin, and essentially has a structure wherein ion-exchange groups are bonded to a copolymer matrix having a three-dimensional network skeleton. The ion-exchange resin is generally classified, with respect to the kinds of ion-exchange groups, into a strongly acidic ion-exchange resin having sulfonic acid groups, a weakly acidic ion-exchange resin having carboxylic or sulfonic acid groups, a strongly basic ion-exchange resin having quaternary ammonium salts, and a weakly basic ion-exchange resin having primary or tertiary amines. In addition, so-called hybrid ion-exchange resin having both acidic and basic ion-exchange groups is included as a special ion-exchange resin. In the present invention, all of the above ion-exchange resins may be used as a starting material, but a phenolic resin is preferably used.

In the spherical activated carbon or the surface-modified spherical activated carbon disclosed in WO2004/39381, a specific surface area (referred to as "SSA" hereinafter) determined by Langmuir's adsorption equation is 1000 $m^2/g$ or more. When the spherical activated carbon or the surface-modified spherical activated carbon has the SSA of less than 1000 $m^2/g$, an adsorbability of toxic substances is unfavorably lowered. The SSA is preferably 1000 $m^2/g$ or more. The upper limit of the SSA is not particularly limited, but the SSA is preferably 3000 $m^2/g$ or less in view of a bulk density and strength.

In the spherical activated carbon or the surface-modified spherical activated carbon disclosed in WO2004/39381, a volume of pores having a diameter of 7.5 to 15000 nm is not particularly limited, but preferably less than 0.25 mL/g, more preferably 0.2 mL/g or less, as a more excellent selective adsorbability is thus obtained.

In a constitution of functional groups of the surface-modified spherical activated carbon, that is, the product prepared by oxidizing and reducing the spherical activated carbon, which is disclosed in WO2004/39381, a total amount of acidic groups is 0.40 to 1.00 meq/g, and a total amount of basic groups is 0.40 to 1.10 meq/g. When the constitution of functional groups satisfies the condition that a total amount of acidic groups is 0.40 to 1.00 meq/g, and a total amount of basic groups is 0.40 to 1.00 meq/g, the selective adsorbability is improved, and particularly, the adsorbability of harmful substances is favorably enhanced. In the constitution of functional groups, a total amount of acidic groups is preferably 0.40 to 0.90 meq/g, and a total amount of basic groups is preferably 0.40 to 1.00 meq/g.

Properties of the spherical activated carbon or the surface-modified spherical activated carbon disclosed in WO2004/39381, namely, the average particle diameter, the pore volume, the total amount of acidic groups, and the total amount of basic groups may be measured by the methods disclosed in Japanese Unexamined Patent Publication (Kokai) No. 2002-308785. In connection with this, the volume of pores having a pore diameter of 7.5 to 15000 nm corresponds to a volume of mercury inserted by applying a pressure increasing from 0.085 MPa to 169 MPa.

Further, the specific surface area is measured by the following method.

(6) A specific surface area(Langmuir's adsorption equation)

An amount of gas adsorbed is measured by a specific surface area measuring apparatus (for example, ASAP2010 manufactured by MICROMERITICS) in accordance with a gas adsorbing method for the spherical activated carbon sample or the surface-modified spherical activated carbon sample, and a specific surface area can be calculated by Langmuir's adsorption equation. More particularly, the spherical activated carbon or the surface-modified spherical activated carbon is charged as a sample in a sample tube, and dried under reduced pressure at 300 °C. Thereafter, a weight of dried sample is measured. Then, the test tube is cooled to -196 °C, and nitrogen is introduced into the test tube, whereby nitrogen is adsorbed to the spherical activated carbon sample or the surface-modified spherical activated carbon sample. A relation of a nitrogen partial pressure and an adsorbed amount (absorption-isotherm line) is measured.

Langmuir's plotting is carried out, given that a relative pressure of nitrogen is p, and an adsorbed amount at that time is $v(cm^3/g$ STP). That is, the plotting in a range wherein p is 0.05 to 0.3 is carried out, in the field wherein a longitudinal axis is p/v, and an abscissa axis is p. Given that the gradient at that time is $b(g/cm^3)$, a specific surface area S (unit = $m^2/g$) can be calculated from the equation:

$$ S = \frac{MA \times \left(6.02 \times 10^{23}\right)}{22414 \times 10^{18} \times b} $$

wherein MA denotes a cross-sectional area of a nitrogen molecule, and is 0.162 $nm^2$.

Further, as the spherical activated carbon which is active ingredient of the tablet-type adsorbent composition of the present invention, the spherical activated carbon, or the surface-modified activated carbon disclosed in WO2004/39380, can be used. That is, an adsorbent for oral administration, comprising a spherical activated carbon, wherein a diameter is 0.01 to 1 mm, a specific surface area determined by Langmuir's adsorption equation is 1000 $m^2/g$ or more, and a diffraction intensity ratio, an R value, determined by an equation (1):

$$R=(I_{15}-I_{35})/(I_{24}-I_{35}) \qquad (1)$$

wherein $I_{15}$ is a diffraction intensity when a diffraction angle ($2\theta$) of an X-ray diffractometry is 15°, $I_{35}$ is a diffraction intensity when a diffraction angle ($2\theta$) of an X-ray diffractometry is 35°, and $I_{24}$ is a diffraction intensity when a diffraction angle ($2\theta$) of an X-ray diffractometry is 24°, is 1.4 or more, can be used.

Regarding the properties such as the average particle diameter, the specific surface area, the pore volume, the total amount of acidic groups, and the total amount of basic groups, the spherical activated carbon or the surface-modified spherical activated carbon disclosed in WO2004/39380 has the same properties as ones disclosed in WO2004/39381. In connection with this, one of the main characteristic features of the spherical activated carbon or the surface-modified spherical activated carbon disclosed in WO2004/39380 is that they are prepared from a thermosetting resin as a carbon source. On the other hand, the main characteristic feature of the spherical activated carbon or the surface-modified spherical activated carbon disclosed in WO2004/39381 is that they have a diffraction intensity ratio, R value, which meets the above equation (1). The surface-modified spherical activated carbon having a diffraction intensity ratio, an R value, of 1.4 or more, has an improved adsorbability of β-aminoisobutyric acid in comparison with the surface-modified spherical activated carbon having a diffraction intensity ratio, an R value, of less than 1.4, and thus the surface-modified spherical activated carbon having a diffraction intensity ratio, an R value, of 1.4 or more, is useful for the adsorbent for oral administration having an improved selective adsorbability of toxic substances. The diffraction intensity ratio, the R value, determined by an equation (1) for the spherical activated carbon or the surface-modified spherical activated carbon disclosed in WO2004/39380 is preferably 1.4 or more, more preferably 1.5 or more, most preferably 1.6 or more.

Further, as the spherical activated carbon which is an active ingredient of the tablet-type adsorbent composition for oral administration of the present invention, the spherical activated carbon having the smaller average particle diameter disclosed in Japanese Unexamined Patent Publication (Kokai) No. 2005-314415, i.e. the spherical activated carbon wherein an average particle diameter is 50 to 200 $\mu$m, a specific surface area determined by a BET method is 700 m$^2$/g or more, or the surface modified spherical activated carbon having the smaller average particle diameter disclosed in Japanese Unexamined Patent Publication (Kokai) No. 2005-314416, i.e. the specific surface area determined by a BET method is 700 m$^2$/g or more, a total amount of acidic groups is 0.30 to 1.20 meq/g, and a total amount of basic groups is 0.20 to 0.9 meq/g, can be used.

The spherical activated carbon disclosed in Japanese Unexamined Patent Publication (Kokai) No. 2005-314415 has a specified range of the average particle diameter, as mentioned above. The average particle diameter is 50 to 200 $\mu$m, preferably 50 to 180 $\mu$m, more preferably 50 to 150 $\mu$m. The wording "average particle diameter" (Dv50) as used herein means a particle size at the particle size cumulative percentage of 50 % in a particle size cumulative diagram based on a volume.

The spherical activated carbon disclosed in Japanese Unexamined Patent Publication (Kokai) No. 2005-314415 preferably has a narrow size distribution. For example, when a length average particle diameter of a number-based-average is $D_1(=\Sigma nD/\Sigma n)$, and a weight average particle diameter of a weight-based-distribution is $D_4$ ($=\Sigma(nD^4)/\Sigma(nD^3)$), the ratio ($D_4/D_1$) of the spherical activated carbon used as the adsorbent for an oral administration according to the present invention is preferably 3 or less, more preferably 2 or less, particularly preferably 1.5 or less. This means that the nearer the above ratio ($D_4/D_1$) is to 1, the narrower the size distribution. In the above equation, D is a representative particle diameter in a fraction of the particle diameters measured, and n is the number of particles.

A carbon source for the spherical activated carbon disclosed in Japanese Unexamined Patent Publication (Kokai) No. 2005-314415 may be any carbon-containing material. The carbon-containing material which may be used is, for example, a synthetic resin or pitch. A heat-fusible resin or a heat-infusible resin can be used as the synthetic resin. The term "heat-fusible resin" as used herein means a resin from which an activated carbon cannot be produced because it is melted and decomposed as a temperature is raised, if an activation treatment is carried out before a treatment to impart infusibility. However, when the heat-fusible resin is treated to impart infusibility in advance, and then is activated, an activated carbon can be produced therefrom. On the contrary, the heat-infusible resin means a resin from which an activated carbon can be produced by the proceeding of carbonization without melting as a temperature is raised, even if a treatment to impart infusibility is not carried out in advance. The treatment to impart infusibility is, for example, an oxidation treatment carried out at 150 °C to 400 °C under an atmosphere containing oxygen, as mentioned below.

A typical example of the heat-fusible resin is a thermoplastic resin, such as a cross-linked vinyl resin. A typical example of the heat-infusible resin is a thermosetting resin, such as a phenol or furan resin. Any known thermoplastic or thermosetting resin from which a spherical shape is formed can be used. When the surface-modified spherical activated carbon is produced from the cross-linked vinyl resin, the above treatment to impart infusibility is necessary. On the other hand, the above treatment to impart infusibility is not necessary when the spherical activated carbon is produced from an ion-exchange resin prepared by applying functional groups to the cross-linked vinyl resin. It is believed that the cross-linked resin is modified from the heat-fusible resin to the heat-infusible resin by the treatment used to introduce the functional groups thereto, and the functional groups introduced thereby. That is, the cross-linked vinyl resin belongs to the heat-fusible resin as used herein, whereas the ion-exchange resin belongs to the heat-infusible resin as used herein.

As a carbon source of the spherical activated carbon disclosed in Japanese Unexamined Patent Publication (Kokai) No. 2005-314415, an ion-exchange resin, a cross-linked vinyl resin, or pitch is preferably used, and an ion-exchange resin or a cross-linked vinyl resin is more preferably used.

In the spherical activated carbon disclosed in Japanese Unexamined Patent Publication (Kokai) No. 2005-314415, a specific surface area (referred to as "SSA" hereinafter) determined by a BET method is 700 $m^2$/g or more. When the spherical activated carbon has the SSA of less than 700 $m^2$/g, an adsorbability of toxic substances is unfavorably lowered. The SSA is preferably 1300 $m^2$/g or more. The upper limit of the SSA is not particularly limited, but the SSA is preferably 3000 $m^2$/g or less in view of a bulk density and strength.

The pore volume of the spherical activated carbon disclosed in Japanese Unexamined Patent Publication (Kokai) No. 2005-314415 is not particularly limited. For example, a volume of pores having a pore diameter of 20 to 15000 nm is preferably 0.01 to 1 mL/g, more preferably from more than 0.04 mL/g to 1 mL/g. Further, the bulk density of the spherical activated carbon disclosed in Japanese Unexamined Patent Publication (Kokai) No. 2005-314415 is not particularly limited, but preferably less than 0.54 g/mL, further preferably less than 0.50 g/mL.

Properties of the spherical activated carbon disclosed in Japanese Unexamined Patent Publication (Kokai) No. 2005-314415, namely, the average particle diameter, and specific surface area (method for calculating a specific surface area by a BET method) may be measured by the methods disclosed in Japanese Unexamined Patent Publication (Kokai) No. 2002-308785 or WO2004/39381. In connection with this, the volume of pores having a pore diameter of 7.5 to 15000 nm corresponds to a volume of mercury inserted by applying a pressure increasing from 0.085 MPa to 169 MPa.

Further, the bulk density is measured by the method described in Examples, and the particle size distribution is measured by the following method.

(7) Particle size distribution

A number-based particle distribution is measured by a laser diffraction apparatus for measuring particle size distribution [SALAD-3000S; Shimadzu Corporation] and a representative particle size D and the number n in a fraction of particles having particle size to be measured are determined. A length average particle diameter $D_1$, and a weight average particle diameter $D_4$ are calculated by the following equations:

$$[\text{equation } 5]$$
$$D_1 = \sum(nD)/\sum n$$

$$[\text{equation } 6]$$
$$D_4 = \sum(nD^4)/\sum(nD^3)$$

Regarding the properties such as the average particle diameter, the specific surface area, and the pore volume, the surface modified spherical activated carbon disclosed in Japanese Unexamined Patent Publication (Kokai) No. 2005-314416 has the same properties as ones disclosed in Japanese Unexamined Patent Publication (Kokai) No. 2005-314415. In particular, the surface modified spherical activated carbon disclosed in Japanese Unexamined Patent Publication (Kokai) No. 2005-314416 can be substantially obtained by oxidizing and reducing the spherical activated carbon disclosed in Japanese Unexamined Patent Publication (Kokai) No. 2005-314415.

A surface-modified spherical activated carbon disclosed in Japanese Unexamined Patent Publication (Kokai) No. 2005-314416 means a spherical activated carbon having a total acidic-group amount of 0.30 meq/g or more. On the other hand, a non-surface-modified spherical activated carbon means a spherical activated carbon having a total acidic-group amount of less than 0.30 meq/g. As mentioned below, the surface-modified spherical activated carbon is a porous material prepared by heat-treating a carbonaceous precursor, activating the resulting product, and subsequently, carrying out a surface-modification by oxidizing and reducing treatments. The surface-modified spherical activated carbon can exhibit a moderate interaction with acids and bases. On the other hand, the non-surface-modified spherical activated carbon is a porous material prepared, for example, by heat-treating a carbonaceous precursor, and activating the resulting product, that is, a spherical activated carbon without a subsequent surface-modification by oxidizing and reducing treatments, or a spherical activated carbon prepared by a heat-treatment at a non-oxidizing atmosphere after the activating treatment as mentioned above.

In the tablet-formed pharmaceutical composition for oral administration of the present invention, the measurement value in an adsorption rate test of indole is preferably 70 to 130 %, more preferably 85 to 115 %, compared to a measurement value of a capsule charged with the spherical activated carbon as the active ingredient. The tablet-formed pharmaceutical composition for oral administration exhibits the same adsorption property as the adsorbent for oral administration contained in the conventional fine granules or capsules. In other words, the adsorption rate of indole of the tablet-type adsorption composition containing excipients of the present invention is not decreased. The reason for this is presumed as follows. That is, a molecular weight of the excipient for tablet formulation is 10,000 or more, and the excipient for tablet formulation has a superior water solubility and water dispersibility. Therefore, the spherical activated carbons are rapidly dispersed in a body, and thus the excipient for tablet formulation does not prevent the adsorbability of the spherical activated carbon. However, the present invention is by no means limited to the above explanation.

The adsorption test of indole of the capsules charged with the spherical activated carbon or the tablet-formed pharmaceutical composition for oral administration of the present invention is performed using a dissolution tester described in Japanese Pharmacopoeia, as described below.

A test liquid is prepared by dissolving 0.1g of indole with 1 L of a 2nd Fluid for dissolution test. The test liquid is heated at 41 °C, and degassed by filtering with suction through 0.45 $\mu$m membrane filter. 900 mL of the degassed test liquid is charged to a vessel of the dissolution tester and is allowed to stand to 37 °C. A capsule containing 200 mg of the spherical activated carbons or a tablet-type composition containing 200 mg of the spherical activated carbons is added to the vessel of the dissolution tester, and is rotated at 100 rpm so as to start the adsorption test. After one hour, 10 mL of the test liquid are collected and filtered through a 0.45 $\mu$m membrane filter. The first 5 mL of test liquid are discarded, and the remaining filtrate is collected as a sample solution. An indole concentration of the sample solution is quantified using high-performance liquid chromatography. The condition of analysis is as follows.

Table 4

| Analysis conditions | |
| --- | --- |
| Column | A stenless steel column packed with octadecyl silanied silica gel 5 $\mu$m 4.6 mmI.D. ×150 mm |
| Mobile phase | Water/Methanol mixture (1:1) |
| Temperature of column | 40°C |
| Flow rate | 1.0 mL/min |
| Detection wavelength | 238 nm |
| Volume of applied sample | 10 $\mu$L |

Further, the tablet-formed pharmaceutical composition for oral administration may be coated with coating agents. The objects are to improve hardness and friability of a plain tablet, to mask disagreeable taste, bad odor, or color, or to stabilize an active ingredient. As the coating agent, there may be talc, precipitated calcium carbonate, gelatin, acacia, pullulan, hydroxypropylcellulose, hypromellose, polyvinylacetal diethylaminoacetate, Aminoalkyl methacrylate copolymer E, cellulose acetate phthalate, methacrylic acid copolymer L, methacrylic acid copolymer LD, methacrylic acid copolymer S, hypromellose phthalate ester, hydroxypropylmethylcellulose Acetate Succinate, carboxyethylcellulose, ethylcellulose, aminoalkyl methacrylate copolymer RS, polyvinyl alcohol-acrylic acid-methyl methacrylate copolymer, or ethyl acrylate methyl methacrylate copolymer dispersion.

After the tablet is formed, the coating agents are used to coat a surface of the plain tablet. Such coating agents are also included in the excipient in the present specification. That is, a compound which is generally classified into a coating agent can be used as the excipient for tablet formulation, so long as the compound meets the thin film-forming ability of the excipient for tablet formulation.

[2] Method for preparing tablet-type composition for oral administration

The tablet-type composition for oral administration of the present invention can be obtained by the following method for preparing a tablet-type composition for oral administration of the present invention. However, the tablet-type composition for oral administration of the present invention can be prepared via a preparation method besides that of the present invention.

The method for preparing a tablet-type composition for oral administration of the present invention comprises the steps of: (1) mixing 65 to 99 parts by weight of spherical activated carbons, 1 to 35 parts by weight of excipient, and 10 parts or more by weight of solvent, wherein a total of the spherical activated carbon and the excipient is 100 parts by weight, (2) forming the mixture into a tablet form product, and (3) drying the tablet form product. The spherical activated carbon does not exhibit water solubility and swelling characteristics. A distortion rate thereof is 2 % or less when 2 MPa of pressure is applied. A fracture strength thereof is 5 MPa or more. Further, the excipients comprise one or more parts by weight of at least one excipient for tablet formulation. The excipient for tablet formulation forms a thin film when 0.5 mL of a water solution or a water dispersion of the excipient for tablet formulation of 1 % by weight is put on a flat surface made of fluorine resin and then heat-dried.

Specifically, in the preparation method of the present invention, the excipient can be added to 65 to 99 parts by weight of spherical activated carbon so as to bring the total to 100 parts by weight. That is, the excipient is 1 to 35 parts by weight. Further, the excipient comprises 1 or more parts by weight of the excipient for tablet formulation. The excipient for tablet formulation is preferably 1.5 or more parts by weight, more preferably 2 or more parts by weight, further preferably 3 or more parts by weight, further preferably 4 or more parts by weight, further preferably 5 or more parts by weight. When 1 or more parts of the excipients for tablet formulation is added thereto, the obtained tablet can exhibit a practical hardness, friability, and disintegrating and dispersing properties. In the method of the present invention, an excipient other than the excipient for tablet formulation (the other excipient) may be added, so long as the obtained tablet has the practical hardness, friability, and disintegrating and dispersing properties. In connection with this, as the spherical activated carbon, the excipient, the excipient for tablet formulation, and the excipient other than the excipient for tablet formulation (the other excipient) used in the method for preparing a tablet-type composition for oral administration, ones described in the above section "[1]tablet-type composition for oral administration" may be used.

More specifically, in the method for preparing tablet-formed pharmaceutical composition for oral administration of the present invention, a total combination of the spherical activated carbon and one or more excipients is 100 parts by weight. In particular, 65 to 99 parts by weight of the spherical activated carbons, and 1 to 35 parts by weight of the excipients are used. As the excipient, (a) 1 or more parts by weight of the excipients for tablet formulation is used in total. Further, (b) the other excipients may be used. That is to say, in the method for preparing a tablet-formed pharmaceutical composition for oral administration, 65 to 99 parts by weight of the spherical activated carbons, and a total 1 to 35 parts by weight of one or more excipients are used. As the excipient, (a) a total 1 to 35 parts by weight of one or more excipients for tablet formulation, and (b) 0 to 34 parts by weight of the other excipients can be used.

More preferably, in the method for preparing a tablet-formed pharmaceutical composition for oral administration of the present invention, a total combination of the spherical activated carbon and one or more excipients is 100 parts by weight. In particular, 65 to 98.5 parts by weight of the spherical activated carbons, and 1.5 to 35 parts by weight of the excipients are used. As the excipient, (a) 1.5 or more parts by weight of the excipients for tablet formulation is used in total. Further, (b) the other excipients may be used. That is to say, in the method for preparing a tablet-formed pharmaceutical composition for oral administration, 65 to 98.5 parts by weight of the spherical activated carbons, and the total 1.5 to 35 parts by weight of one or more excipients are used. As the excipient, (a) the total 1.5 to 35 parts by weight of one or more excipients for tablet formulation, and (b) 0 to 33.5 parts by weight of the other excipients can be used.

Further preferably, in the method for preparing a tablet-formed pharmaceutical composition for oral administration of the present invention, a total combination of the spherical activated carbons and one or more excipients is 100 parts by weight. In particular, 65 to 98 parts by weight of the spherical activated carbons, and 2 to 35 parts by weight of the excipients are used. As the excipient, (a) 2 or more parts by weight of the excipients for tablet formulation is used in total. Further, (b) the other excipients may be used. That is to say, in the method for preparing a tablet-formed pharmaceutical composition for oral administration, 65 to 98 parts by weight of the spherical activated carbons, and total 2 to 35 parts by weight of one or more excipients are used. As the excipient, (a) the total 2 to 35 parts by weight of one or more excipients for tablet formulation, and (b) 0 to 33 parts by weight of the other excipients can be used.

Further preferably, in the method for preparing a tablet-formed pharmaceutical composition for oral administration of the present invention, a total combination of the spherical activated carbons and one or more excipients is 100 parts by weight. In particular, 65 to 97 parts by weight of the spherical activated carbons, and 3 to 35 parts by weight of the excipients are used. As the excipient, (a) 3 or more parts by weight of the excipients for tablet formulation is used in total. Further, (b) the other excipients may be used. That is to say, in the method for preparing a tablet-formed pharmaceutical composition for oral administration, 65 to 97 parts by weight of the spherical activated carbons, and the total 3 to 35 parts by weight of one or more excipients are used. As the excipient, (a) the total 3 to 35 parts by weight of one or more excipients for tablet formulation, and (b) 0 to 32 parts by weight of the other excipients can be used.

Further preferably, in the method for preparing a tablet-formed pharmaceutical composition for oral administration of the present invention, a total combination of the spherical activated carbons and one or more excipients is 100 parts by weight. In particular, 65 to 96 parts by weight of the spherical activated carbons, and 4 to 35 parts by weight of the excipients are used. As the excipient, (a) 4 or more parts by weight of the excipients for tablet formulation is used in total. Further, (b) the other excipients may be used. That is to say, in the method for preparing a tablet-formed pharmaceutical composition for oral administration, 65 to 96 parts by weight of the spherical activated carbons, and the total 4 to 35 parts by weight of one or more excipients are used. As the excipient, (a) the total 4 to 35 parts by weight of one or more excipients for tablet formulation, and (b) 0 to 31 parts by weight of the other excipients can be used.

Further preferably, in the method for preparing a tablet-formed pharmaceutical composition for oral administration of the present invention, a total combination of the spherical activated carbons and one or more excipients is 100 parts by weight. In particular, 65 to 95 parts by weight of the spherical activated carbons, and 5 to 35 parts by weight of the excipients are used. As the excipient, (a) 5 or more parts by weight of the excipients for tablet formulation is used in total. Further, (b) the other excipients may be used. That is to say, in the method for preparing a tablet-formed pharmaceutical composition for oral administration, 65 to 95 parts by weight of the spherical activated carbons, and the total 5 to 35 parts by weight of one or more excipients are used. As the excipient, (a) the total 5 to 35 parts by weight of one or more excipients for tablet formulation, and (b) 0 to 30 parts by weight of the other excipients can be used.

The solvent is added, in order to mix the spherical activated carbon and the excipient, and to form a thin film of the excipient for tablet formulation on a surface of the spherical activated carbon. Therefore, the solvent may be added to a mixture of the spherical activated carbon and the excipient, or a solvent in which the excipient for tablet formulation is preliminarily dissolved or dispersed, can be added to the spherical activated carbon. A volume of solvent is not particularly limited so long as the spherical activated carbon and the excipient are sufficiently mixed and the thin film of the excipient for tablet formulation is formed on the surface of the spherical activated carbon. In connection with this, the most amount of the solvent is removed from the tablet-type composition in the after-mentioned drying step (3). Thus, the amount of the solvent can be approximately adjusted according to the amount of the spherical activated carbon and the excipient, but is 10 parts or more by weight, preferably 10 to 100,000 parts by weight, more preferably 10 to 10,000 parts by weight, more preferably 10 to 1,000 parts by weight, further preferably 20 to 500 parts by weight, further preferably 30 to 300 parts by weight, most preferably 40 to 200 parts by weight. When the amount of solvent is less than 10 parts by weight, the spherical activated carbon may not be mixed sufficiently. Further, even if the amount of solvent is more than 100,000 parts by weight, the solvent can be removed in the drying step. Thus, but it is most preferable to add excessively large amount of the solvent because of economic reasons such as drying efficacy and the like.

Further, when a relatively large amount of solvent, such as 500 parts or more by weight of solvent, is used, the solvent can be removed by good filtration after the spherical activated carbon and excipient are homogeneously dispersed or dissolved by solvent.

The solvent, which may be used in the method for preparing a tablet-formed pharmaceutical compositiontablet-formed pharmaceutical composition for oral administration, is not particularly limited. For example, as the solvent, an aqueous solvent, an organic solvent and the like can be used. Specifically, the possibilities include water (in particular, purified water), ethanol, isopropanol, methylene chloride, or a mixture thereof. However, purified water is preferable.

In the forming step (2) of the preparing method of the present invention, the resulting mixed product (slurry) is charged to a forming die so as to make a certain formation. The forming of the tablet can be carried out by pressing the slurry in the forming die. Alternatively, the tablet can be formed using a tableting machine at low pressure. The pressure is not particularly limited, so long as a rate of destroyed spherical activated carbon is less than 20 %.

In the drying step (3) of the preparing method of the present invention, the mixed product in the forming die is dried. A drying method is not particularly limited, so long as the solvent in the mixed product may be evaporated. However, as the drying method, there may be mentioned freeze drying, drying under reduced pressure, draught drying, or heat-drying.

Spherical activated carbon is used in the method for preparing a tablet-type composition for oral administration of the present invention. That is, for adsorbing harmful substances, the tablet-formed pharmaceutical composition for oral administration can be manufactured via the method of the present invention. In the method for preparing a tablet-formed pharmaceutical composition for oral administration, it is preferable that the drying method is performed under reduced pressure, in order to maintain the adsorption property of the spherical activated carbon.

The spherical activated carbon used as the adsorbent for an oral administration of the present invention exhibits an excellent adsorbability of exacerbation factors of liver diseases or harmful substances of kidney diseases, and therefore, may be used as an adsorbent for an oral administration for treating or preventing kidney diseases or liver diseases.

Regarding kidney diseases, there may be mentioned, for example, chronic renal failure, acute renal failure, chronic pyelonephritis, acute pyelonephritis, chronic nephritis, acute nephritic syndrome, acute progressive nephritic syndrome, chronic nephritic syndrome, nephrotic syndrome, nephrosclerosis, interstitial nephritis, tubulopathy, lipoid nephrosis, diabetic nephropathy, renovascular hypertension, or hypertension syndrome, or secondary kidney diseases caused by these primary diseases, or a light renal failure before a dialysis therapy, and may be used in an improvement of a light renal failure before a dialysis therapy or a disease condition for a patient during a dialysis therapy (see "Clinical Nephrology", Asakura-shoten, Nishio Honda, Kenkichi Koiso, and Kiyoshi Kurokawa, 1990; and "Nephrology" Igaku-shoin, Teruo Omae and Sei Fujimi, ed., 1981).

As for liver diseases, there may be mentioned, for example, fulminant hepatitis, chronic hepatitis, viral hepatitis, alcoholic hepatitis, hepatic fibrosis, liver cirrhosis, hepatic cancer, autoimmune hepatitis, drug allergic hepatopathy, primary biliary cirrhosis, tremor, encephalopathia, dysbolism, or dysfunction. Further, the porous spherical carbonaceous substance can be used in a treatment of a disease caused by toxic substances in a body, such as psychosis.

Therefore, when the adsorbent composition for oral administration according to the present invention is used as an agent for treating kidney diseases, the surface-modified spherical activated carbon is contained therein as an active ingredient. When the adsorbent composition for oral administration according to the present invention is used as an agent for a treatment of liver or kidney diseases, a dosage thereof depends on the subject (human or other animal), age, individual differences, disease conditions, and so on. Therefore, in some cases, a dosage outside of the following dosage may be appropriate, but in general, the oral dosage in the case of a human is usually 1 to 20 g of spherical activated carbon per day, wherein the daily dosage may be divided into three to four portions. The dosage may appropriately vary with the disease conditions. For example, when the Kremezin is used as the agent for treating kidney diseases, the oral dosage is 6 g of the spherical activated carbon per day. As the daily dosage is divided into three portions, a single dosage is 2 g. Therefore, when the tablet-type composition contains 500 mg of the spherical activated carbon per one tablet, 4 tablets per single dosage are administered, and thus 12 tablets per day are administered.

EXAMPLES

The present invention now will be further illustrated by the following Examples.

In these Examples, bulk density, bulk volume in the case that the spherical activated carbons contained in the tablet-type composition are closely packed, hardness of tablet-type composition, friability of tablet-type composition, spherical particle rate of spherical activated carbon in tablet-type composition, and adsorption rate test of indole are measured by the following methods. In the following methods, the spherical activated carbon is measured.

(1) Bulk density (Tap density)

A sample of 20 g of spherical activated carbon was charged to 50 mL of graduated cylinder, and the graduated cylinder was tapped 50 times. Subsequently, a tap density was calculated by dividing the weight of the sample by the volume thereof. The measurement value (i.e. the bulk density described in Manufacturing Examples 2) obtained by such method was not different from a measurement value obtained by a method for measuring packing density in accordance with JIS K 1474-5.7.2 in a significant figure.

(2) Bulk volume in the case that spherical activated carbons contained in tablet-type composition were closely packed

The "bulk volume in the case that spherical activated carbons contained in tablet-type composition were closely packed ($V_2$)" was calculated, per tablet, by dividing the weight of the spherical activated carbon used in a tablet by the tap density obtained in the above method.

(3) Hardness of tablet-type composition

A hardness of the tablet-type composition was measured using a tablet hardness tester (TBH320, ERWEKA). A thickness of the tablet to be tested was measured. The obtained thickness was put into the hardness tester, and then the hardness was measured at room temperature. Measurement conditions were as follows.

Table 5

| Measurement conditions | |
| --- | --- |
| Measurement mode | Constant Speed |
| Compaction rate | 2.3 mm / s |

The sample number in each test was one.

(4) Friability of tablet-type composition

The friability was measured based on the tablet friability test of general information of "The Japanese Pharmacopoeia Fifteenth Edition", as follows.

1 to 6.5 g of tablet-type compositions was prepared. Powders attached to surfaces of the tablet-type compositions were removed before measurement, and then a weight of the tablet-type compositions was precisely measured. The tablet-type compositions were put into a drum. The drum was rotated a hundred times, and the tablet-type compositions were taken out therefrom. In the same manner as the previous measurement, powders attached to surfaces of the tablet-type compositions were removed, and then a weight of the tablet-type compositions was precisely measured. The equation of friability was as follows.

(Calculus equation)

```
Friability(%)=(Weight of tablet-type compositions before
measurement(g)- Weight of tablet-type composition after
measurement (g))/ Weight of tablet-type compositions before
measurement (g)×100
```

(5) Disintegration test of tablet-type composition

A disintegration test was carried out using a disintegration tester (NT-20H, TOYAMA SANGYO CO.,LTD.) based on the disintegration test of Japanese Pharmacopoeia, as follows.

As a test liquid, water was used. Six tablet-type compositions were put into each six glass tube of the disintegration tester, respectively. Then, disks were also put thereinto, and the glass tubes were moved up and down for 30 minutes. Subsequently, the tablet-type compositions in the glass tubes were examined for a degree of disintegration. The term "disintegration" is defined as a condition characterized by a form of the tablet-type composition completely disappearing in the glass tube or a condition characterized by the tablet-type composition clearly becoming a soft substance without original form, though a residue remains in the glass tube.

(6) Spherical particle rate of spherical activated carbon in tablet-type composition

(Measurement using particle shape image analyzer)

A spherical particle rate of the spherical activated carbon contained in the tablet-type composition can be measured by a particle shape image analyzer (PITA-2, SEISHIN ENTERPRISE CO.,LTD.). The measurement method was as follows.

In order to separate spherical activated carbons, including destroyed particles from the tablet-type composition, the following pretreatment was carried out. The 1.5 g to 2.5 g of tablets were put in 50 mL of plastic centrifuging tube, and further 50 mL of purified water was added thereto. The tablets were disintegrated and dispersed by a sonication for 10 minutes. Then, the spherical activated carbons were collected by filtration through a membrane filter. The collected spherical activated carbons were dried at 105 °C, for 4 hours, to obtain a sample to be tested. In order to prevent a clogging of a device by an aggregate, if necessary, the aggregates were removed by an appropriate sieve.

Conditions of measurement and analysis using particle shape image analyzer (PITA-2, SEISHIN ENTERPRISE CO.,LTD.) were as follows.

Table 6

| | Measurement conditions | |
| --- | --- | --- |
| Dispersion medium | Isopropanol |
| Object lens | $\times 2$ |
| Measurement range | 8~1500 $\mu$m |
| Number of measured particles | 500 or more (0.1 g) |

Analysis conditions

- The particles having a degree of circularity of less than 0.6, and the particles having a maximum length of D90 (accumulating 90 % particle-sizes; D90 was separately measured by wet-type laser difraction method) i.e. 473.6 $\mu$m or more, and a degree of circularity of less than 0.925, are considered as an aggregate. Such particles was removed from objects to be analyzed.

- The particles having a degree of circularity of 0.925 or more was considered as particles maintaining spherical form, and the particles having a degree of circularity of less than 0.925 was considered as particles not maintaining spherical form.

- Volume of each particle is calculated by the following equation.

```
Volume = 4/3 x π x (1/2 x equivalent circle diameter)³
```

(continued)

| Measurement conditions |
| --- |
| ■ Spherical particle rate (measured value) (%) was calculated by the following equation.<br><br>Spherical particle rate (measured value) (%) = (Total volume of particles maintaining spherical form / Total volume of all particles) x 100<br><br>■ Additionally, spherical particle rate (measured value) (%) of a standard preparation, wherein spherical activated carbons and crushed products of spherical activated carbons are mixed at a rate of nine-to-one, five-to-five, or ten-to-zero was measured, and thereby primary regression formula (y = ax + b) of true value X and measured value y is calculated. The spherical particle rate (true value) (%) of spherical activated carbons contained in the tablet-type composition is calculated according to the primary regression formula. |

(Measurement using charge-coupled device camera)

In Comparative example 16, the spherical particle rate of the spherical activated carbon in the tablet-type composition was measured according to a simplified method using a charge-coupled device camera (hereinafter, referred to as a CCD camera). In order to separate spherical activated carbons, including destroyed particles from the tablet-type composition, the following pretreatment was carried out. Six tablets were put in 50 mL of plastic centrifuging tube, and further 50 mL of purified water was added thereto. The tablets were disintegrated and dispersed by a sonication for 10 minutes. The mixture was centrifuged at 3500 rpm for 5 minutes, and then a supernatant was removed. A precipitate was dispersed in an appropriate amount of water, and subsequently the spherical activated carbons were collected by filtering through a membrane filter. The collected spherical activated carbons were dried at 105 °C, for 4 hours, to obtain a sample to be tested. The resulting spherical activated carbons were spread out on a sheet of white paper, and 5 fields were photographed by CCD camera. Then, the number of spherical activated carbons maintaining spherical form, and the number of broken particles in a field were measured, respectively. For comparison, the spherical activated carbon before making the tablet was treated in a similar way, and the number of spherical activated carbons maintaining spherical form and the number of broken particles in a field were measured, respectively. The results are shown in Tables 11 and 13.

(7) Adsorption rate test of indole

The adsorption rate test of indole of the tablet-type composition was performed using a dissolution tester described in Japanese Pharmacopoeia, as described below.

A test liquid was prepared by dissolving 0.1 g of indole with 1 L of a 2nd Fluid for dissolution test. The test liquid was heated at 41 °C, and degassed by filtering via suction through a 0.45 $\mu$m membrane filter. 900 mL of the degassed test liquid was charged to a vessel of the dissolution tester and was allowed to stand to 37 °C. A capsule containing 200 mg of the spherical activated carbon or a tablet-type composition containing 200 mg of the spherical activated carbon was added to the vessel of the dissolution tester, and was rotated at 100 rpm so as to start the adsorption test. After 1 hour, 10 mL of the test liquid was collected and filtered through 0.45 $\mu$m membrane filter. The first 5 mL of test liquid was discarded, and the remaining filtrate was collected as a sample solution. An indole concentration of the sample solution was quantified using high-performance liquid chromatography. The condition of analysis was as follows.

Table 7

| Analysis conditions | |
| --- | --- |
| Column | CAPCELL PAK C18 UG120 5 $\mu$m 4.6 mmI.D.×150 mm (Shiseido CO.,LTD.) |
| Mobile phase | Water/Methanol mixture (1:1) |
| Temperature of column | 40°C |
| Flow rate | 1.0 mL/min |
| Detection wavelength | 238 nm |
| Volume of applied sample | 10 $\mu$L |

An adsorbed amount of indole was shown by the "Indole relative adsorption rate". The "Indole relative adsorption rate" was expressed as a percent of an adsorbed amount of indole of the present invention when an adsorbed amount of indole of the capsule of adsorbent for oral administration was scaled to 100 %.

(8) Measurement of weight-average molecular weight of excipient

The weight-average molecular weight of the excipient for tablet formulation can be measured by the following method.

10 mg of an excipient for tablet formulation was dissolved in 1 mL of distilled water, and the mixture was applied to a high performance size exclusion chromatography under the following analysis condition. A weight-average molecular weight was determined by applying the obtained measurement value to a standard curve prepared using pullulan as a known weight-average molecular weight.

Table 8

| Analysis conditions | |
|---|---|
| Column | TSKgel G2000SWXL 7.8 mmI.D.×300 mm(TOSOH Corp.) |
| | TSKgel G3000SWXL 7.8 mmI.D.×300 mm(TOSOH Corp.) or TSKgel G4000SWXL 7.8 mmI.D.×300 mm(TOSOH Corp.) |
| Mobile phase | Water |
| Temperature of column | 40°C |
| Flow rate | 1.0 mL/min |
| Detection | Refractive index detector |
| Volume of applied sample | 10 $\mu$L |

The weight-average molecular weights of excipients were shown in Table 15.

(9) Measurement of thin film-forming ability of excipient

A thin film-forming ability of an excipient for tablet formulation was examined in accordance with the following method in a general laboratory.

[Test method]

1) An adhesive tape made of fluorine resin (NITOFLON Tape; NITTO DENKO Corp.) was stuck to the heat plate surface of a stirrer with heater in an area (12 cm x 12 cm).
2) A water solution or a water dispersion of the excipient for tablet formulation of 1 % by weight was prepared. The purified water can be heated for dissolution or dispersion.
3) 0.5 mL of the resulting water solution or water dispersion was put on the adhesive tape made of fluorine resin.
4) The temperature of the heater was set at about 125 °C, and the water solution or water dispersion was heat-dried for 10 minutes.

[Judgment]

5) Tweezers were inserted between the adhesive tape made of fluorine resin and a thin film, and the thin film was gently peeled away.
6) When a sheet of thin film can be peeled away from the adhesive tape made of fluorine resin, it is determined that a thin film is indeed formed. If a judgment cannot be carried out within 10 minutes, the judgment would be carried out after heat-drying for 20 minutes. That is, if the thin film cannot be formed within 10 minutes or the thin film cannot be peeled away within 10 minutes, the judgment would be carried out after heat-drying for 20 minutes. However, if a formation of thin film and a separation of thin film cannot be observed by heat-drying for 30 minutes, it is determined that an excipient does not have a thin film-forming ability.
FIG. 3 shows a photograph of polyvinyl alcohol which can form a thin film (A) and a photograph of carmellose which cannot form a thin film (B). Further, the excipients for tablet formulation with thin film-forming ability and the excipients without thin film-forming ability are shown in Table 15 as a whole.

<<Manufacturing Example 1: Preparation of porous, spherical carbonaceous substance >>

The porous, spherical carbonaceous substance was obtained by a method similar to the method described in Example 1 of Japanese Patent No. 3522708 (Japanese Unexamined Patent Publication (Kokai) No. 2002-308785). The specific procedure was as follows.

Petroleum pitch (68 kg) (softening point = 210 °C; quinoline insoluble contents = not more than 1 % by weight; ratio of hydrogen atoms/carbon atoms = 0.63) and naphthalene (32 kg) were put into an autoclave (internal volume = 300 L) equipped with stirring fans, melted at 180 °C, and mixed. The mixture was extruded at 80 to 90 °C to form string-like products. Then, the string-like products were broken so that a ratio of diameter to length became about 1 to 2.

The resulting broken products were added to an aqueous solution prepared by dissolving 0.23 % by weight of polyvinyl alcohol (saponification value = 88 %) and heating to 93 °C, and dispersed by stirring to be spheroidized. Then, the whole was cooled by replacing the aqueous polyvinyl alcohol solution with water, at 20 °C and left for 3 hours, whereby the pitch was solidified and naphthalene crystals were precipitated, and a slurry of spherical-shaped products of pitch was obtained.

After most of the water was removed by filtration, the naphthalene in the pitch was extracted and removed with n-hexane at an amount of about 6 times that of the spherical-shaped products of pitch. The resulting porous, spherical pitch was heated to 235 °C by the passing of heated air in a fluidized bed, and allowed to stand at 235 °C for 1 hour, to be oxidized, and a porous, spherical oxidized pitch was obtained, which is not fusible by heat.

Thereafter, the resulting porous, spherical oxidized pitch was activated in a fluidized bed at 900 °C for 170 minutes using a nitrogen gas atmosphere containing 50 % by volume of steam to obtain a spherical activated carbon. Further, the resulting spherical activated carbon was oxidized in the fluidized bed at 470 °C for 195 minutes using a nitrogen-oxygen atmosphere containing 18.5 % by volume of oxygen, and reduced in the fluidized bed at 900 °C for 17 minutes using a nitrogen gas atmosphere, to obtain a porous, spherical carbonaceous substance. The resulting porous spherical carbonaceous substance was used as a spherical activated carbon in Pharmacological Experiments as mentioned below.

The properties of the resulting surface-modified spherical activated carbon are as follows.

Specific surface area = 1300 $m^2$/g(BET method);
Volume of pores = 0.08 mL/g
(Volume of pores having a diameter of 20 to 15000 nm determined by a mercury press-injection method);
Average particle diameter = 350 $\mu$m;
Total amount of acidic groups = 0.67 meq/g; and
Total amount of basic groups = 0.54 meq/g.
Fracture strength = 31.2 MPa; and
Distortion factor when 2 MPa of pressure is applied =0.7 %.

<<Manufacturing Example 2: Preparation of porous, spherical carbonaceous substance >>

The porous, spherical carbonaceous substance (surface-modified spherical activated carbon) was obtained by a method similar to the method described in Example 1 of Japanese Unexamined Patent Publication (Kokai) No. 2005-314416). The specific procedure was as follows.

Deionized water (220 g) and methylcellulose (58 g) were put into a 1 L separable flask. 105 g of styrene, 184 g of divinyl benzene with a purity of 57 % (57 % divinylbenzene and 43 % ethylvinyl benzene), 1.68 g of 2,2'-azobis(2,4-dimethylvaleronitrile), and 63 g of 1-butanol as a porogen were added thereto. Then, the atmosphere was replaced with a nitrogen gas. The two-phase system was stirred at 200 rpm, and heated to 55 °C, and then allowed to stand for 20 hours. The resulting resin was filtered, and dried in a rotary evaporator. In a vacuum dryer, 1-butanol was removed from the resin by distillation, and the product was dried under a reduced pressure at 90 °C for 12 hours to obtain a spherical, porous synthetic resin having an average particle diameter of 180 $\mu$m. A specific surface area of the porous synthetic resin was about 90 $m^2$/g.

The resulting spherical porous synthetic resin (100 g) was put into a reactor, which had a grating, and treated to impart infusibility in a vertical, tubular furnace. The infusibility-imparting treatment was carried out under the conditions that dried air (3 L/min) was upwardly passed from the lower portion of the reactor tube, the temperature was raised to 260 °C at a rate of 5 °C/h, and the whole was allowed to stand at 260 °C for 4 hours to obtain a spherical, porous oxidized resin. The resulting spherical, porous oxidized resin was heat-treated at 600 °C for 1 hour under a nitrogen atmosphere, and then activated in a fluidized bed at 820 °C for 10 hours under a nitrogen gas atmosphere containing 64.5 % by volume of steam, to obtain a spherical activated carbon.

The resulting spherical activated carbon was oxidized in the fluidized bed at 470 °C for 195 minutes under a nitrogen-oxygen atmosphere containing 18.5 % by volume of oxygen, and reduced in the fluidized bed at 900 °C for 17 minutes under a nitrogen gas atmosphere, to obtain a surface-modified, spherical activated carbon. The properties of the resulting surface-modified, spherical activated carbon are as follows.

Specific surface area = 1763 m$^2$/g(BET method);

Volume of pores = 0.05 mL/g

(Volume of pores having a diameter of 20 to 15000 nm determined by a mercury press-injection method);

Average particle diameter = 111 μm(Dv50);

Total amount of acidic groups = 0.59 meq/g; and

Total amount of basic groups = 0.61 meq/g.

Bulk density = 0.50g/cm$^3$;

Fracture strength = 436.5 MPa

Distortion factor when 2 MPa of pressure is applied =0.2 %

<<Example 1>>

In this example, a tablet-formed pharmaceutical composition for oral administration was produced by using pregelatinized starch as an excipient for tablet formulation.

10 g (20 cm$^3$) of the spherical activated carbons obtained in the above-mentioned Preparation Example 2 and 0.3 g of completely pregelatinized starch were homogeneously dispersed in a beaker, and 12 mL of purified water was further added. The obtained mixture was mixed by using a spatula so that an unmixed lump of the excipient would not be formed. The prepared mixed product (slurry) was filled in a shaping die (diameter 13 mm, depth 8 mm) and leveled with a spatula, and the surface of the tablet was arranged. The shaping die was set in a freeze dryer and dried under a reduced pressure at 25 °C to 40 °C and a pressure of $1.5\times10^{-1}$ Pa for 5 hours or more. The specific temperature conditions and times are as follows.

<div align="center">Table 9</div>

| Conditions of drying under reduced pressure | | |
|---|---|---|
| | Temperature(°C) | Time(h) |
| Drying under reduced pressure | 25→40 | 0.5 |
| | 40 | 4 |
| | 40→25 | 0.5 |
| | 25 | Appropriately determined |

After the drying under a reduced pressure, a tablet-type composition was taken out of the shaping die. The obtained tablet-formed pharmaceutical composition for oral administration 1 had a volume of 1.1 cm$^3$. The average mass of 8 tablets of the obtained tablet-type composition was 0.5023 g, and the spherical activated carbon included therein had a mass of 0.4877 g. Since the spherical activated carbon used had a bulk density ($V_2$) in a close-packed state of 0.98 cm$^3$ and the tablet-type composition had a volume ($V_1$) of 1.1 cm$^3$, $V_1/V_2$ was 1.13 (Table 12) .

Furthermore, the hardness and friability of the tablet-formed pharmaceutical composition for oral administration, and the sphericity of the spherical activated carbon were measured by a particle shape image analyzer. The results are shown in Table 13. The tablet-formed pharmaceutical composition for oral administration had a hardness of 30.2 N and a friability of 4.6 %, and thus was excellent. Furthermore, the sphericity was 100 %, and the breaking of the spherical activated carbon was not observed at all. In addition, the time for the disintegration of the composition obtained in this example was within 30 minutes.

<<Examples 2 to 34>>

In Examples 2 to 34, each tablet-formed pharmaceutical composition for oral administration was produced by changing the kind and content of the excipient for tablet formulation. The kinds and contents of the excipients for tablet formulation used are shown in Table 12.

1 g (2 cm$^3$) of the spherical activated carbons obtained in the above-mentioned Preparation Example 2 and an excipient for tablet formulation were homogeneously dispersed in a beaker, and purified water (the amount was shown in Table 12) was added. The obtained mixture was mixed by using a spatula so that an unmixed lump of the excipient would not be formed. The prepared mixed product (slurry) was filled in a shaping die (diameter 13 mm, depth 8 mm) and leveled with a spatula, and the surface of the tablet was arranged. Drying under a reduced pressure was conducted under the same conditions as those of Example 1.

The masses, volumes, $V_1/V_2$s and the like of the obtained tablet-formed pharmaceutical compositions for oral administration 2 to 34 are shown in Table 12, and the hardnesses, friabilities, and sphericities using a particle shape image analyzer, and the like are shown in Table 13. $V_1/V_2$s were 1.31 or less, and hardnesses and friabilities were also excellent.

«Examples 35, 36, and 38~42>>

In Examples 35, 36 and 38 to 42, each tablet-formed pharmaceutical composition for oral administration was produced by changing the kind and content of the excipient for tablet formulation, and by changing the drying under a reduced pressure to freeze drying. The kinds and contents of the excipients for tablet formulation used are shown in Table 12.

1 g (2 cm$^3$) of the spherical activated carbons obtained in the above-mentioned Preparation Example 2 and an excipient for tablet formulation were homogeneously dispersed in a beaker, and purified water (the amount was shown in Table 12) was added. The obtained mixture was mixed by using a spatula so that an unmixed lump of the excipient would not be formed. The prepared mixed product (slurry) was filled in a shaping die (diameter 13 mm, depth 8 mm) and leveled with a spatula, and the surface of the tablet was arranged. The shaping die was set in a freeze dryer, and freeze drying was conducted at -50 °C to 40 °C and a pressure of $1.5\times10^{-1}$ Pa for 8 hours or more. The specific temperature conditions and times were as follows.

Table 10

| Conditions of freeze drying | | |
|---|---|---|
| Steps | Temperature(°C) | Time(h) |
| Freeze | -50 | 3 |
| Drying Under Reduced pressure | -50→40 | 3 |
| | 40 | 4 |
| | 40→25 | 0.5 |
| | 25 | Appropriately determined |

The masses, volumes, $V_1/V_2$s and the like of the obtained tablet-formed pharmaceutical compositions for oral administration 35, 36 and 38 to 42 are shown in Table 12, and the hardnesses, friabilities, and sphericities using a particle shape image analyzer , and the like are shown in Table 13. In Examples 35, 36 and 38 to 42, $V_1/V_2$s were 1.28 or less, and hardnesses and friabilities were also excellent.

<<Example 37>>

In Example 37, a tablet-formed pharmaceutical composition for oral administration was produced by using starch pregelatinized by heating corn starch.

0.1 g of corn starch was dissolved in 1.2 mL of heated purified water, and 1 g (2 cm$^3$) of the spherical activated carbons obtained in the above-mentioned Preparation Example 2 was added. The obtained mixture was mixed by using a spatula. The prepared mixed product (slurry) was filled in a shaping die (diameter 13 mm, depth 8 mm) and leveled with a spatula, and the surface of the tablet was arranged. Freeze drying was conducted under the same conditions as those of Examples 35, 36 and 38 to 42 to obtein adsorbent composition for oral administration 37.

The mass, volume, and $V_1/V_2$ and the like of the obtained tablet-formed pharmaceutical composition for oral administration 37 are shown in Table 12, and the hardness, friability, and sphericity using a particle shape image analyzer, and the like are shown in Table 13.

<<Example 43>>

10 g (20 cm$^3$) of the spherical activated carbons obtained in the above-mentioned Preparation Example 2 and 0.5 g of completely pregelatinized starch were homogeneously dispersed in a beaker, and 12 mL of purified water was further added. The obtained mixture was mixed by using a spatula so that an unmixed lump of the excipient would not be formed. The prepared mixed product (slurry) was filled in a shaping die (diameter 10 mm, height 6 mm, radius of curvature 14 mm) and leveled with a spatula, and the surface of the tablet was arranged by slightly compressing the upper part with a shaping rod attached to a stirrer. Drying under a reduced pressure was conducted under the same conditions as those of Example 1 to obtein adsorbent composition for oral administration 43.

The indole adsorption velocity of the obtained tablet-formed pharmaceutical composition for oral administration was measured. The indole relative adsorption ratio after 1 hour is shown in Table 14. The indole relative adsorption ratio was expressed by the percentage of the indole adsorption amount of the adsorbent composition for oral administration in the case when the adsorption amount of a capsule agent as a control was defined as 100.

<<Examples 44 to 48>>

1 g (2 cm$^3$) of the spherical activated carbons obtained in the above-mentioned Preparation Example 2, and an excipient (the kind and amount of the excipient used are shown in Table 14) were homogeneously dispersed in a beaker, and 1.2 mL of purified water was further added. The obtained mixture was mixed by using a spatula so that an unmixed lump of the excipient would not be formed. The prepared mixed product (slurry) was filled in a shaping die (diameter 13 mm, depth 8 mm) so as to have an amount corresponding to 0.2 g of the spherical activated carbon, and the surface of the tablet was arranged by slightly compressing the upper part by a spatula. Drying under a reduced pressure was conducted under the same conditions as those of Example 1 to obtein adsorbent compositions for oral administration 44 to 48.
The indole adsorption velocities of the obtained tablet-formed pharmaceutical compositions for oral administration 44 to 48 were measured. The indole relative adsorption ratios after 1 hour were shown in Table 14.

<<Examples 49 to 54>>

1 g (2 cm$^3$) of the spherical activated carbons obtained in the above-mentioned Preparation Example 2, and an excipient (the kind and amount of the excipient used are shown in Table 14) were homogeneously dispersed in a beaker, and purified water (the amount was shown in Table 14) was further added. The obtained mixture was mixed by using a spatula so that an unmixed lump of the excipient would not be formed. The prepared mixed product (slurry) was filled in a shaping die (diameter 13 mm, depth 8 mm) so as to have an amount corresponding to 0.2 g of the spherical activated carbons, and the surface of the tablet was arranged by slightly compressing the upper part by a spatula. Freeze drying was conducted under the same conditions as those of Examples 35 to 42 to obtein adsorbent compositions for administration 49 to 54. The indole adsorption velocities of the obtained tablet-formed pharmaceutical compositions for oral administration 49 to 54 were measured. The indole relative adsorption ratios after 1 hour were shown in Table 14.

<<Example 55>>

10 g (20 cm$^3$) of the spherical activated carbons obtained in the above-mentioned Preparation Example 2 and 0.3 g of pullulan were homogeneously dispersed in a beaker, and 12 mL of purified water was further added. The obtained mixture was mixed by using a spatula so that an unmixed lump of the excipient would not be formed. The prepared mixed product (slurry) was filled in a shaping die (diameter 10 mm, height 6 mm, radius of curvature 14 mm) and leveled with a spatula, and the surface of the tablet was arranged by slightly compressing the upper part with a shaping rod attached to a stirrer. Drying under a reduced pressure was conducted under the same conditions as those of Example 1 to obtein adsorbent composition for oral administration 55. The mass, volume, and $V_1/V_2$ and the like of the obtained tablet-formed pharmaceutical composition for oral administration are shown in Table 12, and the hardness, friability, and the sphericity using a particle shape image analyzer were shown in Table 13. Furthermore, the indole adsorption velocity was measured, and the indole relative adsorption ratio after 1 hour was shown in Table 14.
The indole relative adsorption ratios of the adsorbent compositions for oral administration 43 to 55 obtained in Examples 43 to 55 were 81 % to 106 % of that of a capsule agent, and no decrease was observed in the indole adsorption amount by forming the spherical activated carbon into a tablet, and the performance for adsorbing harmful substances was maintained.

<<Example 56>>

Adsorbent composition for oral administration 56 was obtained by repeating the operations in Example 43, except that 10 g (20 cm$^3$) of the spherical activated carbons obtained in the above-mentioned Preparation Example 1 and 0.75 g of pullulan were used.
The mass, volume, and $V_1/V_2$ and the like of the obtained tablet-formed pharmaceutical composition for oral administration were shown in Table 12, and the hardness, friability, and the sphericity using a particle shape image analyzer were shown in Table 13.

<<Examples 57 to 68>>

In Examples 57 to 68, tablet-formed pharmaceutical compositions for oral administration were produced by using plural kinds of excipients for tablet formulation. The kinds and contents of the excipients for tablet formulation used are shown in Table 12.

1 g (2 cm$^3$) of the spherical activated carbons obtained in the above-mentioned Preparation Example 2 and the excipients for tablet formulation were homogeneously dispersed in a beaker, and purified water (the amount was shown in Table 12) was further added. The obtained mixture was mixed by using a spatula so that an unmixed lump of the excipients would not be formed. The prepared mixed product (slurry) was filled in a shaping die (diameter 13 mm, depth 8 mm) and leveled with a spatula, and the surface of the tablet was arranged. Drying under reduced pressure was conducted under the same condition as that in Example 1.

The masses, volumes, $V_1/V_2$s and the like of the obtained tablet-formed pharmaceutical compositions for oral administration 57 to 68 were shown in Table 12, and the hardnesses, friabilities, and sphericities using a particle shape image analyzer, and the like were shown in Table 13.

<<Example 69>>

In this example, a tablet-formed pharmaceutical composition for oral administration comprising 68 % by weight of spherical activated carbon was produced. 1 g (2 cm$^3$) of the spherical activated carbons obtained in the above-mentioned Preparation Example 2, and pullulan and microcrystalline cellulose (the amounts were shown in Table 12) were homogeneously dispersed in a beaker, and purified water (the amount was shown in Table 12) was further added. The obtained mixture was mixed by using a spatula so that an unmixed lump of the excipient would not be formed. The prepared mixed product (slurry) was filled in a shaping die (diameter 10 mm, height 6 mm, radius of curvature 14 mm) and leveled with a spatula, and the surface of the tablet was arranged by slightly compressing the upper part with a shaping rod attached to a stirrer. Drying under a reduced pressure was conducted under the same conditions as those of Example 1 to obtein an adsorbent composition for oral administration. The mass, volume, and $V_1/V_2$ and the like of the obtained tablet-formed pharmaceutical composition for oral administration were shown in Table 12, and the hardness, friability, and the sphericity using a particle shape image analyzer were shown in Table 13.

<<Comparative Example 1>>

In this comparative example, a tablet-type composition for oral administration comprising 30% by weight of spherical activated carbons was produced by using maltitol (molecular weight: 344.31), which is an excipient described in Patent Document 3. Reduced maltose starch syrup (AMALTY MR-50: manufactured by Mitsubishi Shoji Foodtech CO.,LTD.) was used as the maltitol. Maltitol is an excipient having a molecular weight of 10,000 or less, which does not have "thin film-formability".

50 g of the reduced maltose starch syrup was dissolved in 50 mL of purified water under heating. The amount of the solution after the dissolution was 80 mL, and the concentration of the reduced maltose starch syrup in the solution was 0.625 g/mL. 7.84 mL of the reduced maltose starch syrup solution (reduced maltose starch syrup 4.9 g) was added to 2.1 g of two sachets of "Kremezin (registered trademark) fine granule package 2 g" and sufficiently mixed in a beaker. The obtained mixed product was filled in a shaping die made of Teflon (registered trademark) (diameter 10 mm, height 6 mm, radius of curvature 14 mm) and leveled with a spatula, and the surface of the tablet was arranged by compression molding under a low pressure. The obtained shaping die was dried by heating by a hot air circulation-type constant temperature oven at 60 °C overnight, and after the completion of the drying, the shaping die was allowed to cool in a desiccator for 30 minutes.

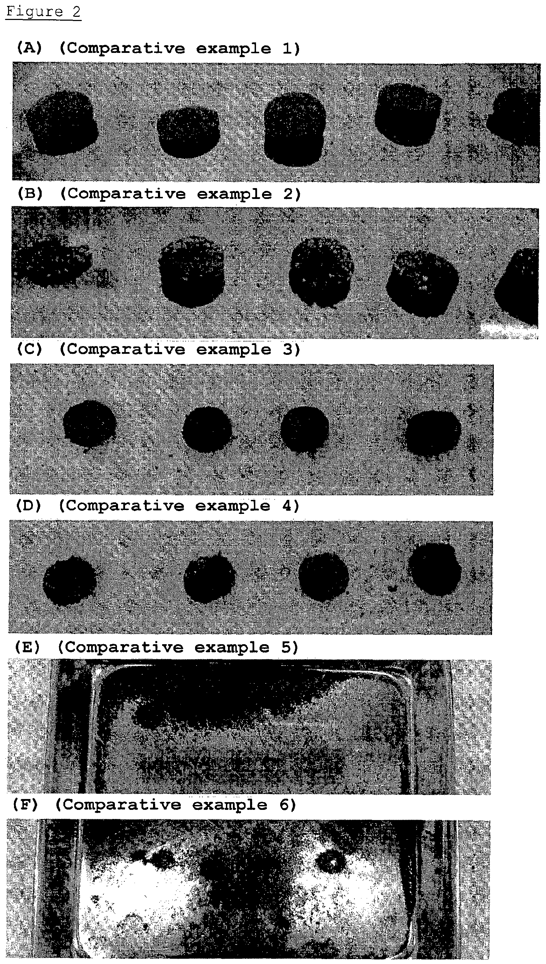

The photograph of the obtained tablet was shown in Fig. 2(A), and the physical properties and the like thereof were shown in Table 12 and Table 13. Since the obtained tablet was brittle and thus was disintegrated on contact, it was impossible to evaluate the physical properties. Furthermore, since the mixed product contained much moisture, it was difficult to homogeneously fill the mixed product in the shaping die, and the sizes of the dried tablets were not even.

<<Comparative Example 2>>

In this comparative example, a tablet-type composition for oral administration was produced by increasing the pressure for the compression molding in Comparative Example 1.

A tablet was produced by repeating the operations of Comparative Example 1, except that the compression molding under a low pressure in Comparative Example 1 was changed to compression molding for 1 minute by a strong force.

The photograph of the obtained tablet is shown in Fig. 2(B), and the physical properties and the like thereof were shown in Table 12 and Table 13. In the obtained tablet, sufficient hardness was not able to be obtained, and thus the hardness was not able to be measured. Furthermore, since the mixed product contained much moisture, it was difficult to homogeneously fill the mixed product in the shaping die, and the sizes of the dried tablets were not even.

<<Comparative Example 3>>

In this comparative example, a tablet-type composition for oral administration comprising 50% by weight of spherical activated carbons was produced by using maltitol.

A tablet was produced by repeating the operations of Comparative Example 1, except that 2.0 g of two sachets of "Kremezin (registered trademark) fine granule package 2 g" and 3.2 mL of a reduced maltose starch syrup solution (reduced maltose starch syrup 2 g) were used.

The photograph of the obtained tablet was shown in Fig. 2(C), and the physical properties and the like thereof were shown in Table 12 and Table 13. In the obtained tablet, only the upper part had been solidified, whereas the lower part was powdery and had not been solidified. The hardness and the like were not able to be measured.

<<Comparative Example 4>>

In this comparative example, a tablet-type composition for oral administration was produced by increasing the pressure for the compression molding in Comparative Example 3.

A tablet was produced by repeating the operations of Comparative Example 3, except that the compression molding under a low pressure in Comparative Example 3 was changed to compression molding by a strong force for 1 minute.

The photograph of the obtained tablet was shown in Fig. 2(D), and the physical properties and the like thereof were shown in Table 12 and Table 13. Similarly to the tablet of Comparative Example 8, only the upper part had been solidified, whereas the lower part was powdery and had not been solidified in the obtained tablet.

<<Comparative Example 5>>

In this comparative example, a tablet-type composition for oral administration comprising 70 % by weight of spherical activated carbons was produced using maltitol.

A tablet was produced by repeating the operations of Comparative Example 1, except that 2.1 g of two sachets of "Kremezin (registered trademark) fine granule package 2 g" and 1.44 mL of a reduced maltose starch syrup solution (reduced maltose starch syrup 0.9 g) were used.

The photograph of the obtained tablet was shown in Fig. 2(E), and the physical properties and the like thereof were shown in Table 12 and Table 13. The tablet was not solidified.

<<Comparative Example 6>>

In this comparative example, a tablet-type composition for oral administration was produced by increasing the pressure for the compression molding in Comparative Example 5.

A tablet was produced by repeating the operations of Comparative Example 5, except that the compression molding under a low pressure in Comparative Example 5 was changed to compression molding for 1 minute by a strong force.

The photograph of the obtained tablet was shown in Fig. 2(F), and the physical properties and the like thereof were shown in Table 12 and Table 13. The tablet was not solidified.

<<Comparative Example 7>>

In this comparative example, a tablet-type composition for oral administration comprising lower than 1 % by weight of an excipient for tablet formulation was produced.

A tablet was produced by repeating the operations of Example 43, except that 10 g (20 cm$^3$) of the spherical activated carbons obtained in the above-mentioned Preparation Example 2 and 0.05 g of pullulan were used.

The mass, volume, and $V_1/V_2$ and the like of the obtained tablet-formed pharmaceutical composition for oral administration were shown in Table 12, and the hardness, friability, and sphericity using a particle shape image analyzer, and the like were shown in Table 13.

<<Comparative Examples 8 to 10>>

In Comparative Examples 8 to 10, each tablet-type composition for oral administration was produced using an excipient having no thin film forming ability.

Tablets were produced by repeating the operations of Example 2, except that 1 g (2 cm$^3$) of the spherical activated carbons obtained in the above-mentioned Preparation Example 2, and Macrogol 6000, lactose hydrate or β-cyclodextrin, which was an excipient having a weight average molecular weight of lower than 10,000 (the kinds and amounts of the excipients used were shown in Table 12), were used.

The masses, volumes, $V_1/V_2$s and the like of the obtained tablet-formed pharmaceutical compositions for oral administration were shown in Table 12, and the hardnesses, friabilities, and sphericities using a particle shape image analyzer, and the like were shown in Table 13. The tablets were not solidified.

<<Comparative Examples 11 to 12>>

In Comparative Examples 11 to 12, each tablet-type composition for oral administration was produced using an excipient having no thin film forming ability.

Tablets were produced by repeating the operations of Example 2, except that 1 g (2 cm$^3$) of the spherical activated carbons obtained in the above-mentioned Preparation Example 2, and carmellose or carmellose calcium, which was an excipient that cannot form a thin film (the kinds and amounts of the excipients used were shown in Table 12), were used.

The masses, volumes, $V_1/V_2$s and the like of the obtained tablet-formed pharmaceutical compositions for oral administration were shown in Table 12, and the hardnesses, friabilities, and sphericities using a particle shape image analyzer, and the like were shown in Table 13. The tablets were not solidified.

<<Comparative Examples 13 to 15>>

In Comparative Examples 13 to 15, each tablet-type composition for oral administration was produced using an excipient having no thin film forming ability.

Tablets were produced by repeating the operations of Example 2, except that 1 g (2 cm$^3$) of the spherical activated carbons obtained in the above-mentioned Preparation Example 2, and a low-substituted hydroxypropylcellulose, polyethylene oxide or copolyvidone, which was an excipient that cannot form a thin film (the kinds and amounst of the excipients used were shown in Table 12), were used.

The masses, volumes, $V_1/V_2$s and the like of the obtained tablet-formed pharmaceutical compositions for oral administration were shown in Table 12, and the hardnesses, friabilities, and sphericities using a particle shape image analyzer, and the like were shown in Table 13.

«Comparative Example 16>>

In this comparative example, a tablet comprising spherical activated carbon was produced by a direct compression method.

10 g of the spherical activated carbons obtained in the above-mentioned Preparation Example 2, 37 g of lactose hydrate (DILACTOSE R, Freund Industrial Corp.), 2.5 g of povidone (KOLLIDON 30, manufactured by BASF) and 0.5 g of magnesium stearate (manufactured by Wako Pure Chemical Industries,Ltd.) were homogeneously mixed in a vinyl bag, and 135 tablets each having a diameter of 12 mm and an average weight of 375 mg were produced by a direct compression method using a single station tablet press (MINIPRESS MII, manufactured by RIVA). The masses, volumes, $V_1/V_2$s, hardnesses and friabilities were measured for the obtained tablet-formed pharmaceutical compositions for oral administration. The results were shown in Table 12 and Table 13.

Furthermore, the sphericity was measured for the adsorbent composition for oral administration obtained in Comparative Example 16, according to a method using a CCD camera. The results were shown in Table 12. In addition, the following Table 11 shows the data of the 5 visual fields before and after the tableting in Comparative Example 16.

Table 11

| | Area | Number of particle maintaining Spherical form in area | Number of broken particle in area | Spherical particle rate |
|---|---|---|---|---|
| Before tableting | 1 | 23 | 0 | 100% |
| | 2 | 19 | 0 | 100% |
| | 3 | 24 | 1 | 96% |
| | 4 | 28 | 0 | 100% |
| | 5 | 18 | 0 | 100% |
| After tableting | 1 | 11 | 33 | 25% |
| | 2 | 15 | 30 | 33% |
| | 3 | 10 | 35 | 22% |
| | 4 | 14 | 21 | 40% |
| | 5 | 10 | 31 | 32% |

<<Comparative Example 17>>

In this comparative example, a tablet comprising spherical activated carbon was produced by a directcompression method.

7.5 g of the spherical activated carbons obtained in the above-mentioned Preparation Example 1, 39.5 g of lactose hydrate, 2.5 g of povidone and 0.5 g of magnesium stearate were homogeneously mixed in a vinyl bag, and tablets each having a diameter of 10 mm and an average weight of 404.5 mg were produced by a direct compression method using a single station tablet press (MINIPRESS MII, manufactured by RIVA). The masses, volumes, $V_1/V_2$s, hardnesses and friabilities were measured for the obtained tablet-formed pharmaceutical compositions for oral administration. The results were shown in Table 12 and Table 13.

<<Comparative Example 18>>

In this comparative example, a tablet comprising spherical activated carbon was produced by a wet granule compression process.

50 g of the spherical activated carbons obtained in the above-mentioned Preparation Example 1, 24 g of lactose hydrate, 10 g of hydroxypropylcellulose, 12 g of povidone and 3 g of pullulan were mixed by a Tumbling fluidized bed granulating-coating machine (Multiplex Coater), and wet granulation was conducted. After the granulation, drying was conducted to obtein granules, and tablets each having a diameter of 10 mm and an average weight 250 mg was produced from the granules by using a single station tablet press (MINIPRESS MII, manufactured by RIVA). For the obtained tablet-formed pharmaceutical composition for oral administration, the mass, volume, $V_1/V_2$, hardness, friability, and the sphericity using a particle shape image analyzer of the obtained tablet-formed pharmaceutical composition for oral administration were measured. The results were shown in Table 12 and Table 13.

<<Comparative Example 19>>

In this comparative example, a tablet-formed pharmaceutical composition for oral administration having spherical activated carbon of lower than 65 % by weight was produced. 1 g (2 cm$^3$) of the spherical activated carbons obtained in the above-mentioned Preparation Example 2, and pullulan and microcrystalline cellulose (the amounts were shown in Table 12) were homogeneously dispersed in a beaker, and purified water (the amount was shown in Table 12) was further added. The obtained mixture was mixed by using a spatula so that an unmixed lump of the excipient would not be formed. The prepared mixed product (slurry) was filled in a shaping die (diameter 10 mm, height 6 mm, radius of curvature 14 mm) and leveled with a spatula, and the surface of the tablet was arranged by slightly compressing the upper part with a shaping rod attached to a stirrer. Drying under a reduced pressure was conducted under the same conditions as those of Example 1 to obtain an adsorbent composition for oral administration. The mass, volume, and

$V_1/V_2$ and the like of the obtained tablet-formed pharmaceutical composition for oral administration were shown in Table 12, and the hardness, friability, and the sphericity using a particle shape image analyzer were shown in Table 13.

Table 12-1

| | Spherical activated carbon | | Excipient | | | Solvent | Solvent mixture ratio (mL/g) | Volume of tablet ($V_1$) (cm³) | $V_1/V_2$ (Volume of tablet/ Bulk volume) | Average tablet mass (g) |
| | Weight (g) | Bulk volume ($V_2$) (cm³) | Name | Weight (mg) | Weight percent of excipient | Purified water (mL) | | | | |
|---|---|---|---|---|---|---|---|---|---|---|
| Example 1 | 0.4877 | 0.98 | Pregelatinized starch | 14.6 | 2.9 | 0.59 | 1.2 | 1.1 | 1.13 | 0.5023 |
| Example 2 | 0.4784 | 0.96 | Pregelatinized starch | 23.9 | 4.8 | 0.57 | 1.2 | 1.1 | 1.15 | 0.5023 |
| Example 3 | 0.4897 | 0.98 | Pregelatinized starch | 49.0 | 9.1 | 0.59 | 1.2 | 1.1 | 1.12 | 0.5387 |
| Example 4 | 0.4907 | 0.98 | Povidone | 14.7 | 2.9 | 0.59 | 1.2 | 1.1 | 1.12 | 0.5055 |
| Example 5 | 0.5289 | 1.06 | Pullulan | 15.9 | 2.9 | 0.63 | 1.2 | 1.1 | 1.04 | 0.5448 |
| Example 6 | 0.4751 | 0.95 | Oxidized starch | 47.5 | 9.1 | 0.57 | 1.2 | 1.1 | 1.16 | 0.5227 |
| Example 7 | 0.4676 | 0.94 | Hydroxypropylmethyicellulose 2208 (hypromellose) | 23.4 | 4.8 | 0.56 | 1.2 | 1.1 | 1.18 | 0.4910 |
| Example 8 | 0.4633 | 0.93 | Ghatti gum | 46.3 | 9.1 | 0.56 | 1.2 | 1.1 | 1.19 | 0.5097 |
| Example 9 | 0.4636 | 0.93 | Distarch phosphate | 46.4 | 9.1 | 0.56 | 1.2 | 1.1 | 1.19 | 0.5100 |
| Example 10 | 0.3428 | 0.69 | Polyvinyl alcohol-acrylic acid-methyl methacrylate copolymer | 17.1 | 4.8 | 0.41 | 1.2 | 0.9 | 1.31 | 0.3599 |
| Example 11 | 0.4262 | 0.85 | Gelatin | 12.8 | 2.9 | 0.51 | 1.2 | 1.1 | 1.29 | 0.4390 |
| Example 12 | 0.4128 | 0.83 | Gellan gum | 41.3 | 9.1 | 0.50 | 1.2 | 1.0 | 1.21 | 0.4540 |

| | Spherical activated carbon | | Excipient | | | Solvent | Solvent mixture ratio (mL/g) | Volume of tablet ($V_1$) ($cm^3$) | $V_1/V_2$ (Volume of tablet/ Bulk volume) | Average tablet mass (g) |
|---|---|---|---|---|---|---|---|---|---|---|
| | Weight (g) | Bulk volume ($V_2$) ($cm^3$) | Name | Weight (mg) | Weight percent of excipient | Purified water (mL) | | | | |
| Example 13 | 0.4954 | 0.99 | Hydroxypropyl starch | 24.8 | 4.8 | 0.59 | 1.2 | 1.1 | 1.11 | 0.5201 |
| Example 14 | 0.5012 | 1.00 | Hydroxypropylmethylcellulose 2906 (hypromellose) | 15.0 | 2.9 | 0.50 | 1.0 | 1.1 | 1.10 | 0.5162 |
| Example 15 | 0.4448 | 0.89 | Dextrin | 89.0 | 16.7 | 0.44 | 1.0 | 1.1 | 1.24 | 0.5337 |
| Example 16 | 0.4883 | 0.98 | Tara gum | 14.6 | 2.9 | 0.59 | 1.2 | 1.1 | 1.13 | 0.5029 |
| Example 17 | 0.4886 | 0.98 | Propylene glycol alginate | 14.7 | 2.9 | 0.59 | 1.2 | 1.1 | 1.13 | 0.5033 |
| Example 18 | 0.4840 | 0.97 | Hydroxypropylcellusose | 14.5 | 2.9 | 0.58 | 1.2 | 1.1 | 1.14 | 0.4985 |
| Example 19 | 0.4352 | 0.87 | Partly pregelatinized starch | 21.8 | 4.8 | 0.52 | 1.2 | 1.1 | 1.26 | 0.4569 |
| Example 20 | 0.4740 | 0.95 | Agar | 14.2 | 2.9 | 0.57 | 1.2 | 1.1 | 1.16 | 0.4883 |
| Example 21 | 0.4938 | 0.99 | Carrageenan | 24.7 | 4.8 | 0.59 | 1.2 | 1.1 | 1.11 | 0.5185 |
| Example 22 | 0.4610 | 0.92 | Methylcellulose | 23.0 | 4.8 | 0.46 | 1.0 | 1.1 | 1.19 | 0.4840 |
| Example 23 | 0.4542 | 0.91 | Glucomannan | 13.6 | 2.9 | 0.55 | 1.2 | 1.1 | 1.21 | 0.4678 |
| Example 24 | 0.4644 | 0.93 | Kanbai power | 32.5 | 6.5 | 0.56 | 1.2 | 1.1 | 1.18 | 0.4970 |

(continued)

| | Spherical activated carbon | | Excipient | | | Solvent | | Volume of tablet ($V_1$) (cm$^3$) | $V_1/V_2$ (Volume of tablet/ Bulk volume) | Average tablet mass (g) |
| | Weight (g) | Bulk volume ($V_2$) (cm$^3$) | Name | Weight (mg) | Weight percent of excipient | Purified water (mL) | Solvent mixture ratio (mL/g) | | | |
|---|---|---|---|---|---|---|---|---|---|---|
| Example 25 | 0.4690 | 0.94 | Microcrystalline cellulose carmellose sodium | 117.2 | 20.0 | 0.56 | 1.2 | 1.1 | 1.17 | 0.5862 |

Table 12-2

| | Spherical activated carbon | | Excipient | | | Solvent | Solvent mixture ratio (mL/g) | Volume of tablet ($v_1$) (cm³) | $V_1/V_2$ (Volume of tablet/ Bulk volume) | Average tablet mass (g) |
|---|---|---|---|---|---|---|---|---|---|---|
| Example 26 | 1.00 | 0.5009 | Tamarind gum | 100.2 | 16.7 | 0.60 | 1.2 | 1.1 | 1.10 | 0.6011 |
| Example 27 | 0.88 | 0.4397 | Quince seed gum | 22.0 | 4.8 | 0.53 | 1.2 | 1.1 | 1.25 | 0.4617 |
| Example 28 | 1.01 | 0.5039 | Carmellose sodium | 15.1 | 2.9 | 0.60 | 1.2 | 1.1 | 1.09 | 0.5191 |
| Example 29 | 1.12 | 0.5587 | Hydroxyethylcellulose | 16.8 | 2.9 | 0.67 | 1.2 | 1.2 | 1.07 | 0.5754 |
| Example 30 | 1.13 | 0.5661 | Sodium alginate | 17.0 | 2.9 | 0.68 | 1.2 | 1.2 | 1.06 | 0.5831 |
| Example 31 | 1.05 | 0.5268 | Tragacanth | 15.8 | 2.9 | 0.63 | 1.2 | 1.2 | 1.14 | 0.5426 |
| Example 32 | 1.05 | 0.5238 | Guar gum | 15.7 | 2.9 | 0.63 | 1.2 | 1.2 | 1.15 | 0.5395 |
| Example 33 | 1.10 | 0.5486 | Carboxy vinyl polymer | 16.5 | 2.9 | 0.66 | 1.2 | 1.2 | 1.09 | 0.5651 |
| Example 34 | 0.90 | 0.4502 | Locust bean gum | 27.0 | 5.7 | 0.54 | 1.2 | 1.1 | 1.22 | 0.4772 |
| Example 35 | 0.88 | 0.4421 | Polyvinyl alcohol | 22.1 | 4.8 | 0.44 | 1.0 | 1.1 | 1.24 | 0.4642 |
| Example 36 | 0.98 | 0.4911 | Gelatin | 14.7 | 2.9 | 0.59 | 1.2 | 1.1 | 1.12 | 0.5058 |
| Example 37 | 0.94 | 0.4678 | Corn starch | 46.8 | 9.1 | 0.56 | 1.2 | 1.1 | 1.18 | 0.5146 |
| Example 38 | 1.05 | 0.5233 | Hydroxyethylcellulose | 15.7 | 2.9 | 0.63 | 1.2 | 1.1 | 1.05 | 0.5390 |
| Example 39 | 0.86 | 0.4306 | Hydroxypropylmethylcellulose 2910 (Hypromellose) | 86.1 | 16.7 | 0.52 | 1.2 | 1.1 | 1.28 | 0.5168 |

(continued)

| | Spherical activated carbon | | Excipient | | | Solvent | Solvent mixture ratio (mL/g) | Volume of tablet ($v_1$) ($cm^3$) | $V_1/V_2$ (Volume of tablet/ Bulk volume) | Average tablet mass (g) |
|---|---|---|---|---|---|---|---|---|---|---|
| Example 40 | 0.4981 | 1.00 | Tara gum | 14.9 | 2.9 | 0.60 | 1.2 | 1.1 | 1.10 | 0.5130 |
| Example 41 | 0.4845 | 0.96 | Sodium hyaluronate | 14.4 | 2.9 | 0.58 | 1.2 | 1.1 | 1.14 | 0.4948 |
| Example 42 | 0.4442 | 0.89 | Xanthan gum | 13.3 | 2.9 | 0.71 | 1.6 | 1.1 | 1.24 | 0.4575 |
| Example 55 | 0.2046 | 0.41 | Pullulan | 6.1 | 2.9 | 0.25 | 1.2 | 0.41 | 1.02 | 0.2107 |
| Example 56 | 0.2147 | 0.43 | Pullulan | 16.1 | 7.0 | 0.21 | 1.0 | 0.46 | 1.02 | 0.2308 |
| Example 57 | 0.5464 | 1.09 | Polyvinyl alcohol | 0.6 | 0.1 | 0.66 | 1.2 | 1.1 | 1.01 | 0.5633 |
| | | | Pullulan | 16.4 | 2.9 | | | | | |
| Example 58 | 0.5113 | 1.02 | Polyethylene oxide※ | 0.5 | 0.1 | 0.61 | 1.2 | 1.1 | 1.08 | 0.5271 |
| | | | Pullulan | 15.4 | 2.9 | | | | | |
| Example 59 | 0.5257 | 1.05 | Hydroxypropylmethylcellulose 2910 (Hypromellose) | 0.5 | 0.1 | 0.63 | 1.2 | 1.1 | 1.05 | 0.5420 |
| | | | Pullulan | 15.8 | 2.9 | | | | | |
| Example 60 | 0.5120 | 1.02 | Ethylcellulose※ | 5.3 | 1.0 | 0.61 | 1.2 | 1.1 | 1.07 | 0.5328 |
| | | | Pullulan | 15.5 | 2.9 | | | | | |

※: Excipient without thin film-forming ability.

Table 12-3

| | Spherical activated carbon | | Excipient | | | Solvent | Solvent mixture ratio (mL/g) | Volume of tablet ($v_1$) (cm$^3$) | $V_1/V_2$ (Volume of tablet/ Bulk volume) | Average tablet mass (g) |
|---|---|---|---|---|---|---|---|---|---|---|
| Example 61 | 0.5170 | 1.03 | Carmellose calcium※ | 5.3 | 1.0 | 0.62 | 1.2 | 1.1 | 1.06 | 0.5380 |
| | | | Pullulan | 15.7 | 2.9 | | | | | |
| Example 62 | 0.5198 | 1.04 | Macrogol 6000※ | 5.5 | 1.0 | 0.62 | 1.2 | 1.1 | 1.06 | 0.5516 |
| | | | Pregelatinized starch | 26.3 | 4.8 | | | | | |
| Example 63 | 0.5185 | 1.04 | Methylcelluose | 5.3 | 1.0 | 0.62 | 1.2 | 1.1 | 1.06 | 0.5395 |
| | | | Pullulan | 15.7 | 2.9 | | | | | |
| Example 64 | 0.5294 | 1.06 | Carmellose※ | 5.6 | 1.0 | 0.64 | 1.2 | 1.1 | 1.04 | 0.5617 |
| | | | Pregelatinized starch | 26.7 | 4.8 | | | | | |
| Example 65 | 0.5301 | 1.06 | Low substituted hydroxypropylcellusose※ | 2.7 | 0.5 | 0.64 | 1.2 | 1.1 | 1.04 | 0.5488 |
| | | | Pregelatinized starch | 16.0 | 2.9 | | | | | |
| Example 66 | 0.5266 | 1.05 | Glucomannan | 2.7 | 0.5 | 0.63 | 1.2 | 1.1 | 1.04 | 0.5452 |
| | | | Pregelatinized starch | 15.8 | 2.9 | | | | | |
| Example 67 | 0.5437 | 1.09 | Carrageenan | 2.8 | 0.5 | 0.65 | 1.2 | 1.1 | 1.01 | 0.5629 |
| | | | Pregelatinized starch | 16.4 | 2.9 | | | | | |
| Example 68 | 0.5247 | 1.05 | Xanthan gum | 0.5 | 0.1 | 0.63 | 1.2 | 1.1 | 1.05 | 0.5410 |
| | | | Pregelatinized starch | 15.8 | 2.9 | | | | | |
| Example 69 | 0.1424 | 0.28 | Pullulan | 6.1 | 2.9 | 0.22 | 1.1 | 0.34 | 1.23 | 0.2095 |
| | | | Microcrystalline cellulose※ | 61.0 | 29.1 | | | | | |

※ : Excipient without thin film-forming ability.

Table 12-4

| | Spherical activated carbon | | Excipient | | | Solvent | Solvent mixture ratio (mL/g) | Volume of tablet ($V_1$) ($cm^3$) | $V_1/V_2$ (Volume of tablet/ Bulk volume) | Average tablet mass (g) |
|---|---|---|---|---|---|---|---|---|---|---|
| Comparative example 1 | - | - | Maltitol※ | | - | 70.0 | - | 1.4 | - | - | - |
| Comparative example 2 | - | - | Maltitol※ | | - | 70.0 | - | 1.4 | - | - | - |
| Comparative example 3 | - | - | Maltitol | | - | 50.0 | - | 0.4 | - | - | - |
| Comparative example 4 | - | - | Maltitol | | - | 50.0 | - | 0.4 | - | - | - |
| Comparative example 5 | - | - | Maltitol | | - | 30.0 | - | 0.3 | - | - | - |
| Comparative example 6 | - | - | Maltitol | | - | 30.0 | - | 0.3 | - | - | - |
| Comparative example 7 | 0.1941 | 0.39 | Pullulan | | 0.97 | 0.5 | 0.23 | 1.2 | 0.40 | 1.03 | 0.1951 |
| Comparative example 8 | - | - | Macrogol 6000 | | - | 9.1 | - | 1.2 | - | - | - |
| Comparative example 9 | - | - | Lactose hydrate※ | | - | 9.1 | - | 1.2 | - | - | - |
| Comparative example 10 | - | - | Beta-cyclodextrin※ | | - | 9.1 | - | 1.2 | - | - | - |
| Comparative example 11 | - | - | Carmellose※ | | - | 2.9 | - | 1.2 | - | - | - |
| Comparative example 12 | - | - | Carmellose calcium | | - | 2.9 | - | 1.2 | - | - | - |
| Comparative example 13 | 0.3318 | 0.66 | Low substituted hydroxypropylcellulose | | 66.4 | 16.7 | 0.40 | 1.2 | 1.1 | 1.66 | 0.3981 |
| Comparative example 14 | 0.4254 | 0.85 | Polyethylene oxide※ | | 85.1 | 16.7 | 0.85 | 2.0 | 1.3 | 1.53 | 0.5105 |

EP 2 684 561 B1

(continued)

| | Spherical activated carbon | | Excipient | | | Solvent | Solvent mixture ratio (mL/g) | Volume of tablet ($V_1$) ($cm^3$) | $V_1/V_2$ (Volume of tablet/ Bulk volume) | Average tablet mass (g) |
|---|---|---|---|---|---|---|---|---|---|---|
| Comparative example 15 | 0.4854 | 0.97 | Copovidone※ | 97.1 | 16.7 | 0.58 | 1.2 | 1.1 | 1.13 | 0.5825 |
| Comparative example 16 | 0.0750 | 0.15 | Povidone Lactose hydrate Magnesium stearate※ | 18.8 277.5 3.8 | 5.0 74.0 1.0 | Without use | 0 | 0.35 | 2.32 | 0.3750 |
| Comparative example 17 | 0.0607 | 0.12 | Povidone Lactose hydrate Magnesium stearate | 20.2 319.6 4.0 | 5.0 79.0 1.0 | Without use | 0 | 0.39 | 3.30 | 0.4045 |
| Comparative example 18 | 0.1263 | 0.25 | Povidone Hydroxypropylcellulose Pullulan Lactose hydrate※ | 30.3 25.3 7.6 60.6 | 12.1 10.1 3.0 24.2 | Without use | 0 | 0.39 | 1.58 | 0.2500 |
| Comparative example 19 | 0.0610 | 0.12 | Pullulan Microcrystalline cellulose | 6.1 142.4 | 2.9 68.0 | 0.24 | 1.2 | 0.34 | 2.70 | 0.2095 |
| ※ : Excipient without thin film-forming ability. | | | | | | | | | | |

Table 13-1

| | Excipient | Excipient Weight % | Hardness (N) | Disintegrating property | Friability (wt%) | Spherical particle rate |
|---|---|---|---|---|---|---|
| Example 1 | Pregelatinized starch | 2.9 | 30.2 | Pass | 4.6 | 100 % |
| Example 2 | Pregelatinized starch | 4.8 | 58.6 | Pass | 2.5 | 100 % |
| Example 3 | Pregelatinized starch | 9.1 | 67.6 | Pass | 1.7 | 100 % |
| Example 4 | Povidone | 2.9 | 101 | Pass | 1.0 | 100 % |
| Example 5 | Pullulan | 2.9 | 42 | Pass | 1.1 | 100 % |
| Example 6 | Oxidized starch | 9.1 | 89 | Pass | 1.5 | 100 % |
| Example 7 | Hydroxypropylmethylcellulose 2208 (Hypromellose) | 4.8 | 89 | Pass | 1.5 | 100 % |
| Example 8 | Ghatti gum | 9.1 | 68 | Pass | 1.6 | 100 % |
| Example 9 | Distarch phosphate | 9.1 | 114 | Pass | 1.7 | 100 % |
| Example 10 | Polyvinyl alcohol-acrylic acid-methyl methacrylate copolymer | 4.8 | 38 | Pass | 1.9 | 100 % |
| Example 11 | Gelatin | 2.9 | 46 | Pass | 2.2 | 100 % |
| Example 12 | Gellan gum | 9.1 | 24 | Pass | 2.5 | 100 % |
| Example 13 | Hydroxypropyl starch | 4.8 | 72 | Pass | 2.7 | 100 % |
| Example 14 | Hydroxypropylmethylcellulose 2906 (Hypromellose) | 2.9 | 52 | Pass | 2.8 | 100 % |
| Example 15 | Dextrin | 16.7 | 38 | Pass | 3.1 | 100 % |
| Example 16 | Tara gum | 2.9 | 55 | Pass | 3.6 | 100 % |
| Example 17 | Propylene glycol alginate | 2.9 | 53 | Pass | 3.6 | 100 % |
| Example 18 | Hydroxypropylcellulose | 2.9 | 22 | Pass | 3.7 | 100 % |
| Example 19 | Partly pregelatinized starch | 4.8 | 57 | Pass | 3.7 | 100 % |
| Example 20 | Agar | 2.9 | 112 | Pass | 3.8 | 100 % |
| Example 21 | Carrageenan | 4.8 | 20 | Pass | 3.9 | 100 % |

(continued)

| | Excipient | Excipient Weight % | Hardness (N) | Disintegrating property | Friability (wt%) | Spherical particle rate |
|---|---|---|---|---|---|---|
| Example 22 | Methylcellulose | 4.8 | 44 | Pass | 3.9 | 100 % |
| Example 23 | Glucomannan | 2.9 | 41 | Pass | 4.0 | 100 % |
| Example 24 | Kanbaiko | 6.5 | 47 | Pass | 4.1 | 100 % |

Table 13-2

| | Excipient | | Hardness (N) | Disintegrating property | Friability (wt%) | Spherical particle rate |
|---|---|---|---|---|---|---|
| | Name | Excipient Weight % | | | | |
| Example 25 | Microcrystalline cellulose carmellose sodium | 20.0 | 51 | Pass | 4.2 | 100 % |
| Example 26 | Tamarind gum | 16.7 | 30 | Pass | 4.3 | 100 % |
| Example 27 | Quince seed gum | 4.8 | 29 | Pass | 4.4 | 100 % |
| Example 28 | Carmellose sodium | 2.9 | 52 | Pass | 4.5 | 100 % |
| Example 29 | Hydroxyethylcellulose | 2.9 | 41 | Pass | 4.7 | 100 % |
| Example 30 | Sodium alginate | 2.9 | 43 | Pass | 5.2 | 100 % |
| Example 31 | Tragacanth | 2.9 | 41 | Pass | 5.4 | 100 % |
| Example 32 | Guar gum | 2.9 | 26 | Pass | 5.8 | 100 % |
| Example 33 | Carboxy vinyl polymer | 2.9 | 42 | Pass | 6.0 | 100 % |
| Example 34 | Locust bean gum | 5.7 | 45 | Pass | 6.2 | 100 % |
| Example 35 | Polyvinyl alcohol | 4.8 | 73 | Pass | 0.3 | 100 % |
| Example 36 | Gelatin | 2.9 | 132 | Pass | 0.8 | 100 % |
| Example 37 | Corn starch | 9.1 | 69 | Pass | 2.3 | 100 % |
| Example 38 | Hydroxyethylcellulose | 2.9 | 31 | Pass | 3.2 | 100 % |
| Example 39 | Hydroxypropylmethylcellulose 2910 (Hypromellose) | 16.7 | 87 | Pass | 3.2 | 100 % |

(continued)

|  | Excipient | | Hardness (N) | Disintegrating property | Friability (wt%) | Spherical particle rate |
|---|---|---|---|---|---|---|
|  | Name | Excipient Weight % | | | | |
| Example 40 | Tara gum | 2.9 | 21 | Pass | 4.1 | 100 % |
| Example 41 | Sodium hyaluronate | 2.9 | 32 | Pass | 4.1 | 100 % |
| Example 42 | Xanthan gum | 2.9 | 20 | Pass | 6.2 | 100 % |
| Example 55 | Pullulan | 2.9 | 38 | Pass | 0 | 100 % |
| Example 56 | Pullulan | 7.0 | 102 | Pass | -0.1 | 100 % |
| Example 57 | Polyvinyl alcohol | 0.1 | 26 | Pass | 0 | 100 % |
| | Pullulan | 2.9 | | | | |
| Example 58 | Polyethylene oxide※ | 0.1 | 33 | Pass | 0.5 | 100 % |
| | Pullulan | 2.9 | | | | |
| ※ : Excipient without thin film-forming ability. | | | | | | |

Table 13-3

| | Excipient | | Hardness(N) | Disintegrating property | Friability (wt%) | Spherical particle rate |
|---|---|---|---|---|---|---|
| | Name | ExcipientWeight % | | | | |
| Example 59 | Hydroxypropylmethylcellulose 2910 (hypromellose) | 0.1 | 21 | Pass | 0.6 | 100 % |
| | Pullulan | 2.9 | | | | |
| Example 60 | Ethylcellulose※ | 1.0 | 49 | Pass | 0.6 | 100 % |
| | Pullulan | 2.9 | | | | |
| Example 61 | Carmellose calcium※ | 1.0 | 21 | Pass | 0.6 | 100 % |
| | Pullulan | 2.9 | | | | |
| Example 62 | Macrogol 6000※ | 1.0 | 66 | Pass | 1.3 | 100 % |
| | Pregelatinized starch | 4.8 | | | | |
| Example 63 | Methylcellulose | 1.0 | 21 | Pass | 1.6 | 100 % |
| | Pullulan | 2.9 | | | | |
| Example 64 | Carmellose※ | 1.0 | 50 | Pass | 2.2 | 100 % |
| | Pregelatinized starch | 4.8 | | | | |
| Example 65 | Low substituted hydroxypropylcellulose※ | 0.5 | 24 | Pass | 3.2 | 100 % |
| | Pregelatinized starch | 2.9 | | | | |
| Example 66 | Glucomannan | 0.5 | 33 | Pass | 3.8 | 100 % |
| | Pregelatinized starch | 2.9 | | | | |
| Example 67 | Carrageenan | 0.5 | 22 | Pass | 4.1 | 100 % |
| | Pregelatinized starch | 2.9 | | | | |
| Example 68 | Xanthan gum | 0.1 | 27 | Pass | 4.2 | 100 % |
| | Pregelatinized starch | 2.9 | | | | |
| Example 69 | Pullulan | 2.9 | 37 | Pass | -0.4 | 100 % |
| | Microcrystalline cellulose※ | 29.1 | | | | |

※ : Excipient without thin film-forming ability.

EP 2 684 561 B1

49

Table 13-4

| | Excipient | | Hardness(N) | Disintegrating property | Friability(wt%) | Spherical particle rate |
|---|---|---|---|---|---|---|
| | Name | ExcipientWeight % | | | | |
| Comparative example 1 | Maltitol※ | 70.0 | Unmeasurable | Unmeasurable | Unmeasurable | 100 % |
| Comparative example 2 | Maltitol※ | 70.0 | Unmeasurable | Unmeasurable | Unmeasurable | 100 % |
| Comparative example 3 | Maltitol※ | 50.0 | Unmeasurable | Unmeasurable | Unmeasurable | 100 % |
| Comparative example 4 | Maltitol※ | 50.0 | Unmeasurable | Unmeasurable | Unmeasurable | 100 % |
| Comparative example 5 | Maltitol※ | 30.0 | Unmeasurable | Unmeasurable | Unmeasurable | 100 % |
| Comparative example 6 | Maltitol※ | 30.0 | Unmeasurable | Unmeasurable | Unmeasurable | 100 % |
| Comparative example 7 | Pullulan | 0.5 | N.D.※※※ | Pass | 79.0 | 100 % |
| Comparative example 8 | Macrogol 6000※ | 9.1 | Unmeasurable | Unmeasurable | Unmeasurable | 100 % |
| Comparative example 9 | Lactose hydrate※ | 9.1 | Unmeasurable | Unmeasurable | Unmeasurable | 100 % |
| Comparative example 10 | Beta-cyclodextrin※ | 9.1 | Unmeasurable | Unmeasurable | Unmeasurable | 100 % |
| Comparative example 11 | Carmellose※ | 2.9 | Unmeasurable | Unmeasurable | Unmeasurable | 100 % |
| Comparative example 12 | Carmellose calcium※ | 2.9 | Unmeasurable | Unmeasurable | Unmeasurable | 100 % |
| Comparative example 13 | Low substituted Hydroxypropylcellulose※ | 16.7 | N.D.※※※ | Pass | 100 | 100 % |
| Comparative example 14 | Polyethylene oxide※ | 16.7 | N.D.※※※ | Fail | 0.6 | 100 % |
| Comparative example 15 | Copovidone※ | 16.7 | 12 | Pass | 32.8 | 100 % |
| Comparative example 16 | Povidone<br>Lactose hydrate※<br>Magnesium stearate※ | 5.0<br>74.0<br>1.0 | N.D.※※※ | Pass | 100 | 29※※ |
| Comparative example 17 | Ppovidone<br>Lactose hydrate※<br>Magnesium stearate※ | 5.0<br>79.0<br>1.0 | N.D.※※※ | Pass | 100 | 98.60 % |

(continued)

| | Excipient | | Hardness(N) | Disintegrating property | Friability(wt%) | Spherical particle rate |
|---|---|---|---|---|---|---|
| | Name | ExcipientWeight % | | | | |
| Comparative example 18 | Povidone | 12.1 | N.D.※※※ | Pass | 100 | 32.70 % |
| | Hydroxypropylcellulose | 10.1 | | | | |
| | Pullulan | 3.0 | | | | |
| | Lactose hydrate※ | 24.2 | | | | |
| Comparative example 19 | Pullulan | 2.9 | 38 | Pass | -0.2 | 100 % |
| | Microcrystalline cellulose※ | 68.0 | | | | |

※ : Excipient without thin film-forming ability.

※※ : Result of comparative example 16 is obtained by CCD camera.

※※※ : Not Detected

Table 14

| | Spherical activated carbon | | Excipient | | | Solvent | Solvent mixture ratio (mL/g) | Average tablet mass (g) | Volume of tablet (cm³) | Relative adsorption rate of indole after 1 hour |
|---|---|---|---|---|---|---|---|---|---|---|
| | Weight (g) | Bulk volume (cm³) | Name | Weight (mg) | Excipient Weight % | Purified water (mL) | | | | |
| Example 43 | 0.1939 | 0.39 | Pregelatinized starch | 9.7 | 4.8 | 0.23 | 1.2 | 0.2036 | 0.4407 | 84 % |
| Example 44 | 0.2080 | 0.42 | Sodium alginate | 6.2 | 2.9 | 0.25 | 1.2 | 0.2142 | 0.5040 | 104 % |
| Example 45 | 0.2068 | 0.41 | Carboxy vinyl polymer | 6.2 | 2.9 | 0.25 | 1.2 | 0.2130 | 0.4920 | 94 % |
| Example 46 | 0.2071 | 0.41 | Guar gum | 6.2 | 2.9 | 0.25 | 1.2 | 0.2133 | 0.4920 | 103 % |
| Example 47 | 0.2086 | 0.42 | Hydroxypropylcellulose | 6.3 | 2.9 | 0.25 | 1.2 | 0.2149 | 0.4788 | 105 % |
| Example 48 | 0.2036 | 0.41 | Povidone | 40.7 | 16.7 | 0.24 | 1.2 | 0.2443 | 0.4510 | 103 % |
| Example 49 | 0.2056 | 0.41 | Gelatin | 6.2 | 2.9 | 0.25 | 1.2 | 0.2118 | 0.4592 | 95 % |
| Example 50 | 0.2044 | 0.41 | Tara gum | 6.1 | 2.9 | 0.25 | 1.2 | 0.2106 | 0.4633 | 106 % |
| Example 51 | 0.2062 | 0.41 | Hydroxyethylcellulose | 6.2 | 2.9 | 0.25 | 1.2 | 0.2124 | 0.4018 | 93 % |
| Example 52 | 0.2044 | 0.41 | Hydroxypropylmethylcellulose 2910 (Hypromellose) | 40.9 | 16.7 | 0.25 | 1.2 | 0.2453 | 0.5248 | 87 % |
| Example 53 | 0.2070 | 0.41 | Polyvinyl alcohol | 10.4 | 4.8 | 0.21 | 1.0 | 0.2174 | 0.4551 | 81 % |
| Example 54 | 0.2035 | 0.41 | Dextrin | 6.1 | 2.9 | 0.24 | 1.2 | 0.2096 | 0.4510 | 103 % |

| | Spherical activated carbon | | Excipient | | | Solvent | Solvent mixture ratio (mL/g) | Average tablet mass (g) | Volume of tablet (cm$^3$) | Relative adsorption rate of indole after 1 hour |
|---|---|---|---|---|---|---|---|---|---|---|
| | Weight (g) | Bulk volume (cm$^3$) | Name | Weight (mg) | Excipient Weight % | Purified water (mL) | | | | |
| Example 55 | 0.2046 | 0.41 | Pullulan | 6.1 | 2.9 | 0.25 | 1.2 | 0.2107 | 0.4264 | 106 % |

Table 15

| Excipient | Weight-average molecular weight | Thin film-forming ability |
|---|---|---|
| Pregelatinized starch | 1000000 | Yes |
| Carmellose | ≥10000 | No |
| Agar | 290000 | Yes |
| Gelatin | 100000 | Yes |
| Tara gum | 6900000 | Yes |
| Hydroxyethylcellulose | 720000 | Yes |
| Hydroxypropylcellulose | 60000 | Yes |
| Hydroxypropylmethylcellulose 2910 (hypromellose) | 20000 | Yes |
| Pullulan | 200000 | Yes |
| Polyvinyl alcohol | 30000 | Yes |
| Methylcellulose | 300000 | Yes |
| Carmellose calcium | ≥10000 | No |
| Tamarind gum | 3400000 | Yes |
| Low substituted hydroxypropylcellulose | ≥10000 | No |
| Carmellose sodium | 126000~170000 | Yes |
| Carrageenan | ≥10000 | Yes |
| Xanthan gum | 42000000 | Yes |
| Glucomannan | ≥10000 | Yes |
| Povidone | ≥10000 | Yes |
| Polyethylene oxide | ≥10000 | No |
| Macrogol 6000 | 7300~9300 | No |
| Ethylcellulose | ≥10000 | No |
| Microcrystalline cellulose | ≥10000 | No |
| Lactose hydrate | 360.32 | No |
| Magnesium stearate | 591.25 | No |
| Sodium alginate | 89145~95088 | Yes |
| Carboxy vinyl polymer | 4000000~4500000 | Yes |
| Guar gum | 12000000 | Yes |
| Dextrin | 400000 | Yes |
| Tragacanth | 840000 | Yes |
| Beta-cyclodextrin | 1134.98 | No |
| Copovidone | ≥10000 | No |

<<Dose test for tablet-type composition for oral administration>>

A dose test was conducted so as to confirm that the amount of water intake during the dosing of the tablet-type composition for oral administration of the present invention is lower than that of a conventional capsule agent, due to the small volume of the tablet.

For each of the tablet-type compositions for oral administration and capsule agents showing $V_1/V_2 = 1.02$ to 2.00, the amount of water required for taking formulations in which the amounts correspond to 2 g of spherical activated

54

carbons was measured. As tablets to be taken, placebo tablets free from spherical activated carbon (diameter 10 mm, height 6 mm, radius of curvature 14 mm, volume 0.42 cm$^3$) were prepared in accordance with the volumes of the tablet of Example 55 ($V_1/V_2$ = 1.02), the tablet of Example 69 ($V_1/V_2$ = 1.23), and a tablet corresponding to $V_1/V_2$ = 2.00. The number of the tablets corresponding to 2 g of spherical activated carbons at that time was 10 tablets for Example 55 ($V_1/V_2$ = 1.02) and 12 tablets for Example 69 ($V_1/V_2$ = 1.23). Furthermore, the number of the tablets corresponding to $V_1/V_2$ = 2.00 calculated from Example 55 and Example 69 was 19 tablets.

The placebo tablets ($V_1/V_2$ = 1.02, 1.23, 2.00) in numbers corresponding to 2 g of spherical activated carbons (i.e., 10 tablets, 12 tablets, 19 tablets) and capsule agents (as the capsule agents, "Kremezin (registered trademark) capsule 200 mg" was used) were given to four monitor males, and the amount of water required for taking the whole amount of each was measured. The results are shown in Table 16. Although the amount of water required for taking differed depending on the monitor, the water required for taking was small in all of the monitors, in the order of the placebo tablets ($V_1/V_2$ = 1.02), the placebo tablets ($V_1/V_2$ = 1.23), the capsule agent ($V_1/V_2$ = 1.533) and the placebo tablets ($V_1/V_2$ = 2.00).

Table 16

| | | Water volume when taking | | | |
|---|---|---|---|---|---|
| | | Placebo tablet | | | Capsule |
| $V_1/V_2$ | | 1.02 | 1.23 | 2.00 | 1.533 |
| Number | | 10 tablets | 12 tablets | 19 tablets | 10 capsules |
| Monitor No. | Male 1 | 74 mL | 112 mL | 300 mL | 133 mL |
| | Male 2 | 160 mL | 207 mL | 338 mL | 235 mL |
| | Male 3 | 94 mL | 142 mL | 324 mL | 192 mL |
| | Male 4 | 148 mL | 188 mL | 392 mL | 268 mL |

<<Observation of surface and inside of tablet by scanning electron microscope>>

For the tablet-type compositions for oral administration prepared in Example 55 and Comparative Example 15, the observation was performed using a scanning electron microscope (Type JSM7401F, manufactured by JEOL Ltd.). The results of the observation of the surfaces and insides of the tablets were shown in Fig. 4 and Fig. 5. In the tablet-type composition for oral administration of Example 55, the surrounding of each particle of the spherical activated carbon was covered with the excipient for tablet formulation, and the particles were bound to each other through a thin film of the excipient over a wide surface area. On the other hand, in the tablet-type composition for oral administration of Comparative Example 15, the surrounding of each particle of the spherical activated carbon was covered with the excipient for tablet formulation, but a thin film was not able to be formed, and thus cracks were present among the particles and the particles were not sufficiently bound.

<<Measurement of fracture strength and distortion rate of spherical activated carbon>>

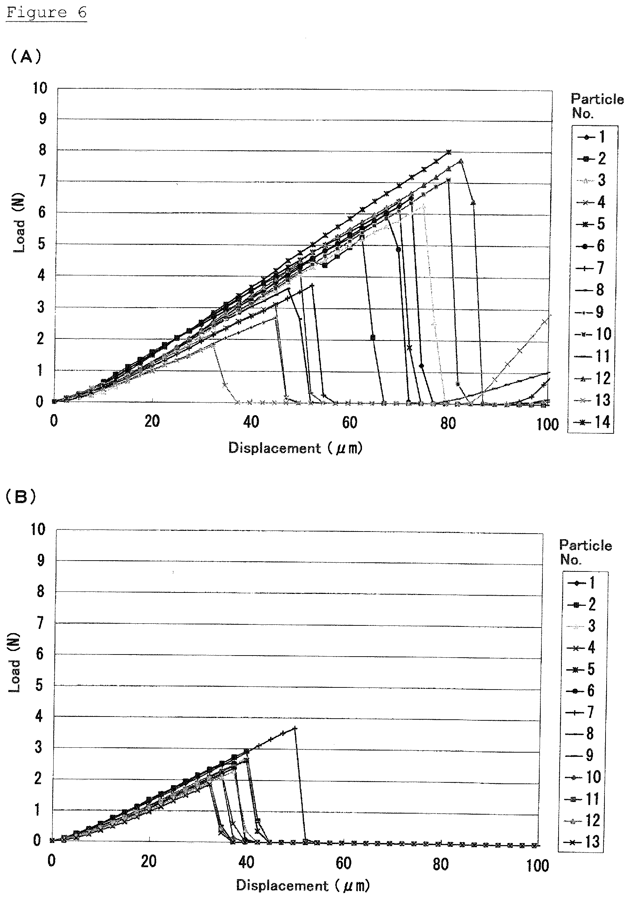

Using a hardness meter, the fracture strength and distortion rate of the spherical activated carbon were measured. The measurement was conducted on one particle by using Better Hardness Tester (BHT-500, manufactured by SEISHIN ENTERPRISE CO.,LTD.) according to a conventional method. The result of the measurement of 13 particles of the spherical activated carbon prepared in Preparation Example 1 (n = 13) is shown in Table 17 and Fig. 6A. Furthermore, the result of the measurement of 14 particles of the spherical activated carbon prepared in Preparation Example 2 (n = 14) is shown in Table 18 and Fig. 6B. Furthermore, the distortion rate when a pressure of 2 MPa was applied was obtained according to the following calculation formula.

```
Distortion rate when pressure of 2 MPa is applied =
distortion rate at break (%) / fracture strength (MPa) × 2
MPa
```

Table 17

|  | Average | SD | RSD(%) | Maximal value | Munimum value |
|---|---|---|---|---|---|
| Particle size ($\mu$m) | 318.1 | 35.1 | 11.0 | 396 | 270 |
| Displacement at flacturing ($\mu$m) | 37.5 | 4.9 | 13.0 | 50 | 32 |
| Distortion rate at flacturing (%) | 11.8 | 1.3 | 10.6 | 14.7 | 10.1 |
| Breaking load (N) | 2.46 | 0.48 | 19.3 | 3.66 | 1.85 |
| Fracture strength (MPa) | 31.2 | 5.1 | 16.2 | 40.5 | 23.7 |

The average fracture strength of the spherical activated carbon of Preparation Example 1 was 31.2 MPa. Furthermore, the distortion rate when a pressure of 2 MPa was applied was 0.76 % according to the following calculation formula.

```
11.8 (%) / 31.2 × 2 = 0.76 %
```

Table 18

|  | Average | SD | RSD (%) | Maximal value | Munimum value |
|---|---|---|---|---|---|
| Particle size ($\mu$m) | 124.4 | 25.1 | 20.2 | 158 | 87 |
| Displacement at flacturing ($\mu$m) | 60.4 | 15.9 | 26.4 | 82 | 32 |
| Distortion rate at flacturing (%) | 49.2 | 13.0 | 26.5 | 83.1 | 33.3 |
| Breaking load (N) | 5.17 | 1.97 | 38.1 | 7.98 | 1.81 |
| Fracture strength (MPa) | 436.5 | 194.1 | 44.5 | 1006.2 | 274.5 |

The average fracture strength of the spherical activated carbon of Preparation Example 2 was 436.5 MPa. Furthermore, the distortion rate when a pressure of 2 MPa was applied was 0.23 % according to the following calculation formula.

```
49.2 (%) / 436.5 × 2 = 0.23 %
```

In addition, as is apparent from Fig. 6A and Fig. 6B, it is understood that, in the cases when a load was applied to each of the spherical activated carbons of Preparation Examples 1 and 2, the spherical activated carbon was broken at a predetermined load and thereafter the load became 0. Therefore, the spherical activated carbons have such properties that they are deformed little when stress is applied thereto, whereas they are broken when a force above a certain degree is applied thereto.

INDUSTRIAL APPLICABILITY

The tablet-formed pharmaceutical composition for oral administration of the present invention can be used as an adsorbent for an oral administration for treating or preventing kidney diseases or liver diseases.

As the kidney diseases, there may be mentioned, for example, chronic renal failure, acute renal failure, chronic pyelonephritis, acute pyelonephritis, chronic nephritis, acute nephritic syndrome, acute progressive nephritic syndrome, chronic nephritic syndrome nephrotic syndrome, nephrosclerosis, interstitial nephritis, tubulopathy, lipoid nephrosis, diabetic nephropathy, renovascular hypertension, or hypertension syndrome, or secondary kidney diseases caused by these primary diseases, or a light renal failure before a dialysis therapy, and may be used in an improvement of a light renal failure before a dialysis therapy or a disease condition for a patient during a dialysis therapy (see "Clinical Nephrology", Asakura-shoten, Nishio Honda, Kenkichi Koiso, and Kiyoshi Kurokawa, 1990; and "Nephrology" Igaku-shoin, Teruo Omae and Sei Fujimi, ed., 1981).

As the liver diseases, there may be mentioned, for example, fulminant hepatitis, chronic hepatitis, viral hepatitis, alcoholic hepatitis, hepatic fibrosis, liver cirrhosis, hepatic cancer, autoimmune hepatitis, drug allergic hepatopathy, primary biliary cirrhosis, tremor, encephalopathia, dysbolism, or dysfunction. Further, the porous spherical carbonaceous substance can be used in a treatment of a disease caused by toxic substances in a body, such as psychosis.

## Claims

1.  A tablet-formed pharmaceutical composition for oral administration consisting of 65 % or more by weight of a spherical activated carbon as an active ingredient, and one or more excipients; wherein

    (a) the spherical activated carbon does not exhibit a water solubility and a swelling characteristic; a distortion rate thereof is 2 % or less when 2 MPa of pressure is applied; and a fracture strength thereof is 5 MPa or more;
    (b) the tablet-formed pharmaceutical composition for oral administration comprises one or more excipients for tablet formulation selected from the group consisting of pullulan, polyvinyl alcohol, gelatin, povidone, pregelatinized starch, oxidized starch, hypromellose, hydroxypropyl starch, dextrin, polyvinyl alcohol-acrylic acid-methyl methacrylate copolymer, Kanbaiko, propylene glycol alginate, agar, glucomannan, carmellose sodium, hydroxyethylcellulose, hydroxypropylcellulose, tara gum, tamarind gum, carrageenan, methylcellulose, sodium hyaluronate, sodium alginate, carboxyvinyl polymer, partly pregelatinized starch, tragacanth, guar gum, xanthan gum, quince seed gum, locust bean gum, distarch phosphate, ghatti gum, and gellan gum, wherein the amount of the excipients for tablet formulation totals 1 % or more by weight with respect to the tablet-formed pharmaceutical composition for oral administration, provided that when the excipients for tablet formulation include pullulan, the amount of pullulan is 1 % or more by weight with respect to the tablet-formed pharmaceutical composition for oral administration, and the excipient for tablet formulation forms a thin film when 0.5 mL of a water solution or a water dispersion of the excipient for tablet formulation of 1 % by weight is heat-dried after being placed on a flat surface made of fluorine resin wherein the distortion rate is determined as referred in the description.

2.  The tablet-formed pharmaceutical composition for oral administration according to claim 1, wherein the composition satisfies the following equation(1) :

$$V_1/V_2 \leq 1.53 \quad (1)$$

    wherein $V_1$ is a volume of the tablet-formed pharmaceutical composition for oral administration, and V2 is a bulk volume of the spherical activated carbons contained in the tablet-formed pharmaceutical composition for oral administration in the case that the spherical activated carbons are closely packed.

3.  The tablet-formed pharmaceutical composition for oral administration according to claim 1 or 2, wherein an average particle diameter of the spherical activated carbon is 0.02 to 1 mm, and a specific surface thereof is 500 $m^2/g$ or more. wherein the average particle diameter and the specific surface are determined as referred in the description.

4.  The tablet-formed pharmaceutical composition for oral administration according to any of the preceding claims, wherein a volume of the tablet-formed pharmaceutical composition for oral administration is 3.06 $cm^3$ or less with respect to 1 g of the spherical activated carbon contained therein as an active ingredient.

5.  The tablet-formed pharmaceutical composition for oral administration according to any of the preceding claims, wherein a spherical form of 80 % or more of the spherical activated carbon contained therein is maintained.

6.  The tablet-formed pharmaceutical composition for oral administration according to any of the preceding claims, wherein a hardness of the tablet-formed pharmaceutical composition for oral administration is 20 N or more. wherein the hardness is determined as referred in the description.

7.  The tablet-formed pharmaceutical composition for oral administration according to any of the preceding claims, wherein a friability of the tablet-formed pharmaceutical composition for oral administration is 7 Wt% or less wherein the friability is determined as referred in the description.

8. The tablet-formed pharmaceutical composition for oral administration according to any of the preceding claims, wherein a weight-average molecular weight of the excipient for tablet formulation is 10,000 or more.

9. A method for preparing a tablet-formed pharmaceutical composition for oral administration comprising the steps of:

(1) mixing a total of 100 parts by weight of spherical activated carbon and one or more excipients selected from the group consisting of pullulan, polyvinyl alcohol, gelatin, povidone, pregelatinized starch, oxidized starch, hypromellose, hydroxypropyl starch, dextrin, polyvinyl alcohol-acrylic acid-methyl methacrylate copolymer, Kanbaiko, propylene glycol alginate, agar, glucomannan, carmellose sodium, hydroxyethylcellulose, hydroxypropylcellulose, tara gum, tamarind gum, carrageenan, methylcellulose, sodium hyaluronate, sodium alginate, carboxyvinyl polymer, partly pregelatinized starch, tragacanth, guar gum, xanthan gum, quince seed gum, locust bean gum, distarch phosphate, ghatti gum, and gellan gum, and 10 parts or more by weight of solvent, wherein the spherical activated carbon is 65 to 99 parts by weight and the excipient is 1 to 35 parts by weight, and (a) the spherical activated carbon does not exhibit a water solubility and a swelling characteristic; a distortion rate thereof is 2 % or less when 2 MPa of pressure is applied; and a fracture strength thereof is 5 MPa or more, (b) one or more excipients for tablet formulation as the excipient totals 1 part or more by weight, provided that when the excipients for tablet formulation include pullulan, the amount of pullulan is 1 part or more by weight with respect to the tablet-formed pharmaceutical composition for oral administration, and the excipient for tablet formulation forms a thin film when 0.5 mL of a water solution or a water dispersion of the excipient for tablet formulation of 1 % by weight is heat-dried after being placed on a flat surface made of fluorine resin,
(2) forming the mixture into a tablet form product, and
(3) drying the tablet form product.

10. The method for preparing a tablet-formed pharmaceutical composition for oral administration according to claim 9, the drying process in the drying step (3) is freeze drying, drying under reduced pressure, draught drying, or heat drying.

**Patentansprüche**

1. Tablettenförmige pharmazeutische Zusammensetzung zur oralen Verabreichung, bestehend zu 65 Gew.-% oder mehr aus einer kugelförmigen Aktivkohle als ein Wirkstoff und einem oder mehreren Hilfsstoffen; wobei

(a) die kugelförmige Aktivkohle keine Wasserlöslichkeit und keine Quelleigenschaft vorweist; eine Verformungsrate davon 2 % oder weniger beträgt, wenn ein Druck von 2 MPa angelegt wird; und eine Bruchfestigkeit davon 5 MPa oder mehr beträgt;
(b) die tablettenförmige pharmazeutische Zusammensetzung zur oralen Verabreichung einen oder mehrere Hilfsstoffe zur Tablettenformulierung umfasst, ausgewählt aus der Gruppe bestehend aus Pullulan, Polyvinylalkohol, Gelatine, Povidon, vorgelatinierter Stärke, oxidierter Stärke, Hypromellose, Hydroxypropylstärke, Dextrin, Polyvinylalkohol-Acrylsäure-Methylmethacrylat-Copolymer, Kanbaiko, Propylenglycolalginat, Agar, Glucomannan, Carmellose-Natrium, Hydroxyethylcellulose, Hydroxypropylcellulose, Taragummi, Tamarindengummi, Carrageenan, Methylcellulose, Natriumhyaluronat, Natriumalginat, Carboxyvinylpolymer, teilweise vorgelatinierter Stärke, Tragant, Guargummi, Xanthangummi, Quittensamengummi, Johannisbrotkernmehl, Distärkephosphat, Ghattigummi und Gellangummi, wobei die Menge der Hilfsstoffe zur Tablettenformulierung bezüglich der tablettenförmigen pharmazeutischen Zusammensetzung zur oralen Verabreichung insgesamt 1 Gew.-% oder mehr beträgt, vorausgesetzt, dass, wenn die Hilfsstoffe zur Tablettenformulierung Pullulan einschließen, die Menge an Pullulan bezüglich der tablettenförmigen pharmazeutischen Zusammensetzung zur oralen Verabreichung 1 Gew.,-% oder mehr beträgt, und der Hilfsstoff zur Tablettenformulierung einen dünnen Film bildet, wenn 0,5 ml einer Wasserlösung oder einer Wasserdispersion des Hilfsstoffs zur Tablettenformulierung von 1 Gew.-% wärmegetrocknet werden, nachdem sie auf eine flache Oberfläche, hergestellt aus Fluorharz, platziert worden ist, wobei die Verformungsrate wie in der Beschreibung referenziert bestimmt wird.

2. Tablettenförmige pharmazeutische Zusammensetzung zur oralen Verabreichung nach Anspruch 1, wobei die Zusammensetzung die folgende Gleichung (1) erfüllt:

$$V_1/V_2 \leq 1,53 \quad (1)$$

wobei $V_1$ ein Volumen der tablettenförmigen pharmazeutischen Zusammensetzung zur oralen Verabreichung ist und V2 ein Schüttvolumen der kugelförmigen Aktivkohlen ist, die in der tablettenförmigen pharmazeutischen Zusammensetzung zur oralen Verabreichung enthalten sind, in dem Fall, dass die kugelförmigen Aktivkohlen eng gepackt sind.

3. Tablettenförmige pharmazeutische Zusammensetzung zur oralen Verabreichung nach Anspruch 1 oder 2, wobei ein durchschnittlicher Partikeldurchmesser der kugelförmigen Aktivkohle 0,02 bis 1 mm beträgt und eine spezifische Oberfläche davon 500 $m^2$/g oder mehr beträgt.
wobei der durchschnittliche Partikeldurchmesser und die spezifische Oberfläche wie in der Beschreibung referenziert bestimmt werden.

4. Tablettenförmige pharmazeutische Zusammensetzung zur oralen Verabreichung nach einem der vorstehenden Ansprüche, wobei ein Volumen der tablettenförmigen pharmazeutischen Zusammensetzung zur oralen Verabreichung bezüglich 1 g der darin enthaltenen kugelförmigen Aktivkohle als ein Wirkstoff 3,06 $cm^3$ oder weniger beträgt.

5. Tablettenförmige pharmazeutische Zusammensetzung zur oralen Verabreichung nach einem der vorstehenden Ansprüche, wobei eine kugelförmige Form von 80 % oder mehr der darin enthaltenen kugelförmigen Aktivkohle aufrechterhalten wird.

6. Tablettenförmige pharmazeutische Zusammensetzung zur oralen Verabreichung nach einem der vorstehenden Ansprüche, wobei eine Härte der tablettenförmigen pharmazeutischen Zusammensetzung zur oralen Verabreichung 20 N oder mehr beträgt.
wobei die Härte wie in der Beschreibung referenziert bestimmt wird.

7. Tablettenförmige pharmazeutische Zusammensetzung zur oralen Verabreichung nach einem der vorstehenden Ansprüche, wobei eine Brüchigkeit der tablettenförmigen pharmazeutischen Zusammensetzung zur oralen Verabreichung 7 Gew.-% oder weniger beträgt,
wobei die Brüchigkeit wie in der Beschreibung referenziert bestimmt wird.

8. Tablettenförmige pharmazeutische Zusammensetzung zur oralen Verabreichung nach einem der vorstehenden Ansprüche, wobei ein gewichtsmittleres Molekulargewicht des Hilfsstoffs zur Tablettenformulierung 10.000 oder mehr beträgt.

9. Verfahren zum Herstellen einer tablettenförmigen pharmazeutischen Zusammensetzung zur oralen Verabreichung, umfassend die Schritte:

(1) Mischen einer Gesamtheit von 100 Gewichtsteilen kugelförmiger Aktivkohle und einem oder mehreren Hilfsstoffen, ausgewählt aus der Gruppe bestehend aus Pullulan, Polyvinylalkohol, Gelatine, Povidon, vorgelatinierter Stärke, oxidierter Stärke, Hypromellose, Hydroxypropylstärke, Dextrin, Polyvinylalkohol-Acrylsäure-Methylmethacrylat-Copolymer, Kanbaiko, Propylenglycolalginat, Agar, Glucomannan, Carmellose-Natrium, Hydroxyethylcellulose, Hydroxypropylcellulose, Taragummi, Tamarindengummi, Carrageenan, Methylcellulose, Natriumhyaluronat, Natriumalginat, Carboxyvinylpolymer, teilweise vorgelatinierter Stärke, Tragant, Guargummi, Xanthangummi, Quittensamengummi, Johannisbrotkernmehl, Distärkephosphat, Ghattigummi und Gellangummi, und 10 Gewichtsteilen oder mehr an Lösungsmittel, wobei die kugelförmige Aktivkohle 65 bis 99 Gewichtsteile beträgt und der Hilfsstoff 1 bis 35 Gewichtsteile beträgt, und (a) die kugelförmige Aktivkohle keine Wasserlöslichkeit und keine Quelleigenschaft vorweist; eine Verformungsrate davon 2 % oder weniger beträgt, wenn ein Druck von 2 MPa angelegt wird; und eine Bruchfestigkeit davon 5 MPa oder mehr beträgt, (b) ein oder mehrere Hilfsstoffe zur Tablettenformulierung als der Hilfsstoff insgesamt 1 Gewichtsteil oder mehr beträgt, vorausgesetzt, dass, wenn die Hilfsstoffe zur Tablettenformulierung Pullulan einschließen, die Menge an Pullulan bezüglich der tablettenförmigen pharmazeutische Zusammensetzung zur orale Verabreichung 1 Gewichtsteil oder mehr beträgt, und der Hilfsstoff zur Tablettenformulierung einen dünnen Film bildet, wenn 0,5 ml einer Wasserlösung oder einer Wasserdispersion des Hilfsstoffs zur Tablettenformulierung von 1 Gew.-% wärmegetrocknet werden, nachdem sie auf einer flachen Oberfläche, hergestellt aus Fluorharz, platziert worden ist,
(2) Bilden der Mischung in ein Tablettenformprodukt, und
(3) Trocknen des Tablettenformprodukts.

10. Verfahren zum Herstellen einer tablettenförmigen pharmazeutischen Zusammensetzung zur oralen Verabreichung nach Anspruch 9, wobei der Trocknungsprozess in dem Trocknungsschritt (3) Gefriertrocknen, Trocknen unter reduziertem Druck, Luftzugtrocknung oder Wärmetrocknung ist.

## Revendications

1. Composition pharmaceutique formée en comprimé pour administration orale constituée de 65 % ou plus en poids d'un charbon actif sphérique en tant qu'ingrédient actif, et un ou plusieurs excipients ; dans laquelle

(a) le charbon actif sphérique ne présente pas de solubilité dans l'eau ni de caractéristique de gonflement ; un taux de déformation de celui-ci est de 2 % ou moins lorsque l'on applique une pression de 2 MPa ; et une résistance à la rupture de celui-ci est de 5 MPa ou plus ;
(b) la composition pharmaceutique formée en comprimé pour administration orale comprend un ou plusieurs excipients pour formulation de comprimé choisis dans le groupe constitué de pullulane, alcool polyvinylique, gélatine, povidone, amidon prégélatinisé, amidon oxydé, hypromellose, hydroxypropylamidon, dextrine, copolymère d'alcool polyvinylique-acide acrylique-méthacrylate de méthyle, Kanbaiko, alginate de propylène glycol, agar-agar, glucomannane, carmellose sodique, hydroxyéthylcellulose, hydroxypropylcellulose, gomme tara, gomme de tamarin, carraghénane, méthylcellulose, hyaluronate de sodium, alginate de sodium, polymère carboxyvinylique, amidon partiellement prégélatinisé, gomme adragante, gomme de guar, gomme de xanthane, gomme de pépins de coing, gomme de caroube, phosphate de diamidon, gomme ghatti et gomme gellane, dans laquelle la quantité des excipients pour formulation de comprimé totalise 1 % ou plus en poids par rapport à la composition pharmaceutique formée en comprimé pour administration orale, à condition que lorsque les excipients pour formulation de comprimé incluent du pullulane, la quantité de pullulane soit de 1 % ou plus en poids par rapport à la composition pharmaceutique formée en comprimé pour administration orale, et l'excipient pour formulation de comprimé forme un film mince lorsqu'on sèche à la chaleur 0,5 mL d'une solution aqueuse ou d'une dispersion aqueuse de l'excipient pour formulation de comprimé à 1 % en poids après être placée sur une surface plate constituée de résine fluorée dans laquelle le taux de déformation est déterminé de la façon mentionnée dans la description.

2. Composition pharmaceutique formée en comprimé pour administration orale selon la revendication 1, dans laquelle la composition respecte l'équation (1) suivante :

$$V_1/V_2 \leq 1,53 \quad (1)$$

dans laquelle $V_1$ est un volume de la composition pharmaceutique formée en comprimé pour administration orale, et $V_2$ est un volume en vrac des charbons actifs sphériques contenus dans la composition pharmaceutique formée en comprimé pour administration orale dans le cas où les charbons actifs sphériques sont étroitement tassés.

3. Composition pharmaceutique formée en comprimé pour administration orale selon la revendication 1 ou 2, dans laquelle un diamètre particulaire moyen du charbon actif sphérique va de 0,02 à 1 mm, et une surface spécifique de celui-ci est de 500 $m^2$/g ou plus.
dans laquelle le diamètre particulaire moyen et la surface spécifique sont déterminés de la façon mentionnée dans la description.

4. Composition pharmaceutique formée en comprimé pour administration orale selon l'une quelconque des revendications précédentes, dans laquelle un volume de la composition pharmaceutique formée en comprimé pour administration orale est de 3,06 $cm^3$ ou moins par rapport à 1 g du charbon actif sphérique contenu en son sein en tant qu'ingrédient actif.

5. Composition pharmaceutique formée en comprimé pour administration orale selon l'une quelconque des revendications précédentes, dans laquelle une forme sphérique de 80 % ou plus du charbon actif sphérique contenu en son sein est maintenue.

6. Composition pharmaceutique formée en comprimé pour administration orale selon l'une quelconque des revendications précédentes, dans laquelle une dureté de la composition pharmaceutique formée en comprimé pour administration orale est de 20 N ou plus.
dans laquelle la dureté est déterminée de la façon mentionnée dans la description.

**7.** Composition pharmaceutique formée en comprimé pour administration orale selon l'une quelconque des revendications précédentes, dans laquelle une friabilité de la composition pharmaceutique formée en comprimé pour administration orale est de 7 % en poids ou moins
dans laquelle la friabilité est déterminée de la façon mentionnée dans la description.

**8.** Composition pharmaceutique formée en comprimé pour administration orale selon l'une quelconque des revendications précédentes, dans laquelle une masse moléculaire moyenne en poids de l'excipient pour formulation de comprimé est de 10 000 ou plus.

**9.** Procédé de préparation d'une composition pharmaceutique formée en comprimé pour administration orale comprenant les étapes consistant à : (1) mélanger un total de 100 parties en poids de charbon actif sphérique et d'un ou plusieurs excipients choisis dans le groupe constitué de pullulane, alcool polyvinylique, gélatine, povidone, amidon prégélatinisé, amidon oxydé, hypromellose, hydroxypropylamidon, dextrine, copolymère d'alcool polyvinylique-acide acrylique-méthacrylate de méthyle, Kanbaiko, alginate de propylène glycol, agar-agar, glucomannane, carmellose sodique, hydroxyéthylcellulose, hydroxypropylcellulose, gomme tara, gomme de tamarin, carraghénane, méthylcellulose, hyaluronate de sodium, alginate de sodium, polymère carboxyvinylique, amidon partiellement prégélatinisé, gomme adragante, gomme de guar, gomme de xanthane, gomme de pépins de coing, gomme de caroube, phosphate de diamidon, gomme ghatti, et gomme gellane, et 10 parties ou plus en poids de solvant, dans lequel le charbon actif sphérique représente de 65 à 99 parties en poids et l'excipient représente de 1 à 35 parties en poids, et (a) le charbon actif sphérique ne présente pas de solubilité dans l'eau ni de caractéristique de gonflement ; un taux de déformation de celui-ci est de 2 % ou moins lorsque l'on applique une pression de 2 MPa ; et une résistance à la rupture de celui-ci est de 5 MPa ou plus, (b) un ou plusieurs excipients pour formulation de comprimé en guise d'excipient totalisent 1 partie ou plus en poids, à condition que lorsque les excipients pour formulation de comprimé incluent du pullulane, la quantité de pullulane soit de 1 partie ou plus en poids par rapport à la composition pharmaceutique formée en comprimé pour administration orale, et l'excipient pour formulation de comprimé forme un film mince lorsqu'on sèche à la chaleur 0,5 mL d'une solution aqueuse ou d'une dispersion aqueuse de l'excipient pour formulation de comprimé de 1 % en poids après être placée sur une surface plate constituée de résine fluorée,

(2) former le mélange en un produit sous forme de comprimé, et
(3) sécher le produit sous forme de comprimé.

**10.** Procédé de préparation d'une composition pharmaceutique formée en comprimé pour administration orale selon la revendication 9, le processus de séchage dans l'étape de séchage (3) est une lyophilisation, un séchage sous pression réduite, un séchage par aspiration, ou un séchage à la chaleur.

Figure 1

(Front view)          (Side view)

Figure 2

(A) (Comparative example 1)

(B) (Comparative example 2)

(C) (Comparative example 3)

(D) (Comparative example 4)

(E) (Comparative example 5)

(F) (Comparative example 6)

Figure 3

(A)

(B)

Figure 4

(A)

(B)

Figure 5

(A)

(B)

Figure 6

(A)

(B)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 62011611 B
- JP 2006008602 A
- JP 2006036734 A
- US 2787579 A
- JP 11292770 A
- JP 2002308785 A
- JP 3522708 A
- WO 200439381 A
- WO 200439380 A
- JP 2005314415 A
- JP 2005314416 A
- JP 2004244414 A
- JP 2007197338 A
- JP 2008303193 A
- JP 2002293906 A
- JP 2002293907 A
- JP 3522708 B

**Non-patent literature cited in the description**

- *Japanese Pharmaceutical Excipients,* 2003
- Japanese Pharmaceutical Excipients Directory 2007. Yakuji Nippo Limited, 2007
- The Japanese Pharmacopoeia
- **NISHIO HONDA ; KENKICHI KOISO ; KIYOSHI KUROKAWA.** Clinical Nephrology. Asakura-shoten, 1990
- Nephrology. Igaku-shoin, 1981